# NEW EUROPEAN PATENT SPECIFICATION

(11) **EP 0 705 903 B2**
(45) Date of publication and mention of the opposition decision: **12.08.2009**
(45) Mention of the grant of the patent: 23.05.2001
(21) Application number: 95305605.8
(22) Date of filing: 11.08.1995
(51) Int. Cl.: C07K 14/82, C12Q 1/68, G01N 33/574, C12N 5/10

(54) **Mutations in the 17q-linked breast and ovarian cancer susceptibility gene**
Mutationen des mit 17q verbundenen Ovarial- und Brustkrebs Empfindlichkeitsgens
Mutations du gène lié à 17q conférant une susceptibilité au cancer du sein et des ovaires

(30) Priority: 12.08.1994 US 289221; 02.09.1994 US 300266; 16.09.1994 US 308104; 29.11.1994 US 348824; 24.03.1995 US 409305; 07.06.1995 US 480784; 07.06.1995 US 483553
(43) Date of publication of application: 10.04.1996
(73) Proprietor: The University of Utah Research Foundation, Salt Lake City, Utah 84108 (US)
(72) Inventor: Shattuck-Eidens, Donna M., Salt Lake City, Utah 84108 (US); Simard, Jacques, Quebec, Canada G3A 1ZG (CA); Emi, Mitsuru, Tokyo (JP); Nakamura, Yusuke, Yokohama-shi, 225, Kanagawa-ken (JP); Durocher, Francine, Ste-foy, Quebec, G1X 1CI (CA)
(74) Representative: Vossius & Partner

(56) References cited:
- WO-A1-92/00311
- WO-A1-94/00764
- WO-A1-95/19369
- FRIEDMAN LS ET AL: 'Confirmation of BRCA1 by analysis of germline mutations linked to breast and ovarian cancer in ten families.' NAT GENET vol. 8, no. 4, 01 August 1994, pages 399 - 404, XP001180726
- PENG LIANG ET AL.: 'Differential display and cloning of messenger RNAs from human breast cancer versus mammary epithelial cells' CANCER RESEARCH vol. 52, pages 6966 - 6968, XP002032010
- M.R.STAMPFER ET AL.: 'Culture systems for study of human mammary epithelial cell proliferation, differentiation and transformation' CANCER SURVEYS vol. 18, pages 7 - 15, XP000770678
- HALL J.M. ET AL.: 'Linkage of early-onset familial breast cancer to chromosome 1 7q21' SCIENCE vol. 250, pages 1684 - 1689, XP000199763
- BOWCOCK A.M. ET AL.: 'thra1 and d17s183 flank an interval of <4 cM for the breast-ovarian cnacer gene BRCA1 on chromosome 17q21' AM.J.HUM.GENET. vol. 52, pages 718 - 722, XP000770702
- NEUHAUSEN SL ET AL: 'A P1-based physical map of the region from D17S776 to D17S78 containing the breast cancer susceptibility gene BRCA1.' HUM MOL GENET vol. 3, no. 11, 1994, pages 1919 - 1926, XP000770701
- GOLDGAR DE ET AL: 'A large kindred with 17q-linked breast and ovarian cancer: genetic, phenotypic, and genealogical analysis.' J NATL CANCER INST vol. 86, no. 3, 1994, pages 200 - 209, XP000770675
- DATABASE BIOSIS [Online] BIOSCIENCES INFORMATION SERVICE, PHILADELPHIA, PA, US HALL J M ET AL: 'CLOSING IN ON A BREAST CANCER GENE ON CHROMOSOME 17Q', XP002982893 Database accession no. 94064027 & AM J HUM GENET vol. 50, no. 6, 1992, pages 1235 - 1242
- DATABASE CANCERLIT [Online] NATIONAL CANCER INSTITUTE, BETHESDA, MD, US SATO T ET AL: 'THE HUMAN PROHIBITIN GENE LOCATED ON CHROMOSOME 17Q21 IS MUTATED IN SPORADIC BREAST CANCER', XP002983903 Database accession no. 92174193 & CANCER RES vol. 52, no. 6, 1992, pages 1643 - 1646
- MIKI Y ET AL: 'A strong candidate for the breast and ovarian cancer susceptibility gene BRCA1.' SCIENCE vol. 266, no. 5182, 1994, pages 66 - 71, XP000202410
- FUTREAL P.A. ET AL.: 'Brca1 mutations in primary breast and ovarian carcinomas' SCIENCE vol. 266, 07 October 1994, pages 120 - 122, XP002024011
- CASTILLA LH ET AL: 'Mutations in the BRCA1 gene in families with early-onset breast and ovarian cancer.' NAT GENET vol. 8, no. 4, 1994, pages 387 - 391, XP000770676
- WOOSTER R ET AL: 'Localization of a breast cancer susceptibility gene, BRCA2, to chromosome 13q12-13.' SCIENCE vol. 265, no. 5181, 1994, pages 2088 - 2090, XP002029509

## Description

The present invention relates generally to the field of human genetics. Specifically, the present invention relates a method for diagnosing a predisposition for breast and ovarian cancer in a human subject which comprises determining whether there is germline alteration 185delAG→ter39 in the BRCA1 gene in a tissue sample of said subject, said alteration indicating a predisposition to said cancer. The present invention furthermore relates to a method for diagnosing a breast or ovarian lesion of a human subject for neoplasia associated with the BRCA1 gene locus which comprises determining whether there is mutation 185delAG→ter39 in the BRCA1 gene in a sample from said lesion. The present invention also provides a nucleic acid probe having 15 to 30 nucleoticles of SEQ ID NO:1 and containing the mutation 185delAG→ter 39 as well as vectors and host cells as defined in the claims.

The publications and other materials used herein to illuminate the background of the invention, and in particular, cases to provide additional details respecting the practice, are incorporated herein by reference, and for convenience, are referenced by author and date in the following text and respectively grouped in the appended List of References.

### BACKGROUND OF THE INVENTION

The genetics of cancer is complicated, involving multiple dominant, positive regulators of the transformed state (oncogenes) as well as multiple recessive, negative regulators (tumor suppressor genes). Over one hundred oncogenes have been characterized. Fewer than a dozen tumor suppressor genes have been identified, but the number is expected to increase beyond fifty (Knudson, 1993).

The involvement of so many genes underscores the complexity of the growth control mechanisms that operate in cells to maintain the integrity of normal tissue. This complexity is manifest in another way. So far, no single gene has been shown to participate in the development of all, or even the majority of human cancers. The most common oncogenic mutations are in the H-ras gene, found in 10-15% of all solid tumors (Anderson *et al*., 1992). The most frequently mutated tumor suppressor genes are the TP53 gene, homozygously deleted in roughly 50% of all tumors, and CDKN2, which was homozygously deleted in 46% of tumor cell lines examined (Kamb *et al.,* 1994). Without a target that is common to all transformed cells, the dream of a "magic bullet" that can destroy or revert cancer cells while leaving normal tissue unharmed is improbable. The hope for a new generation of specifically targeted antitumor drugs may rest on the ability to identify tumor suppressor genes or oncogenes that play general roles in control of cell division.

The tumor suppressor genes which have been cloned and characterized influence susceptibility to: 1) Retinoblastoma (RB1); 2) Wilms' tumor (WT1); 3) Li-Fraumeni (TP53); 4) Familial adenomatous polyposis (APC); 5) Neurofibromatosis type 1 (NF1); 6) Neurofibromatosis type 2 (NF2); 7) von Hippel-Lindau syndrome (VHL); 8) Multiple endocrine neoplasia type 2A (MEN2A); and 9) Melanoma (CDKN2).

Tumor suppressor loci that have been mapped genetically but not yet isolated include genes for: Multiple endocrine neoplasia type 1 (MEN1); Lynch cancer family syndrome 2 (LCFS2); Neuroblastoma (NB); Basal cell nevus syndrome (BCNS); Beckwith-Wiedemann syndrome (BWS); Renal cell carcinoma (RCC); Tuberous sclerosis 1 (TSC1); and Tuberous sclerosis 2 (TSC2). The tumor suppressor genes that have been characterized to date encode products with similarities to a variety of protein types, including DNA binding proteins (WT1), ancillary transcription regulators (RB1), GTPase activating proteins or GAPs (NF1), cytoskeletal components (NF2), membrane bound receptor kinases (MEN2A), cell cycle regulators (CDKN2) and others with no obvious similarity to known proteins (APC and VHL).

In many cases, the tumor suppressor gene originally identified through genetic studies has been shown to be lost or mutated in some sporadic tumors. This result suggests that regions of chromosomal aberration may signify the position of important tumor suppressor genes involved both in genetic predisposition to cancer and in sporadic cancer.

One of the hallmarks of several tumor suppressor genes characterized to date is that they are deleted at high frequency in certain tumor types. The deletions often involve loss of a single allele, a so-called loss of heterozygosity (LOH), but may also involve homozygous deletion of both alleles. For LOH, the remaining allele is presumed to be nonfunctional, either because of a preexisting inherited mutation, or because of a secondary sporadic mutation.

Breast cancer is one of the most significant diseases that affects women. At the current rate, American women have a 1 in 8 risk of developing breast cancer by age 95 (American Cancer Society, 1992). Treatment of breast cancer at later stages is often futile and disfiguring, making early detection a high priority in medical management of the disease. Ovarian cancer, although less frequent than breast cancer is often rapidly fatal and is the fourth most common cause of cancer mortality in American women. Genetic factors contribute to an ill-defined proportion of breast cancer incidence, estimated to be about 5% of all cases but approximately 25% of cases diagnosed before age 40 (Claus *et al.,* 1991). Breast cancer has been subdivided into two types, early-age onset and late-age onset, based on an inflection in the age-specific incidence curve around age 50. Mutation of one gene, BRCA1, is thought to account for approximately 45% of familial breast cancer, but at least 80% of families with both breast and ovarian cancer (Easton *et al.,* 1993).

Intense efforts to isolate the BRCA1 gene have proceeded since it was first mapped in 1990 (Hall *et al.,* 1990; Narod *et al.,* 1991). A second locus, BRCA2, has recently been mapped to chromosome 13q (Wooster *et al.,* 1994) and appears to account for a proportion of early-onset breast cancer roughly equal to BRCA1, but confers a lower risk of ovarian cancer. The remaining susceptibility to early-onset breast cancer is divided between as yet unmapped genes for familial cancer, and rarer germline mutations in genes such as TP53 (Malkin *et al.,* 1990). It has also been suggested that heterozygote carriers for defective forms of the Ataxia-Telangectasia gene are at higher risk for breast cancer (Swift *et al*., 1976; Swift *et al.,* 1991). Late-age onset breast cancer is also often familial although the risks in relatives are not as high as those for early-onset breast cancer (Cannon-Albright *et al.,* 1994; Mettlin *et al*., 1990). However, the percentage of such cases due to genetic susceptibility is unknown.

Breast cancer has long been recognized to be, in part, a familial disease (Anderson, 1972). Numerous investigators have examined the evidence for genetic inheritance and concluded that the data are most consistent with dominant inheritance for a major susceptibility locus or loci (Bishop and Gardner, 1980; Go *et al*., 1983; Willams and Anderson, 1984; Bishop *et al.,* 1988; Newman *et al*., 1988; Claus *et al*., 1991). Recent results demonstrate that at least three loci exist which convey susceptibility to breast cancer as well as other cancers. These loci are the TP53 locus on chromosome 17p (Malkin *et al*., 1990), a 17q-linked susceptibility locus known as BRCA1 (Hall *et al*., 1990), and one or more loci responsible for the unmapped residual. Hall *et al*. (1990) indicated that the inherited breast cancer susceptibility in kindreds with early age onset is linked to chromosome 17q21; although subsequent studies by this group using a more appropriate genetic model partially refuted the limitation to early onset breast cancer (Margaritte *et al.,* 1992).

Most strategies for cloning the 17q-linked breast cancer predisposing gene (BRCA1) require precise genetic localization studies. The simplest model for the functional role of BRCA1 holds that alleles of BRCA1 that predispose to cancer are recessive to wild type alleles; that is, cells that contain at least one wild type BRCA1 allele are not cancerous. However, cells that contain one wild type BRCA1 allele and one predisposing allele may occasionally suffer loss of the wild type allele either by random mutation or by chromosome loss during cell division (nondisjunction). All the progeny of such a mutant cell lack the wild type function of BRCA1 and may develop into tumors. According to this model, predisposing alleles of BRCA1 are recessive, yet susceptibility to cancer is inherited in a dominant fashion: women who possess one predisposing allele (and one wild type allele) risk developing cancer, because their mammary epithelial cells may spontaneously lose the wild type BRCA1 allele. This model applies to a group of cancer susceptibility loci known as tumor suppressors or antioncogenes, a class of genes that includes the retinoblastoma gene and neurofibromatosis gene.

By inference this model may also explain the BRCA1 function, as has recently been suggested (Smith *et al*., 1992).

A second possibility is that BRCA1 predisposing alleles are truly dominant; that is, a wild type allele of BRCA1 cannot overcome the tumor forming role of the predisposing allele. Thus, a cell that carries both wild type and mutant alleles would not necessarily lose the wild type copy of BRCA1 before giving rise to malignant cells. Instead, mammary cells in predisposed individuals would undergo some other stochastic change(s) leading to cancer.

If BRCA1 predisposing alleles are recessive, the BRCA1 gene is expected to be expressed in normal mammary tissue but not functionally expressing in mammary tumours. In contrast, if BRCA1 predisposing alleles are dominant, the wild type BRCA1 gene may or may not be expressed in normal mammary tissue. However, the predisposing allele will likely be expressed in breast tumour cells.

The 17q linkage of BRCA1 was independently confirmed in three of five kindred with both breast cancer and ovarian cancer (Narod *et al.,* 1991). These studies claimed to localize the gene within a very large region, 15 centiMorgans (cM), or approximately 15 million base pairs, to either side of the linked marker pCMM86 (D17S74). However, attempts to define the region further by genetic studies, using markers surrounding pCMMS6, proved unsuccessful. Subsequent studies indicted that the gene was considerably more proximal (Easton *et al.,* 1993) and that the original analysis was flawed (Margaritte *et al*., 1992). Hall *et al.,* (1992) recently localized the BRCA1 gene to an approximately 8 cM interval (approximately 8 million base pairs) bounded by Mfd15 (D17S250) on the proximal side and the human GIP gene on the distal side. A slightly narrower interval for the BRCA1 locus, based on publicly available data, was agreed upon at the Chromosome 17 workshop in March of 1992 (Fain, 1992). The size of these regions and the uncertainty associated with them has made it exceedingly difficult to design and implement physical mapping and/or cloning strategies for isolating the BRCA1 gene.

Identification of a breast cancer susceptibility locus would permit the early detection of susceptible individuals and greatly increase our ability to understand the initial steps which lead to cancer. As susceptibility loci are often altered during tumour progression, cloning these genes could also be important in the development of better diagnostic and prognostic products, as well as better cancer therapies.

### SUMMARY OF INVENTION

In one aspect, the present invention provides a method for diagnosing a predisposition for breast and ovarian cancer in a human subject which comprises determining whether there is germline alteration 185delAG→ter39 in the BRCA1 gene in a tissue sample of said subject, said alteration indicating a predisposition to said cancer. The present invention furthermore relates to a method for diagnosing a breast or ovarian lesion of a human subject for neoplasia associated with the BRCA1 gene locus which comprises determining whether there is mutation 185delAG→ter39 in the BRCA1 gene in a sample from said lesion. The present invention also provides a nucleic acid probe having 15 to 30 nucleoticles of SEQ ID NO:1 and containing the mutation 185delAG→ter 39 as well as vectors and host cells as defined in the claims.

### BRIEF DESCRIPTION OF THE DRAWINGS

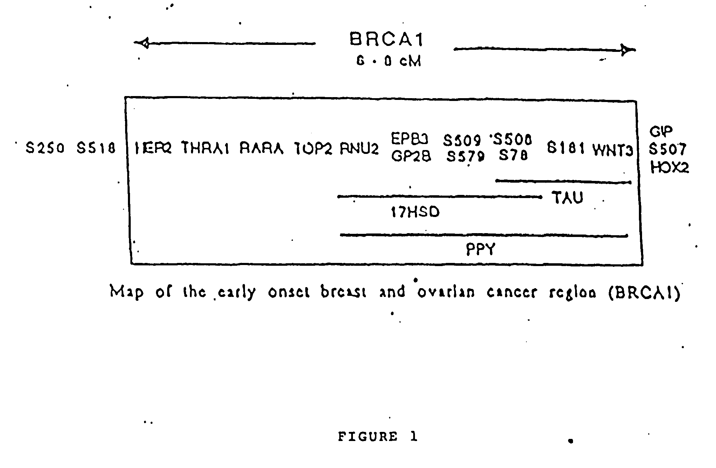

Figure 1 is a diagram showing the order of loci neighbouring BRCA1 as determined by the chromosome 17 workshop. Figure 1 is reproduced from Fain, 1992.

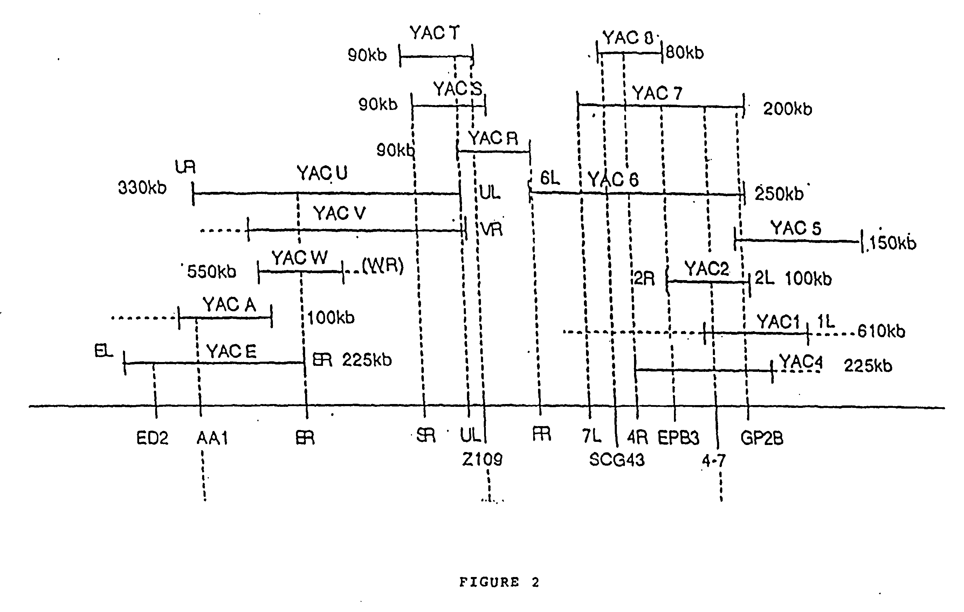

Figure 2 is a schematic map of YACs which define part of Mfd15-Mfd188 region.

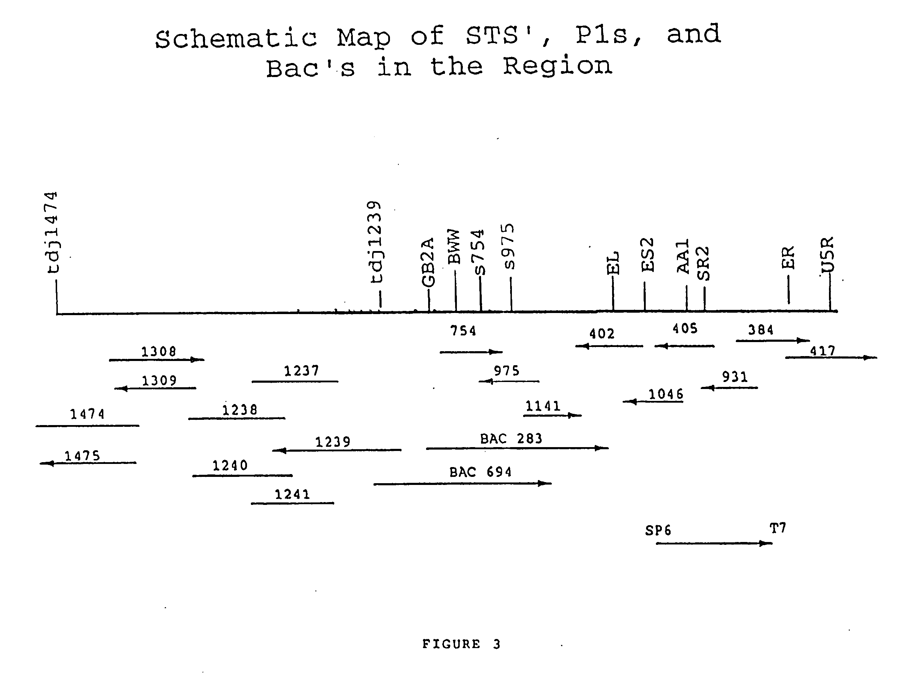

Figure 3 is a schematic map of STSs, P1, and BACs in the BRCA1 region.

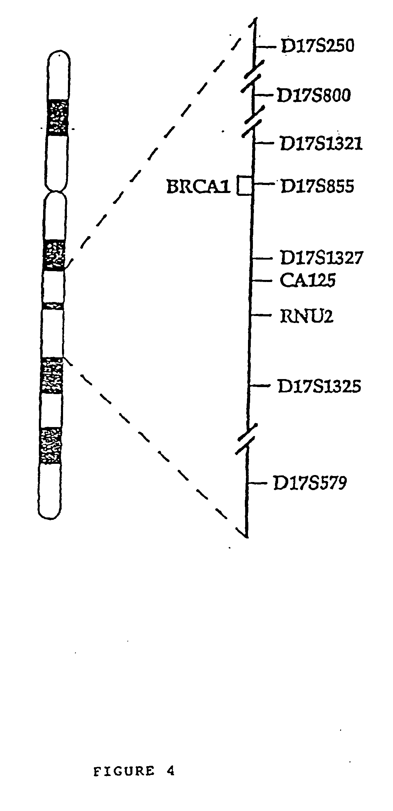

Figure 4 is a schematic map of human chromosome 17. The pertinent region containing BRCA1 is expanded to indicate the relative positions of two previously identified genes, CA125 and RNU2, BRCA1 spans the marker D17S855.

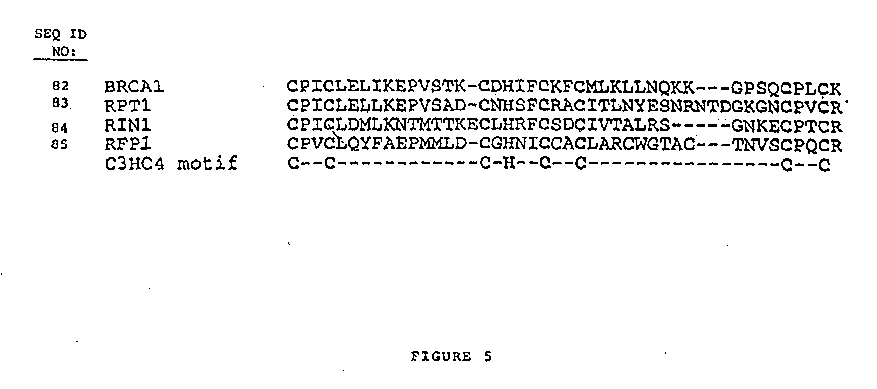

Figure 5 shows alignment of the BRCA1 zinc-finger domain with 3 other zinc-finger domains that scored highest in a Smith-Waterman alignment. RPT1 encodes a protein that appears to be a negative regulator of the IL-2 receptor in mouse. RIN1 encodes a DNA-binding protein that includes a RING-finger motif related to the zinc-finger. RFP1 encodes a putative transcription factor that is the N-terminal domain of the RET oncogene product. The bottom line contains the C3HC4 consensus zinc-finger sequence showing the positions of cysteines and one histidine that form the zinc ion binding pocket.

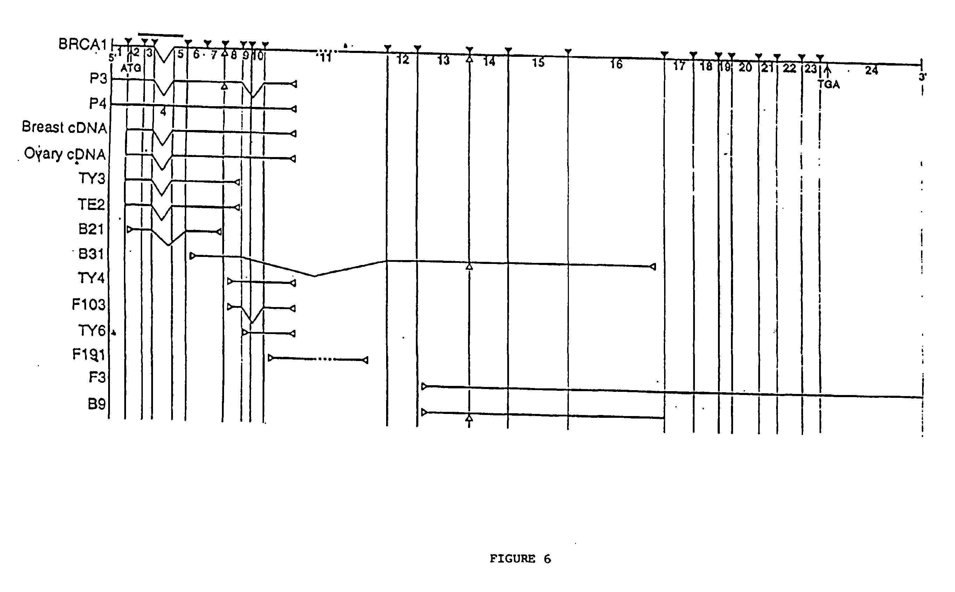

Figure 6 is a diagram of BRCA1 mRNA showing the locations of introns and the variants of BRCA1 mRNA produced by alternative splicing. Intron locations are shown by dark triangles and the exons are numbered below the line representing the cDNA. The top cDNA is the composite used to generate the peptide sequence of BRCA1. Alternative forms identified as cDNA clones or hybrid selection clones are shown below.

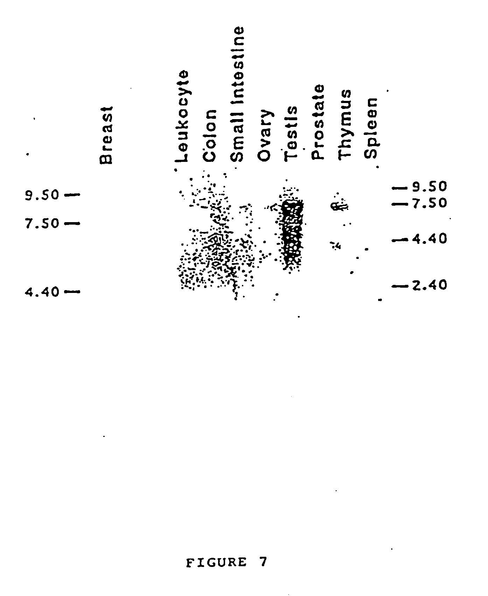

Figure 7 shows the tissue expression pattern of BRCA1. The blot was obtained from Clontech and contains RNA from the indicated tissues. Hybridization conditions were as recommended by the manufacturer using a probe consisting of nucleotide positions 3631 to 3930 of BRCA1. Note that both breast and ovary are heterogeneous tissues and the percentage of relevant epithelial cells can be variable. Molecular weight standards are in kilobases.

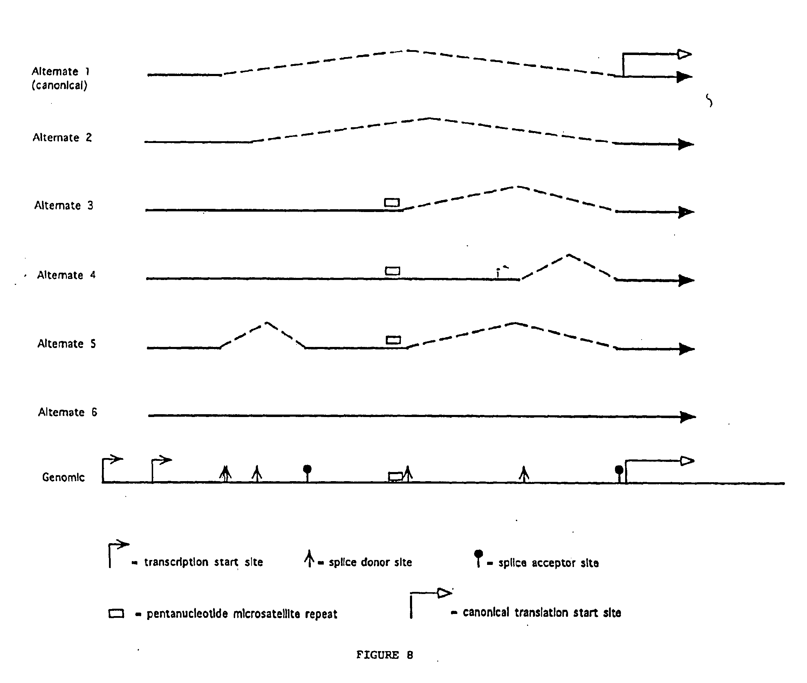

Figure 8 is a diagram of the 5' untranslated region plus the beginning of the translated region of BRCA1 showing the locations of introns and the variants of BRCA1 mRNA produced by alternative splicing. Intron locations are shown by broken dashed lines. Six alternate splice forms are shown.

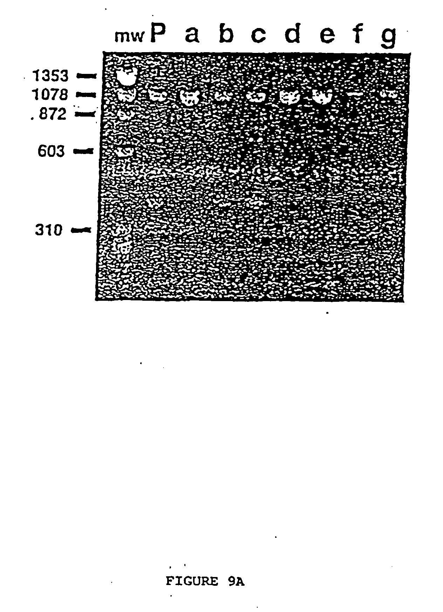

Figure 9A shows a nonsense mutation in Kindred 2082. P indicates the person originally screened, b and c are haplotype carriers, a, d, e, f, and g do not carry the BRCA1 haplotype. The C to T mutation results in a stop codon and creates a site for the restriction enzyme AvrII. PCR amplification products are cut with this enzyme. The carriers are heterozygous for the site and therefore show three bands. Non-carriers remain uncut.

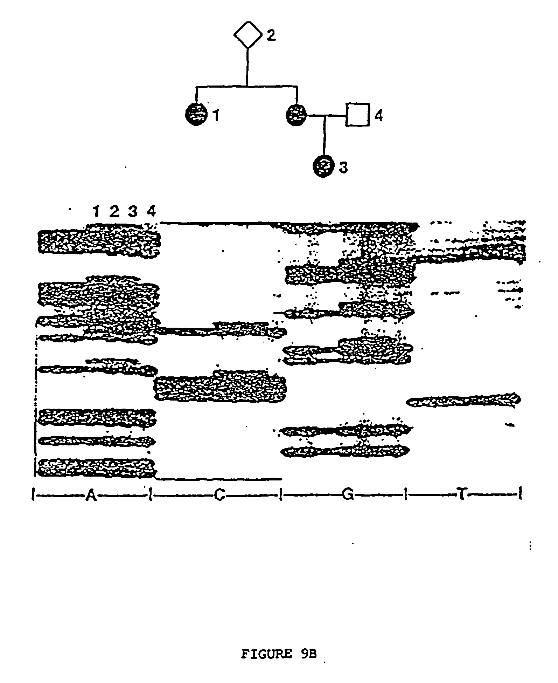

Figure 9B shows a mutation and cosegregation analysis in BRCA1 kindreds. Carrier individuals are represented as filled circles and squares in the pedigree diagrams. Frameshift mutation in Kindred 1910. The first three lanes are control, noncarrier samples. Lanes labeled 1-3 contain sequences from carrier individuals. Lane 4 contains DNA from a kindred member who does not carry the BRCA1 mutation. The diamond is used to prevent identification of the kindred. The frameshift resulting from the additional C is apparent in lanes labeled 1, 2, and 3.

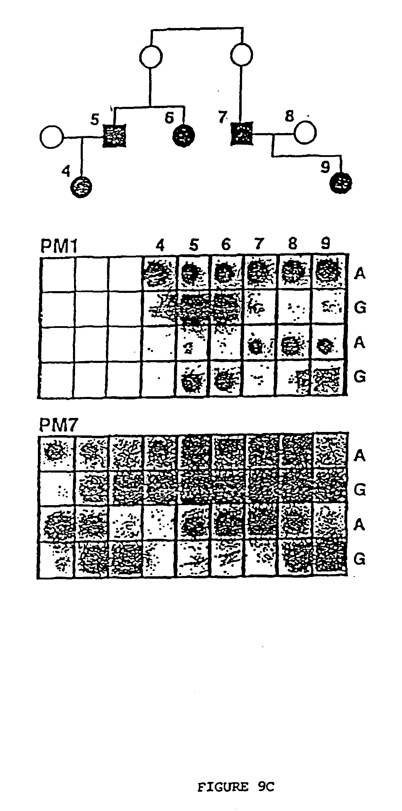

Figure 9C shows a mutation and cosegregation analysis in BRCA1 kindreds. Carrier individuals are represented as filled circles and squares in the pedigree diagrams. Inferred regulatory mutation in Kindred 2035. ASO analysis of carriers and noncarriers of 2 different polymorphisms (PM1 and PM7) which were examined for heterozygosity in the germline and compared to the heterozygosity of lymphocyte mRNA. The top 2 rows of each panel contain PCR products amplified from genomic DNA and the bottom 2 rows contain PCR products amplified from cDNA. "A" and "G" are the two alleles detected by the ASO. The dark spots indicate that a particular allele is present in the sample. The first three lanes of PM7 represent the three genotypes in the general population.

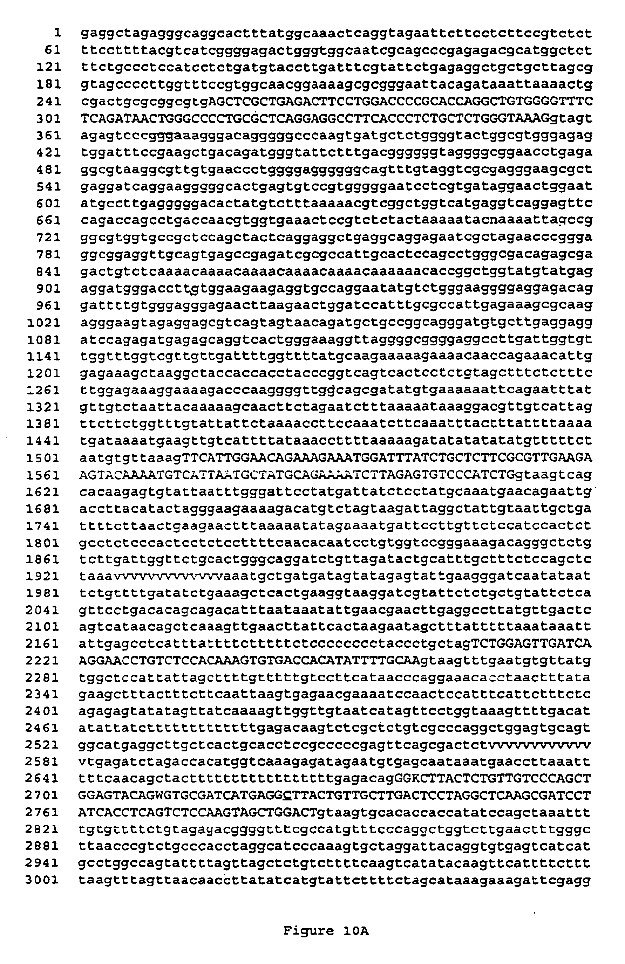

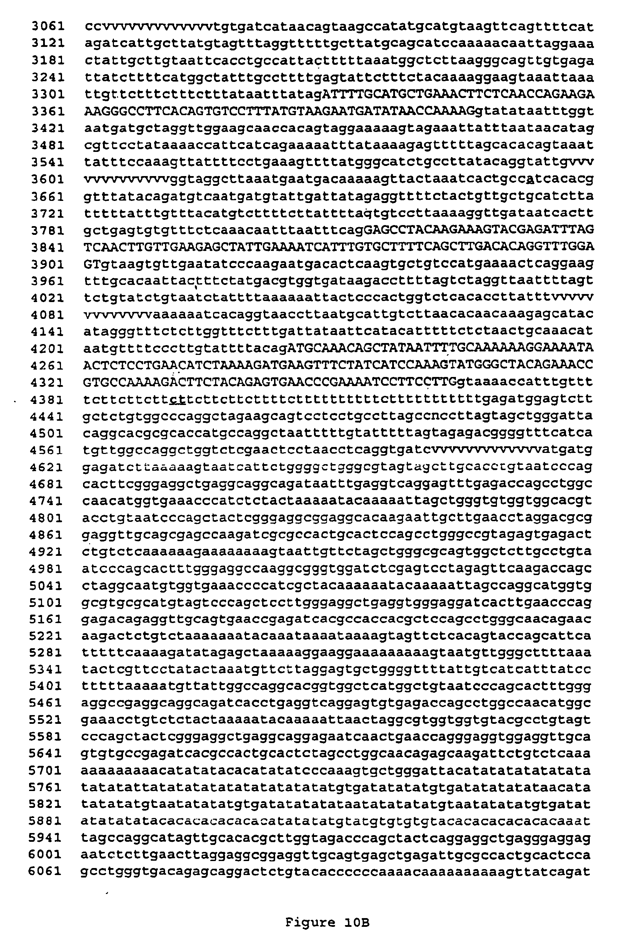

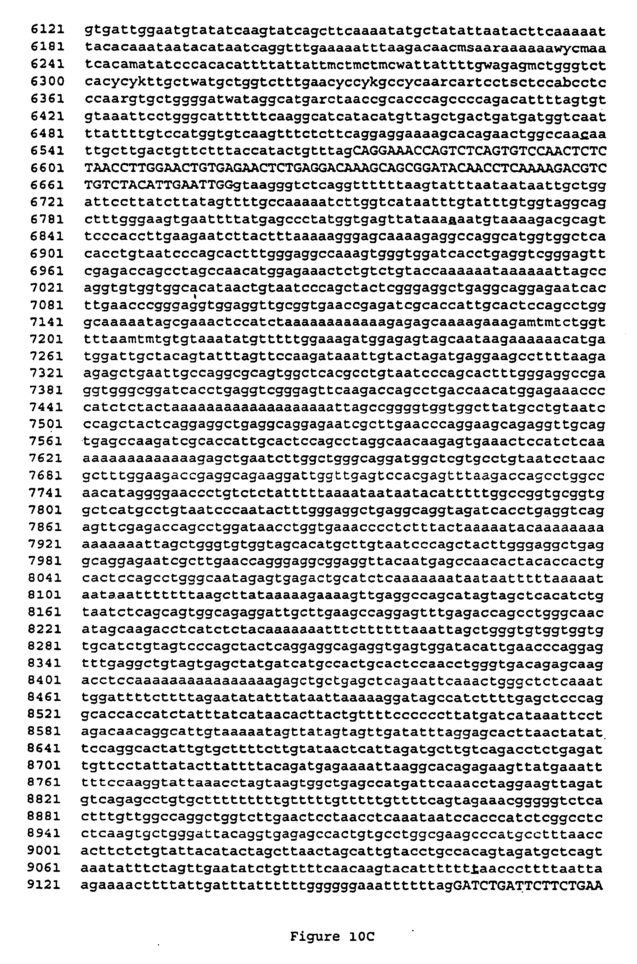

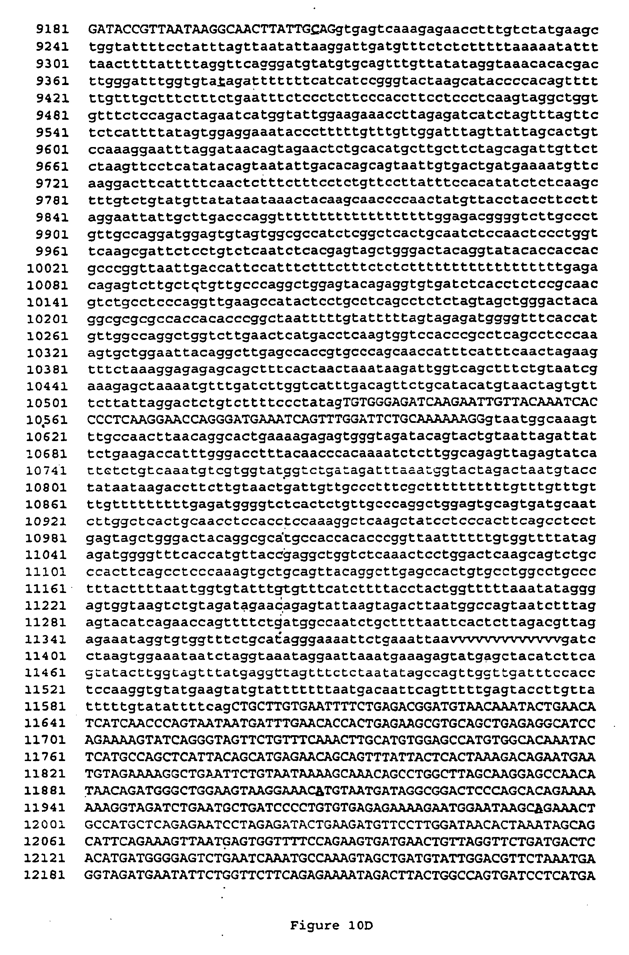

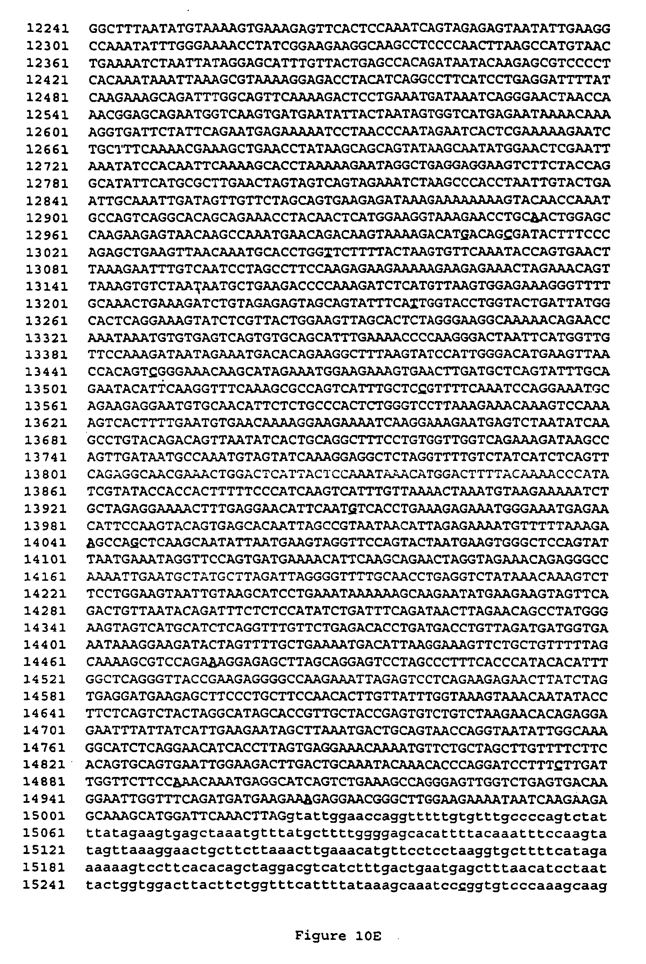

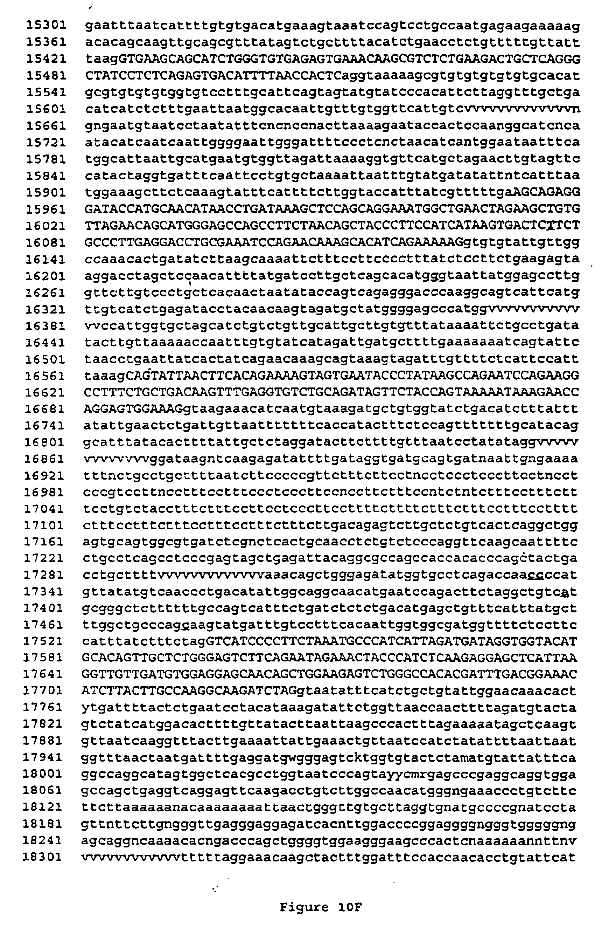

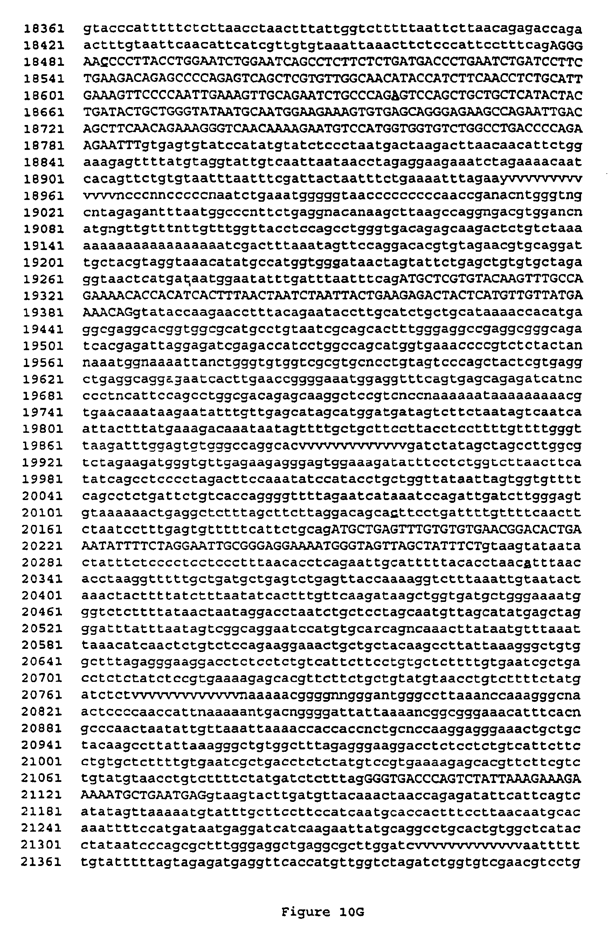

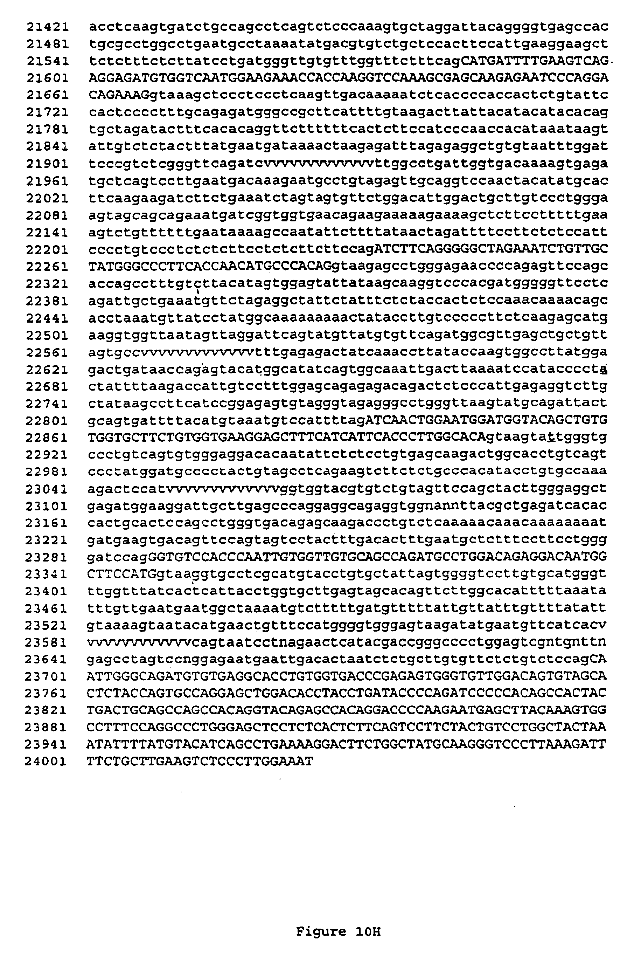

Figures 10A-10H show genomic sequence of BRCA1. The lower case letters denote intron sequence while the upper case letters denote exon sequence. Indefinite intervals within introns are designated with vvvvvvvvvvvvv. Known polymorphic sites are shown as underlined and boldface type.

### DETAILED DESCRIPTION OF THE INVENTION

The present invention relates generally to the field of human genetics. Specifically, the present invention relates to method for diagnosing a predisposition for breast and ovarian cancer in a human subject which comprises determining whether there is germline alteration 185delAG→ter39 in the BRCA1 gene in a tissue sample of said subject, said alteration indicating a predisposition to said cancer. The present invention furthermore relates to a method for diagnosing a breast or ovarian lesion of a human subject for neoplasia associated with the BRCA1 gene locus which comprises determining whether there is mutation 185delAG→ter39 in the BRCA1 gene in a sample from said lesion. The present invention also provides a nucleic acid probe having 15 to 30 nucleoticles of SEQ ID NO:1 and containing the mutation 185delAG→ter 39 as well as vectors and host cells as defined in the claims.

The present disclosure provides an isolated polynucleotide comprising all, or a portion of the BRCA1 locus or of a mutated BRCA1 locus, preferably at least eight bases and not more than about 100 kb in length. Such polynucleotides may be antisense polynucleotides. The present invention also provides a recombinant construct comprising such an isolated polynucleotide, for example, a recombinant construct suitable for expression in a transformed host cell.

The present disclosure further provides methods of preparing a polynucleotide comprising polymerizing nucleotides to yield a sequence comprised of at least eight consecutive nucleotides of the BRCA1 locus; and methods of preparing a polypeptide comprising polymerizing amino acids to yield a sequence comprising at least five amino acids encoded within the BRCA1 locus.

The method of the invention may further comprise the step of amplifying a portion of the BRCA1 locus, and may further include a step of providing a set of polynucleotides which are primers for amplification of said portion of the BRCA1 locus. The method is useful for identifying mutation 185delAG for use in either diagnosis of the predisposition to cancer or the diagnosis or prognosis of cancer.

The BRCA1 locus predisposes individuals to breast cancer and ovarian cancer, is a gene encoding a BRCA1 protein, which has been found to have no significant homology with known protein or DNA sequences. This gene is termed BRCA1 herein. Mutations in the BRCA1 locus in the germline are indicative of a predisposition to breast cancer and ovarian cancer. Finally, somatic mutations in the BRCA1 locus are also associated with breast cancer, ovarian cancer and other cancers, which represents an indicator of these cancers or of the prognosis of these cancers. The mutational events of the BRCA1 locus can involve deletions, insertions and point mutations within the coding sequence and the non-coding sequence.

Starting from a region on the long arm of human chromosome 17 of the human genome, 17q, which has a size estimated at about 8 million base pairs, a region which contains a genetic locus, BRCA1, which causes susceptibility to cancer, including breast and ovarian cancer, has been identified.

The region containing the BRCA1 locus was identified using a variety of genetic techniques. Genetic mapping techniques initially defined the BRCA1 region in terms of recombination with genetic markers. Based upon studies of large extended families ("kindreds") with multiple cases of breast cancer (and ovarian cancer crises in some kindreds), a chromosomal region has been pinpointed that contains the BRCA1 gene as well as other putative susceptibility alleles in the BRCA1 locus. Two meiotic breakpoints have been discovered on the distal side of the BRCA1 locus which are expressed as recombinants between genetic markers and the disease, and one recombinant on the proximal side of the BRCA1 locus. Thus, a region which contains the BRCA1 locus is physically bounded by these markers.

The use of the genetic markers provided allowed the identification of clones which cover the region from a human yeast artificial chromosome (YAC) or a human bacterial artificial chromosome (BAC) library. It also allowed for the identification and preparation of more easily manipulated cosmid, P1 and BAC clones from this region and the construction of a contig from a subset of the clones. These cosmids, P1s, YACs and BACs provide the basis for cloning the BRCA1 locus and provide the basis for developing reagents effective, for example, in the diagnosis and treatment of breast and/or ovarian cancer. The BRCA1 gene and other potential susceptibility genes have been isolated from this region. The isolation was done using software trapping (a computational method for identifying sequences likely to contain coding exons, from contiguous or discontinuous genomic DNA sequences), hybrid selection techniques and direct screening, with whole or partial cDNA inserts from cosmids, P1s and BACs, in the region to screen cDNA libraries. These methods were used to obtain sequences of loci expressed in breast and other tissue. These candidate loci were analyzed to identify sequences which confer cancer susceptibility. We have discovered that there are mutations in the coding sequence of the BRCA1 locus in kindreds which are responsible for the 17q-linked cancer susceptibility known as BRCA1. This gene was not known to be in this region. The present invention not only facilitates the early detection of certain cancers, so vital to patient survival, but also permits the detection of susceptible individuals before they develop cancer.

### Population Resources

Large, well-documented Utah kindreds are especially important in providing good resources for human genetic studies. Each large kindred independently provides the power to detect whether a BRCA1 susceptibility allele is segregating in that family. Recombinants informative for localization and isolation of the BRCA1 locus could be obtained only from kindreds large enough to confirm the presence of a susceptibility allele. Large sibships are especially important for studying breast cancer, since penetrance of the BRCA1 susceptibility allele is reduced both by age and sex, making informative sibships difficult to find. Furthermore, large sibships are essential for constructing haplotypes of deceased individuals by inference from the haplotypes of their close relatives.

While other populations may also provide beneficial information, such studies generally require much greater effort, and the families are usually much smaller and thus less informative. Utah's age-adjusted breast cancer incidence is 20% lower than the average U.S. rate. The lower incidence in Utah is probably due largely to an early age at first pregnancy, increasing the probability that cases found in Utah kindreds carry a genetic predisposition.

### Genetic Mapping

Given a set of informative families, genetic markers are essential for linking a disease to a region of a chromosome. Such markers include restriction fragment length polymorphisms (RFLPs) (Botstein *et al.,* 1980), markers with a variable number of tandem repeats (VNTRs) (Jeffreys *et al.,* 1985; Nakamura *et al.,* 1987), and an abundant class of DNA polymorphisms based on short tandem repeats (STRs), especially repeats of CpA (Weber and May, 1989; Litt *et al.,* 1989). To generate a genetic map, one selects potential genetic markers and tests them using DNA extracted from members of the kindreds being studied.

Genetic markers useful in searching for a genetic locus associated with a disease can be selected on an *ad hoc* basis, by densely covering a specific chromosome, or by detailed analysis of a specific region of a chromosome. A preferred method for selecting genetic markers linked with a disease involves evaluating the degree of informativeness of kindreds to determine the ideal distance between genetic markers of a given degree of polymorphism, then selecting markers from known genetic maps which are ideally spaced for maximal efficiency. Informativeness of kindreds is measured by the probability that the markers will be heterozygous in unrelated individuals. It is also most efficient to use STR markers which are detected by amplification of the target nucleic acid sequence using PCR; such markers are highly informative, easy to assay (Weber and May, 1989), and can be assayed simultaneously using multiplexing strategies (Skolnick and Wallace, 1988), greatly reducing the number of experiments required.

Once linkage has been established, one needs to find markers that flank the disease locus, i.e., one or more markers proximal to the disease locus, and one or more markers distal to the disease locus. Where possible, candidate markers can be selected from a known genetic map. Where none is known, new markers can be identified by the STR technique, as shown in the Examples.

Genetic mapping is usually an iterative process. It began by defining flanking genetic markers around the BRCA1 locus, then replacing these flanking markers with other markers that were successively closer to the BRCA1 locus. As an initial step, recombination events, defined by large extended kindreds, helped specifically to localize the BRCA1 locus as either distal or proximal to a specific genetic marker (Goldgar *et al*., 1994).

The region surrounding BRCA1 was not well mapped and there were few markers. Therefore, short repetitive sequences on cosmids subcloned from YACs, which had been physically mapped, were analyzed in order to develop new genetic markers. Using this approach, one marker, 42D6, was discovered which replaced pCMM86 as the distal flanking marker for the BRCA1 region. Since 42D6 is approximately 14 cM from pCMM86, the BRCA1 region was thus reduced by approximately 14 centiMorgans (Easton *et al.,* 1993)and began by finding a much more closely linked distal flanking marker of the BRCA1 region, BRCA1 was then discovered to be distal to the genetic marker Mfd15. Therefore, BRCA1 was shown to be in a region of 6 to 10 million bases bounded by Mfd15 and 42D6. Marker Mfd191 was subsequently discovered to be distal to Mfd15 and proximal to BRCA1. Thus, Mfd15 was replaced with Mfd191 as the closest proximal genetic marker. Similarly, it was discovered that genetic marker Mfd188 could replace genetic marker 42D6, narrowing the region containing the BRCA1 locus to approximately 1.5 million bases. Then the marker Mfd191 was replaced with tdj1474 as the proximal marker and Mfd188 was replaced with USR as the distal marker, further narrowing the BRCA1 region to a small enough region to allow isolation and characterization of the BRCA1 locus (see Figure 3), using techniques known in the art and described herein.

### Physical Mapping

Three distinct methods were employed to physically map the region. The first was the use of yeast artificial chromosomes (YACs) to clone the region which is flanked by tdj1474 and U5R. The second was the creation of a set of P1, BAC and cosmid clones which cover the region containing the BRCA1 locus.

Yeast Artificial Chromosomes (YACs). Once a sufficiently small region containing the BRCA1 locus was identified, physical isolation of the DNA in the region proceeded by identifying a set of overlapping YACs which covers the region. Useful YACs can be isolated from known libraries, such as the St. Louis and CEPH YAC libraries, which are widely distributed and contain approximately 50,000 YACs each. The YACs isolated were from these publicly accessible libraries and can be obtained from a number of sources including the Michigan Genome Center. Clearly, others who had access to these YACs, without the present disclosure, would not have known the value of the specific YACs we selected since they would not have known which YACs were within, and which YACs outside of, the smallest region containing the BRCA1 locus.

Cosmid, P1 and BAC Clones. In the present disclosure, it is advantageous to proceed by obtaining cosmid, P1, and BAC clones to cover this region. The smaller size of these inserts, compared to YAC inserts, makes them more useful as specific hybridization probes. Furthermore, having the cloned DNA in bacterial cells, rather than in yeast cells, greatly increases the ease with which the DNA of interest can be manipulated, and improves the signal-to-noise ratio of hybridization assays. For cosmid subclones of YACs, the DNA is partially digested with the restriction enzyme Sau3A and cloned into the BamHI site of the pWE15 cosmid vector (Stratagene, cat. #1251201). The cosmids containing human sequences are screened by hybridization with human repetitive DNA (e.g., Gibco/BRL, Human Cₒt-1 DNA, cat. 5279SA), and then fingerprinted by a variety of techniques, as detailed in the Examples.

P1 and BAC clones are obtained by screening libraries constructed from the total human genome with specific sequence tagged sites (STSs) derived from the YACs, cosmids or P1s and BACs, isolated as described herein.

These P1, BAC and cosmid clones can be compared by interspersed repetitive sequence (IRS) PCR and/or restriction enzyme digests followed by gel electrophoresis and comparison of the resulting DNA fragments ("fingerprints") (Maniatis *et al.,* 1982). The clones can also be characterized by the presence of STSs. The fingerprints are used to define an overlapping contiguous set of clones which covers the region but is not excessively redundant, referred to herein as a "minimum tiling path". Such a minimum tiling path forms the basis for subsequent experiments to identify cDNAs which may originate from the BRCA1 locus.

Coverage of the Gap with P1 and BAC Clones. To cover any gaps in the BRCA1 contig between the identified cosmids with genomic clones, clones in P1 and BAC vectors which contain inserts of genomic DNA roughly twice as large as cosmids for P1s and still greater for BACs (Stemberg, 1990; Sternberg *et al*., 1990; Pierce *et al.,* 1992; Shizuya *et al.,* 1992) were used. P1 clones were isolated by Genome Sciences using PCR primers provided by us for screening. BACs were provided by hybridization techniques in Dr. Mel Simon's laboratory. The strategy of using P1 clones also permitted the covering of the genomic region with an independent set of clones not derived from YACs. This guards against the possibility of other deletions in YACs that have not been detected. These new sequences derived from the P1 clones provide the material for further screening for candidate genes, as described below.

### Gene Isolation.

There are many techniques for testing genomic clones for the presence of sequences likely to be candidates for the coding sequence of a locus one is attempting to isolate, including but not limited to:
a. zoo blots
b. identifying HTF islands
c. exon trapping
d. hybridizing cDNA to cosmids or YACs.
e. screening cDNA libraries.

(a) Zoo blots. The first technique is to hybridize cosmids to Southern blots to identify DNA sequences which are evolutionarily conserved, and which therefore give positive hybridization signals with DNA from species of varying degrees of relationship to humans (such as monkey, cow, chicken, pig, mouse and rat). Southern blots containing such DNA from a variety of species are commercially available (Clonetech, Cat. 7753-1).
(b) Identifying HTF islands. The second technique involves finding regions rich in the nucleotides C and G, which often occur near or within coding sequences. Such sequences are called HTF (HpaI tiny fragment) or CpG islands, as restriction enzymes specific for sites which contain CpG dimers cut frequently in these regions (Lindsay *et al.,* 1987).
(c) Exon trapping. The third technique is exon trapping, a method that identifies sequences in genomic DNA which contain splice junctions and therefore are likely to comprise coding sequences of genes. Exon amplification (Buckler *et al*., 1991) is used to select and amplify exons from DNA clones described above. Exon amplification is based on the selection of RNA sequences which are flanked by functional 5' and/or 3' splice sites. The products of the exon amplification are used to screen the breast cDNA libraries to identify a manageable number of candidate genes for further study. Exon trapping can also be performed on small segments of sequenced DNA using computer programs or by software trapping.
(d) Hybridizing cDNA to Cosmids, P1s, BACs or YACs. The fourth technique is a modification of the selective enrichment technique which utilizes hybridization of cDNA to cosmids, P1s, BACs or YACs and permits transcribed sequences to be identified in, and recovered from cloned genomic DNA (Kandpal *et al.,* 1990). The selective enrichment technique, as modified for the present purpose, involves binding DNA from the region of BRCA1 present in a YAC to a column matrix and selecting cDNAs from the relevant libraries which hybridize with the bound DNA, followed by amplification and purification of the bound DNA, resulting in a great enrichment for cDNAs in the region represented by the cloned genomic DNA.
(e) Identification of cDNAs. The fifth technique is to identify cDNAs that correspond to the BRCA1 locus. Hybridization probes containing putative coding sequences, selected using any of the above techniques, are used to screen various libraries, including breast tissue cDNA libraries, ovarian cDNA libraries, and any other necessary libraries.

Another variation on the theme of direct selection of cDNA was also used to find candidate genes for BRCA1 (Lovett *et al.,* 1991; Futreal, 1993). This method uses cosmid, P1 or BAC DNA as the probe. The probe DNA is digested with a blunt cutting restriction enzyme such as HaeIII. Double stranded adapters are then ligated onto the DNA and serve as binding sites for primers in subsequent PCR amplification reactions using biotinylated primers. Target cDNA is generated from mRNA derived from tissue samples, e.g., breast tissue, by synthesis of either random primed or oligo(dT) primed first strand followed by second strand synthesis. The cDNA ends are rendered blunt and ligated onto double-stranded adapters. These adapters serve as amplification sites for PCR. The target and probe sequences are denatured and mixed with human Cₒt-1 DNA to block repetitive sequences. Solution hybridization is carried out to high Cₒt-1/2 values to ensure hybridization of rare target cDNA molecules. The annealed material is then captured on avidin beads, washed at high stringency and the retained cDNAs are eluted and amplified by PCR. The selected cDNA is subjected to further rounds of enrichment before cloning into a plasmid vector for analysis.

### Testing the cDNA for Candidacy

Proof that the cDNA is the BRCA1 locus is obtained by finding sequences in DNA extracted from affected kindred members which create abnormal BRCA1 gene products or abnormal levels of BRCA1 gene product. Such BRCA1 susceptibility alleles will co-segregate with the disease in large kindreds. They will also be present at a much higher frequency in non-kindred individuals with breast and ovarian cancer then in individuals in the general population. Finally, since tumors often mutate somatically at loci which are in other instances mutated in the germline, we expect to see normal germline BRCA1 alleles mutated into sequences which are identical or similar to BRCA1 susceptibility alleles in DNA extracted from tumor tissue. Whether one is comparing BRCA1 sequences from tumor tissue to BRCA1 alleles from the germline of the same individuals, or one is comparing germline BRCA1 alleles from cancer cases to those from unaffected individuals, the key is to find mutations which are serious enough to cause obvious disruption to the normal function of the gene product. These mutations can take a number of forms. The most severe forms would be frame shift mutations or large deletions which would cause the gene to code for an abnormal protein or one which would significantly alter protein expression. Less severe disruptive mutations would include small in-frame deletions and nonconservative base pair substitutions which would have a significant effect on the protein produced, such as changes to or from a cysteine residue, from a basic to an acidic amino acid or vice versa, from a hydrophobic to hydrophilic amino acid or vice versa, or other mutations which would affect secondary, tertiary or quaternary protein structure. Silent mutations or those resulting in conservative amino acid substitutions would not generally be expected to disrupt protein function.

According to the diagnostic and prognostic method of the present invention, the alteration 185delAG of the wild-type BRCA1 locus is detected. In addition, the method can be performed by detecting the wild-type BRCA1 locus and confirming the lack of a predisposition to cancer at the BRCA1 locus. "Alteration of a wild-type gene" generally encompasses all forms of mutations including deletions, insertions and point mutations in the coding and noncoding regions. Deletions may be of the entire gene or of only a portion of the gene. Point mutations may result in stop codons, frameshift mutations or amino acid substitutions. Somatic mutations are those which occur only in certain tissues, e.g., in the tumor tissue, and are not inherited in the germline. Germline mutations can be found in any of a body's tissues and are inherited. If only a single allele is somatically mutated, an early neoplastic state is indicated. However, if both alleles are somatically mutated, then a late neoplastic state is indicated. The finding of BRCA1 mutations thus provides both diagnostic and prognostic information. A BRCA1 allele which is not deleted (e.g., found on the sister chromosome to a chromosome carrying a BRCA1 deletion) can be screened for other mutations, such as insertions, small deletions, and point mutations. It is believed that many mutations found in tumor tissues will be those leading to decreased expression of the BRCA1 gene product. However, mutations leading to non-functional gene products would also lead to a cancerous state. Point mutational events may occur in regulatory regions, such as in the promoter of the gene, leading to loss or diminution of expression of the mRNA. Point mutations may also abolish proper RNA processing, leading to loss of expression of the BRCA1 gene product, or to a decrease in mRNA stability or translation efficiency.

Useful diagnostic techniques include, but are not limited to fluorescent *in situ* hybridization (FISH), direct DNA sequencing, PFGE analysis, Southern blot analysis, single stranded conformation analysis (SSCA), RNase protection assay, allele-specific oligonucleotide (ASO), dot blot analysis and PCR-SSCP, as discussed in detail further below.

Predisposition to cancers, such as breast and ovarian cancer, and the other cancers identified herein, can be ascertained by testing any tissue of a human for mutations of the BRCA1 gene. For example, a person who has inherited a germline BRCA1 mutation would be prone to develop cancers. This can be determined by testing DNA from any tissue of the person's body. Most simply, blood can be drawn and DNA extracted from the cells of the blood. In addition, prenatal diagnosis can be accomplished by testing fetal cells, placental cells or amniotic cells for mutations of the BRCA1 gene. Alteration of a wild-type BRCA1 allele, whether, for example, by point mutation or deletion, can be detected by any of the means discussed herein.

There are several methods that can be used to detect DNA sequence variation. Direct DNA sequencing, either manual sequencing or automated fluorescent sequencing can detect sequence variation. For a gene as large as BRCA1, manual sequencing is very labor-intensive, but under optimal conditions, mutations in the coding sequence of a gene are rarely missed. Another approach is the single-stranded conformation polymorphism assay (SSCA) (Orita *et al*., 1989). This method does not detect all sequence changes, especially if the DNA fragment size is greater than 200 bp, but can be optimized to detect most DNA sequence variation. The reduced detection sensitivity is a disadvantage, but the increased throughput possible with SSCA makes it an attractive, viable alternative to direct sequencing for mutation detection on a research basis. The fragments which have shifted mobility on SSCA gels are then sequenced to determine the exact nature of the DNA sequence variation. Other approaches based on the detection of mismatches between the two complementary DNA strands include clamped denaturing gel electrophoresis (CDGE) (Sheffield *et al*., 1991), heteroduplex analysis (HA) (White *et al.,* 1992) and chemical mismatch cleavage (CMC) (Grompe *et al*., 1989). None of the methods described above will detect large deletions, duplications or insertions, nor will they detect a regulatory mutation which affects transcription or translation of the protein. Other methods which might detect these classes of mutations such as a protein truncation assay or the asymmetric assay, detect only specific types of mutations and would not detect missense mutations. A review of currently available methods of detecting DNA sequence variation can be found in a recent review by Grompe (1993). Once a mutation is known, an allele specific detection approach such as allele specific oligonucleotide (ASO) hybridization can be utilized to rapidly screen large numbers of other samples for that same mutation.

In order to detect the alteration of the wild-type BRCA1 gene in a tissue, it is helpful to isolate the tissue free from surrounding normal tissues. Means for enriching tissue preparation for tumor cells are known in the art. For example, the tissue may be isolated from paraffin or cryostat sections. Cancer cells may also be separated from normal cells by flow cytometry. These techniques, as well as other techniques for separating tumor cells from normal cells, are well known in the art. If the tumor tissue is highly contaminated with normal cells, detection of mutations is more difficult.

A rapid preliminary analysis to detect polymorphisms in DNA sequences can be performed by looking at a series of Southern blots of DNA cut with one or more restriction enzymes, preferably with a large number of restriction enzymes. Each blot contains a series of normal individuals and a series of cancer cases, tumors, or both. Southern blots displaying hybridizing fragments (differing in length from control DNA when probed with sequences near or including the BRCA1 locus) indicate a possible mutation. If restriction enzymes which produce very large restriction fragments are used, then pulsed field gel electrophoresis (PFGE) is employed.

Detection of point mutations may be accomplished by molecular cloning of the BRCA1 allele(s) and sequencing the allele(s) using techniques well known in the art. Alternatively, the gene sequences can be amplified directly from a genomic DNA preparation from the tumor tissue, using known techniques. The DNA sequence of the amplified sequences can then be determined.

There are six well known methods for a more complete, yet still indirect, test for confirming the presence of a susceptibility allele: 1) single stranded conformation analysis (SSCA) (Orita *et al.,* 1989); 2) denaturing gradient gel electrophoresis (DGGE) (Wartell *et al.,* 1990; Sheffield *et al.,* 1989); 3) RNase protection assays (Finkelstein *et al.,* 1990; Kinszler *et al.,* 1991); 4) allele-specific oligonucleotides (ASOs) (Conner *et al.,* 1983); 5) the use of proteins which recognize nucleotide mismatches, such as the *E. coli* mutS protein (Modrich, 1991); and 6) allele-specific PCR (Rano & Kidd, 1989). For allele-specific PCR, primers are used which hybridize at their 3' ends to a particular BRCA1 mutation. If the particular BRCA1 mutation is not present, an amplification product is not observed. Amplification Refractory Mutation System (ARMS) can also be used, as disclosed in European Patent Application Publication No. 0332435 and in Newton *et al.,* 1989. Insertions and deletions of genes can also be detected by cloning, sequencing and amplification. In addition, restriction fragment length polymorphism (RFLP) probes for the gene or surrounding marker genes can be used to score alteration of an allele or an insertion in a polymorphic fragment. Such a method is particularly useful for screening relatives of an affected individual for the presence of the BRCA1 mutation found in that individual. Other techniques for detecting insertions and deletions as known in the art can be used.

In the first three methods (SSCA, DGGE and RNase protection assay), a new electrophoretic band appears. SSCA detects a band which migrates differentially because the sequence change causes a difference in single-strand, intramolecular base pairing. RNase protection involves cleavage of the mutant polynucleotide into two or more smaller fragments. DGGE detects differences in migration rates of mutant sequences compared to wild-type sequences, using a denaturing gradient gel. In an allele-specific oligonucleotide assay, an oligonucleotide is designed which detects a specific sequence, and the assay is performed by detecting the presence or absence of a hybridization signal. In the mutS assay, the protein binds only to sequences that contain a nucleotide mismatch in a heteroduplex between mutant and wild-type sequences.

Mismatches are hybridized nucleic acid duplexes in which the two strands are not 100% complementary. Lack of total homology may be due to deletions, insertions, inversions or substitutions. Mismatch detection can be used to detect point mutations in the gene or in its mRNA product. While these techniques are less sensitive than sequencing, they are simpler to perform on a large number of tumor samples. An example of a mismatch cleavage technique is the RNase protection method. In the practice of the present invention, the method involves the use of a labeled riboprobe which is complementary to the human wild-type BRCA1 gene coding sequence. The riboprobe and either mRNA or DNA isolated from the tumor tissue are annealed (hybridized) together and subsequently digested with the enzyme RNase A which is able to detect some mismatches in a duplex RNA structure. If a mismatch is detected by RNase A, it cleaves at the site of the mismatch. Thus, when the annealed RNA preparation is separated on an electrophoretic gel matrix, if a mismatch has been detected and cleaved by RNase A, an RNA product will be seen which is smaller than the full length duplex RNA for the riboprobe and the mRNA or DNA. The riboprobe need not be the full length of the BRCA1 mRNA or gene but can be a segment of either. If the riboprobe comprises only a segment of the BRCA1 mRNA or gene, it will be desirable to use a number of these probes to screen the whole mRNA sequence for mismatches.

In similar fashion, DNA probes can be used to detect mismatches, through enzymatic or chemical cleavage. See, e.g., Cotton *et al*., 1988; Shenk *et al*., 1975; Novack *et al.,* 1986. Alternatively, mismatches can be detected by shifts in the electrophoretic mobility of mismatched duplexes relative to matched duplexes. See, e.g., Cariello, 1988. With either riboprobes or DNA probes, the cellular mRNA or DNA which might contain a mutation can be amplified using PCR (see below) before hybridization. Changes in DNA of the BRCA1 gene can also be detected using Southern hybridization, especially if the changes are gross rearrangements, such as deletions and insertions.

DNA sequences of the BRCA1 gene which have been amplified by use of PCR may also be screened using allele-specific probes. These probes are nucleic acid oligomers, each of which contains a region of the BRCA1 gene sequence harboring a known mutation. For example, one oligomer may be about 30 nucleotides in length, corresponding to a portion of the BRCA1 gene sequence. By use of a battery of such allele-specific probes, PCR amplification products can be screened to identify the presence of a previously identified mutation in the BRCA1 gene. Hybridization of allele-specific probes with amplified BRCA1 sequences can be performed, for example, on a nylon filter. Hybridization to a particular probe under stringent hybridization conditions indicates the presence of the same mutation in the tumor tissue as in the allele-specific probe.

The most definitive test for mutations in a candidate locus is to directly compare genomic BRCA1 sequences from cancer patients with those from a control population. Alternatively, one could sequence messenger RNA alter amplification, e.g., by PCR, thereby eliminating the necessity of determining the exon structure of the candidate gene.

Mutations from cancer patients falling outside the coding region of BRCA1 can be detected by examining the non-coding regions, such as introns and regulatory sequences near or within the BRCA1 gene. An early indication that mutations in noncoding regions are important may come from Northern blot experiments that reveal messenger RNA molecules of abnormal size or abundance in cancer patients as compared to control individuals.

Alteration of BRCA1 mRNA expression can be detected by any techniques known in the art. These include Northern blot analysis, PCR amplification and RNase protection. Diminished mRNA expression indicates an alteration of the wild-type BRCA1 gene. Alteration of wild-type BRCA1 genes can also be detected by screening for alteration of wild-type BRCA1 protein. For example, monoclonal antibodies immunoreactive with BRCA1 can be used to screen a tissue. Lack of cognate antigen would indicate a BRCA1 mutation. Antibodies specific for products of mutant alleles could also be used to detect mutant BRCA1 gene product. Such immunological assays can be done in any convenient formats known in the art. These include Western blots, immunohistochemical assays and ELISA assays. Any means for detecting an altered BRCA1 protein can be used to detect alteration of wild-type BRCA1 genes. Functional assays, such as protein binding determinations, can be used. In addition, assays can be used which detect BRCA1 biochemical function. Finding a mutant BRCA1 gene product indicates alteration of a wild-type BRCA1 gene.

Mutant BRCA1 genes or gene products can also be detected in other human body samples, such as serum, stool, urine and sputum. The same techniques discussed above for detection of mutant BRCA1 genes or gene products in tissues can be applied to other body samples. Cancer cells are sloughed off from tumors and appear in such body samples. In addition, the BRCA1 gene product itself may be secreted into the extracellular space and found in these body samples even in the absence of cancer cells. By screening such body samples, a simple early diagnosis can be achieved for many types of cancers. In addition, the progress of chemotherapy or radiotherapy can be monitored more easily by testing such body samples for mutant BRCA1 genes or gene products.

The methods of diagnosis of the present invention are applicable to any tumor in which the mutation 185delAG in BRCA1 has a role in tumorigenesis. The diagnostic method of the present invention is useful for clinicians, so they can decide upon an appropriate course of treatment.

The primer pairs of the present disclosure are useful for determination of the nucleotide sequence of a particular BRCA1 allele using PCR. The pairs of single-stranded DNA primers can be annealed to sequences within or surrounding the BRCA1 gene on chromosome 17q21 in order to prime amplifying DNA synthesis of the BRCA1 gene itself. A complete set of these primers allows synthesis of all of the nucleotides of the BRCA1 gene coding sequences, i.e., the exons. The set of primers preferably allows synthesis of both intron and exon sequences. Allele-specific primers can also be used. Such primers anneal only to particular BRCA1 mutant alleles, and thus will only amplify a product in the presence of the mutant allele as a template.

In order to facilitate subsequent cloning of amplified sequences, primers may have restriction enzyme site sequences appended to their 5' ends. Thus, all nucleotides of the primers are derived from BRCA1 sequences or sequences adjacent to BRCA1, except for the few nucleotides necessary to form a restriction enzyme site. Such enzymes and sites are well known in the art. The primers themselves can be synthesized using techniques which are well known in the art. Generally, the primers can be made using oligonucleotide synthesizing machines which are commercially available. Given the sequence of the BRCA1 open reading frame shown in SEQ ID NO:1, design of particular primers is well within the skill of the art.

The nucleic acid probes provided by the present invention are useful for a number of purposes. They can be used in Southern hybridization to genomic DNA and in the RNase protection method for detecting point mutations already discussed above. The probes can be used to detect PCR amplification products. They may also be used to detect mismatches with the BRCA1 gene or mRNA using other techniques.

It has been discovered that individuals with the wild-type BRCA1 gene do not have cancer which results from the BRCA1 allele. However, mutations which interfere with the function of the BRCA1 protein are involved in the pathogenesis of cancer. Thus, the presence of an altered (or a mutant) BRCA1 gene which produces a protein having a loss of function, or altered function, directly correlates to an increased risk of cancer. In order to detect a BRCA1 gene mutation, a biological sample is prepared and analyzed for a difference between the sequence of the BRCA1 allele being analyzed and the sequence of the wild-type BRCA1 allele. Mutant BRCA1 alleles can be initially identified by any of the techniques described above. The mutant alleles are then sequenced to identify the specific mutation of the particular mutant allele. Alternatively, mutant BRCA1 alleles can be initially identified by identifying mutant (altered) BRCA1 proteins, using conventional techniques. The mutant alleles are then sequenced to identify the specific mutation for each allele. The 185delAG mutation which leads to an altered function of the BRCA1 protein, is then used for the diagnostic and prognostic methods of the present invention.

### Definitions

The present invention employs the following definitions:

"**Amplification of Polynucleotides**" utilizes methods such as the polymerase chain reaction (PCR), ligation amplification (or ligase chain reaction, LCR) and amplification methods based on the use of Q-beta replicase. These methods are well known and widely practiced in the art. See, e.g., U.S. Patents 4,683,195 and 4,683,202 and Innis *et al.,* 1990 (for PCR); and Wu *et al*., 1989a (for LCR). Reagents and hardware for conducting PCR are commercially available. Primers useful to amplify sequences from the BRCA1 region are preferably complementary to, and hybridize specifically to sequences in the BRCA1 region or in regions that flank a target region therein. BRCA1 sequences generated by amplification may be sequenced directly. Alternatively, but less desirably, the amplified sequence(s) may be cloned prior to sequence analysis. A method for the direct cloning and sequence analysis of enzymatically amplified genomic segments has been described by Scharf, 1986.

"**Analyte polynucleotide**" and "**analyte strand**" refer to a single- or double-stranded polynucleotide which is suspected of containing a target sequence, and which may be present in a variety of types of samples, including biological samples.

"**Binding partner**" refers to a molecule capable of binding a ligand molecule with high specificity, as for example, an antigen and an antigen-specific antibody or an enzyme and its inhibitor. In general, the specific binding partners must bind with sufficient affinity to immobilize the analyte copy/complementary strand duplex (in the case of polynucleotide hybridization) under the isolation conditions. Specific binding partners are known in the art and include, for example, biotin and avidin or streptavidin, IgG and protein A, the numerous, known receptor-ligand couples, and complementary polynucleotide strands. In the case of complementary polynucleotide binding partners, the partners are normally at least about 15 bases in length, and may be at least 40 bases in length. The polynucleotides may be composed of DNA, RNA, or synthetic nucleotide analogs.

A **"biological sample"** refers to a sample of tissue or fluid suspected of containing an analyte polynucleotide or polypeptide from an individual including, but not limited to, e.g., plasma, serum, spinal fluid, lymph fluid, the external sections of the skin, respiratory, intestinal, and genitourinary tracts, tears, saliva, blood cells, tumors, organs, tissue and samples of *in vitro* cell culture constituents.

As used herein, the terms **"diagnosing"** or **"prognosing,"** as used in the context of neoplasia, are used to indicate 1) the classification of lesions as neoplasia, 2) the determination of the severity of the neoplasia, or 3) the monitoring of the disease progression, prior to, during and after treatment.

**"Encode".** A polynucleotide is said to "encode" a polypeptide if, in its native state or when manipulated by methods well known to those skilled in the art, it can be transcribed and/or translated to produce the mRNA for and/or the polypeptide or a fragment thereof. The anti-sense strand is the complement of such a nucleic acid, and the encoding sequence can be deduced therefrom.

**"Isolated"** or **"substantially pure".** An "isolated" or "substantially pure" nucleic acid (e.g., an RNA, DNA or a mixed polymer) is one which is substantially separated from other cellular components which naturally accompany a native human sequence or protein, e.g., ribosomes, polymerases, many other human genome sequences and proteins. The term embraces a nucleic acid sequence or protein which has been removed from its naturally occurring environment, and includes recombinant or cloned DNA isolates and chemically synthesized analogs or analogs biologically synthesized by heterologous systems.

**"BRCA1 Allele"** refers to normal alleles of the BRCA1 locus as well as alleles carrying variations that predispose individuals to develop cancer of many sites including, for example, breast, ovarian, colorectal and prostate cancer. Such predisposing alleles are also called "**BRCA1 susceptibility alleles**".

"**BRCA1 Locus,**" "**BRCA1 Gene,**" "**BRCA1 Nucleic Acids**" or "**BRCA1 Polynucleotide**" each refer to polynucleotides, all of which are in the BRCA1 region, that are likely to be expressed in normal tissue, certain alleles of which predispose an individual to develop breast, ovarian, colorectal and prostate cancers. Mutations at the BRCA1 locus may be involved in the initiation and/or progression of other types of tumors. The locus is indicated in part by mutations that predispose individuals to develop cancer. These mutations fall within the BRCA1 region described *infra*. The BRCA1 locus is intended to include coding sequences, intervening sequences and regulatory elements controlling transcription and/or translation. The BRCA1 locus is intended to include all allelic variations of the DNA sequence.

These terms, when applied to a nucleic acid, refer to a nucleic acid which encodes a BRCA1 polypeptide, fragment, homolog or variant, including, e.g., protein fusions or deletions. The nucleic acids of the present disclosure will possess a sequence which is either derived from, or substantially similar to a natural BRCA1-encoding gene or one having substantial homology with a natural BRCA1-encoding gene or a portion thereof. The coding sequence for a BRCA1 polypeptide is shown in SEQ ID NO:1, with the amino acid sequence shown in SEQ ID NO:2.

The polynucleotide compositions of this disclosure include RNA, cDNA, genomic DNA, synthetic forms, and mixed polymers, both sense and antisense strands, and may be chemically or biochemically modified or may contain non-natural or derivatized nucleotide bases, as will be readily appreciated by those skilled in the art. Such modifications include, for example, labels, methylation, substitution of one or more of the naturally occurring nucleotides with an analog, internucleotide modifications such as uncharged linkages (e.g., methyl phosphonates, phosphotriesters, phosphoamidates, carbamates, etc.), charged linkages (e.g., phosphorothioates, phosphorodithioates, etc.), pendent moieties (e.g., polypeptides), intercalators (e.g., acridine, psoralen, etc.), chelators, alkylators, and modified linkages (e.g., alpha anomeric nucleic acids, etc.). Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule.

The present invention provides recombinant nucleic acids which are replicative cloning vectors comprising said nucleic acid probes of the BRCA1 region. The recombinant construct may be capable of replicating autonomously in a host cell. Alternatively, the recombinant construct may become integrated into the chromosomal DNA of the host cell. Such a recombinant polynucleotide comprises a polynucleotide of genomic, cDNA, semisynthetic, or synthetic origin which, by virtue of its origin or manipulation, 1) is not associated with all or a portion of a polynucleotide with which it is associated in nature; 2) is linked to a polynucleotide other than that to which it is linked in nature; or 3) does not occur in nature.

Therefore, recombinant nucleic acids comprising sequences otherwise not naturally occurring are provided by this invention. Although the wild-type sequence may be employed, it will often be altered, e.g., by deletion, substitution or insertion.

cDNA or genomic libraries of various types may be screened as natural sources of the nucleic acids of the present disclosure, or such nucleic acids may be provided by amplification of sequences resident in genomic DNA or other natural sources, e.g., by PCR. The choice of cDNA libraries normally corresponds to a tissue source which is abundant in mRNA for the desired proteins. Phage libraries are normally preferred, but other types of libraries may be used. Clones of a library are spread onto plates, transferred to a substrate for screening, denatured and probed for the presence of desired sequences.

The DNA sequences used in this invention will usually comprise at least about five codons (15 nucleotides), more usually at least about 7-15 codons, and most preferably, at least about 35 codons. One or more introns may also be present. This number of nucleotides is usually about the minimal length required for a successful probe that would hybridize specifically with a BRCA1-encoding sequence.

Techniques for nucleic acid manipulation are described generally, for example, in Sambrook *et al*., 1989 or Ausubel *et al*., 1992. Reagents useful in applying such techniques, such as restriction enzymes and the like, are widely known in the art and commercially available from such vendors as New England BioLabs, Boehringer Mannheim, Amersham, Promega Biotec, U. S. Biochemicals, New England Nuclear, and a number of other sources.

**"BRCA1 Region"** refers to a portion of human chromosome 17q21 bounded by the markers tdj 1474 and U5R. This region contains the BRCA1 locus, including the BRCA1 gene.

As used herein, the terms **"BRCA1 locus," "BRCA1 allele"** and **"BRCA1 region"** all refer to the double-stranded DNA comprising the locus, allele, or region, as well as either of the single-stranded DNAs comprising the locus, allele or region.

As used herein, a "**portion**" of the BRCA1 locus or region or allele is defined as having a minimal size of at least about eight nucleotides, or preferably about 15 nucleotides, or more preferably at least about 25 nucleotides, and may have a minimal size of at least about 40 nucleotides.

**"Operably linked"** refers to a juxtaposition wherein the components so described are in a relationship permitting them to function in their intended manner. For instance, a promoter is operably linked to a coding sequence if the promoter affects its transcription or expression.

**"Probes"**. Polynucleotide polymorphisms associated with BRCA1 alleles which predispose to certain cancers or are associated with most cancers are detected by hybridization with a polynucleotide probe which forms a stable hybrid with that of the target sequence, under stringent to moderately stringent hybridization and wash conditions. If it is expected that the probes will be perfectly complementary to the target sequence, stringent conditions will be used. Hybridization stringency may be lessened if some mismatching is expected, for example, if variants are expected with the result that the probe will not be completely complementary. Conditions are chosen which rule out nonspecific/adventitious bindings, that is, which minimize noise. Since such indications identify neutral DNA polymorphisms as well as mutations, these indications need further analysis to demonstrate detection of a BRCA1 susceptibility allele.

Probes for BRCA1 alleles may be derived from the sequences of the BRCA1 region or its cDNAs. The 185delAG probes will have 15 to 30 nucleotides, which span a portion of the BRCA1 region, and which allow specific hybridization to the BRCA1 region. If the target sequence contains a sequence identical to that of the probe, the hybrid will be relatively stable under even stringent conditions. If some degree of mismatch is expected with the probe, i.e., if it is suspected that the probe will hybridize to a variant region, a longer probe may be employed which hybridizes to the target sequence with the requisite specificity.

The probes will include an isolated polynucleotide attached to a label or reporter molecule and may be used to isolate other polynucleotide sequences, having sequence similarity by standard methods. For techniques for preparing and labeling probes see, e.g., Sambrook *et al.,* 1989 or Ausubel *et al.,* 1992. Other similar polynucleotides may be selected by using homologous polynucleotides. Alternatively, polynucleotides encoding these or similar polypeptides may be synthesized or selected by use of the redundancy in the genetic code. Various codon substitutions may be introduced, e.g., by silent changes (thereby producing various restriction sites) or to optimize expression for a particular system. Mutations may be introduced to modify the properties of the polypeptide, perhaps to change ligand-binding affinities, interchain affinities, or the polypeptide degradation or turnover rate.

Probes of the present invention comprising synthetic oligonucleotides or other polynucleotides may be derived from naturally occurring or recombinant single- or double-stranded polynucleotides, or be chemically synthesized. Probes may also be labeled by nick translation, Klenow fill-in reaction, or other methods known in the art.

Portions of the polynucleotide sequence having 15 to 30 nucleotides, from a polynucleotide sequence encoding BRCA1 are preferred as 185delAG probes. The probes may also be used to determine whether mRNA encoding BRCA1 is present in a cell or tissue.

**"Recombinant nucleic acid"** is a nucleic acid which is not naturally occurring, or which is made by the artificial combination of two otherwise separated segments of sequence. This artificial combination is often accomplished by either chemical synthesis means, or by the artificial manipulation of isolated segments of nucleic acids, e.g., by genetic engineering techniques. Such is usually done to replace a codon with a redundant codon encoding the same or a conservative amino acid, while typically introducing or removing a sequence recognition site. Alternatively, it is -performed to join together nucleic acid segments of desired functions to generate a desired combination of functions.

"**Regulatory sequences**" refers to those sequences normally within 100 kb of the coding region of a locus, but they may also be more distant from the coding region, which affect the expression of the gene (including transcription of the gene, and translation, splicing, stability or the like of the messenger RNA).

**"Substantial homology or similarity".** A nucleic acid or fragment thereof is "substantially homologous" ("or substantially similar") to another if, when optimally aligned (with appropriate nucleotide insertions or deletions) with the other nucleic acid (or its complementary strand), there is nucleotide sequence identity in at least about 60% of the nucleotide bases, usually at least about 70%, more usually at least about 80%, preferably at least about 90%, and more preferably at least about 95-98% of the nucleotide bases.

Alternatively, substantial homology or (similarity) exists when a nucleic acid or fragment thereof will hybridize to another nucleic acid (or a complementary strand thereof) under selective hybridization conditions, to a strand, or to its complement. Selectivity of hybridization exists when hybridization which is substantially more selective than total lack of specificity occurs. Typically, selective hybridization will occur when there is at least about 55% homology over a stretch of at least about 14 nucleotides, preferably at least about 65%, more preferably at least about 75%, and most preferably at least about 90%. See, Kanehisa, 1984. The length of homology comparison, as described, may be over longer stretches, and in certain embodiments will often be over a stretch of at least about nine nucleotides, usually at least about 20 nucleotides, more usually at least about 24 nucleotides, typically at least about 28 nucleotides, more typically at least about 32 nucleotides, and preferably at least about 36 or more nucleotides.

Nucleic acid hybridization will be affected by such conditions as salt concentration, temperature, or organic solvents, in addition to the base composition, length of the complementary strands, and the number of nucleotide base mismatches between the hybridizing nucleic acids, as will be readily appreciated by those skilled in the art. Stringent temperature conditions will generally include temperatures in excess of 30°C, typically in excess of 37°C, and preferably in excess of 45°C. Stringent salt conditions will ordinarily be less than 1000 mM, typically less than 500 mM, and preferably less than 200 mM. However, the combination of parameters is much more important than the measure of any single parameter. See, e.g., Wetmur & Davidson, 1968.

Probe sequences may also hybridize specifically to duplex DNA under certain conditions to form triplex or other higher order DNA complexes. The preparation of such probes and suitable hybridization conditions are well known in the art.

The terms "**substantial homology**" or "**substantial identity**", when referring to polypeptides, indicate that the polypeptide or protein in question exhibits at least about 30% identity with an entire naturally-occurring protein or a portion thereof, usually at least about 70% identity, and preferably at least about 95% identity.

**"Substantially similar function"** refers to the function of a modified nucleic acid or a modified protein, with reference to the wild-type BRCA1 nucleic acid or wild-type BRCA1 polypeptide. The modified polypeptide will be substantially homologous to the wild-type BRCA1 polypeptide and will have substantially the same function. The modified polypeptide may have an altered amino acid sequence and/or may contain modified amino acids. In addition to the similarity of function, the modified polypeptide may have other useful properties, such as a longer half-life. The similarity of function (activity) of the modified polypeptide may be substantially the same as the activity of the wild-type BRCA1 polypeptide. Alternatively, the similarity of function (activity) of the modified polypeptide may be higher than the activity of the wild-type BRCA1 polypeptide. The modified polypeptide is synthesized using conventional techniques, or is encoded by a modified nucleic acid and produced using conventional techniques. The modified nucleic acid is prepared by conventional techniques. A nucleic acid with a function substantially similar to the wild-type BRCA1 gene function produces the modified protein described above.

Homology, for polypeptides, is typically measured using sequence analysis software. See, e.g., the Sequence Analysis Software Package of the Genetics Computer Group, University of Wisconsin Biotechnology Center, 910 University Avenue, Madison, Wisconsin 53705. Protein analysis software matches similar sequences using measure of homology assigned to various substitutions, deletions and other modifications. Conservative substitutions typically include substitutions within the following groups: glycine, alanine; valine, isoleucine, leucine; aspartic acid, glutamic acid; asparagine, glutamine; serine, threonine; lysine, arginine; and phenylalanine, tyrosine.

A polypeptide **"fragment," "portion"** or **"segment"** is a stretch of amino acid residues of at least about five to seven contiguous amino acids, often at least about seven to nine contiguous amino acids, typically at least about nine to 13 contiguous amino acids and, most preferably, at least about 20 to 30 or more contiguous amino acids.

The polypeptides, if soluble, may be coupled to a solid-phase support, e.g., nitrocellulose, nylon, column packing materials (e.g., Sepharose beads), magnetic beads, glass wool, plastic, metal, polymer gels, cells, or other substrates. Such supports may take the form, for example, of beads, wells, dipsticks, or membranes.

"**Target region**" refers to a region of the nucleic acid which is amplified and/or detected. The term "**target sequence**" refers to a sequence with which a probe or primer will form a stable hybrid under desired conditions.

The practice of the present invention employs, unless otherwise indicated, conventional techniques of chemistry, molecular biology, microbiology, recombinant DNA, genetics, and immunology. See, e.g., Maniatis *et al.,* 1982; Sambrook *et al.,* 1989; Ausubel *et al.,* 1992; Glover, 1985; Anand, 1992; Guthrie & Fink, 1991. A general discussion of techniques and materials for human gene mapping, including mapping of human chromosome 17q, is provided, e.g., in White and Lalouel, 1988.

### Preparation of recombinant or chemically synthesized nucleic acids; vectors, transformation, host cells

Large amounts of the probes of the present invention may be produced by replication in a suitable host cell. Natural or synthetic polynucleotide fragments will be incorporated into recombinant polynucleotide constructs, usually DNA constructs, capable of introduction into and replication in a prokaryotic or eukaryotic cell. Usually the polynucleotide constructs will be suitable for replication in a unicellular host, such as yeast or bacteria, but may also be intended for introduction to (with and without integration within the genome) cultured mammalian or plant or other eukaryotic cell lines. The purification of nucleic acids produced by the methods of the present invention is described, e.g., in Sambrook *et al.,* 1989 or Ausubel *et al.,* 1992.

The probes of the present invention may also be produced by chemical synthesis, e.g., by the phosphoramidite method described by Beaucage & Carruthers, 1981 or the triester method according to Matteucci and Caruthers, 1981, and may be performed on commercial, automated oligonucleotide synthesizers. A double-stranded fragment may be obtained from the single-stranded product of chemical synthesis either by synthesizing the complementary strand and annealing the strands together under appropriate conditions or by adding the complementary strand using DNA polymerase with an appropriate primer sequence.

Polynucleotide constructs prepared for introduction into a prokaryotic or eukaryotic host may comprise a replication system recognized by the host, including the intended polynucleotide fragment encoding the desired polypeptide, and will preferably also include transcription and translational initiation regulatory sequences operably linked to the polypeptide encoding segment. Expression vectors may include, for example, an origin of replication or autonomously replicating sequence (ARS) and expression control sequences, a promoter, an enhancer and necessary processing information sites, such as ribosome-binding sites, RNA splice sites, polyadenylation sites, transcriptional terminator sequences, and mRNA stabilizing sequences. Secretion signals may also be included where appropriate, whether from a native BRCA1 protein or from other receptors or from secreted polypeptides of the same or related species, which allow the protein to cross and/or lodge in cell membranes, and thus attain its functional topology, or be secreted from the cell. Such vectors may be prepared by means of standard recombinant techniques well known in the art and discussed, for example, in Sambrook *et al*., 1989 or Ausubel *et al.* 1992.

An appropriate promoter and other necessary vector sequences will be selected so as to be functional in the host, and may include, when appropriate, those naturally associated with BRCA1 genes. Examples of workable combinations of cell lines and expression vectors are described in Sambrook *et al.,* 1989 or Ausubel *et al.,* 1992; see also, e.g., Metzger *et al.,* 1988. Many useful vectors are known in the art and may be obtained from such vendors as Stratagene, New England Biolabs, Promega Biotech, and others. Promoters such as the trp, lac and phage promoters, tRNA promoters and glycolytic enzyme promoters may be used in prokaryotic hosts. Useful yeast promoters include promoter regions for metallothionein, 3-phosphoglycerate kinase or other glycolytic enzymes such as enolase or glyceraldehyde-3-phosphate dehydrogenase, enzymes responsible for maltose and galactose utilization, and others. Vectors and promoters suitable for use in yeast expression are further described in Hitzeman *et al*., EP 73,675A. Appropriate non-native mammalian promoters might include the early and late promoters from SV40 (Fiers *et al.,* 1978) or promoters derived from murine Moloney leukemia virus, mouse tumor virus, avian sarcoma viruses, adenovirus II, bovine papilloma virus or polyoma. In addition, the construct may be joined to an amplifiable gene (e.g., DHFR) so that multiple copies of the gene may be made. For appropriate enhancer and other expression control sequences, see also Enhancers and Eukaryotic Gene Expression, Cold Spring Harbor Press, Cold Spring Harbor, New York (1983).

While such expression vectors may replicate autonomously, they may also replicate by being inserted into the genome of the host cell, by methods well known in the art.

Expression and cloning vectors will likely contain a selectable marker, a gene encoding a protein necessary for survival or growth of a host cell transformed with the vector. The presence of this gene ensures growth of only those host cells which express the inserts. Typical selection genes encode proteins that a) confer resistance to antibiotics or other toxic substances, e.g. ampicillin, neomycin, methotrexate, etc.; b) complement auxotrophic deficiencies, or c) supply critical nutrients not available from complex media, e.g., the gene encoding D-alanine racemase for *Bacilli.* The choice of the proper selectable marker will depend on the host cell, and appropriate markers for different hosts are well known in the art.

The vectors containing the nucleic acids of interest can be transcribed *in vitro,* and the resulting RNA introduced into the host cell by well-known methods, e.g., by injection (see, Kubo *et al.,* 1988), or the vectors can be introduced directly into host cells by methods well known in the art, which vary depending on the type of cellular host, including electroporation; transfection employing calcium chloride, rubidium chloride, calcium phosphate, DEAE-dextran, or other substances; microprojectile bombardment; lipofection; infection (where the vector is an infectious agent, such as a retroviral genome); and other methods. See generally, Sambrook *et al.,* 1989 and Ausubel *et al*., 1992. The introduction of the polynucleotides into the host cell by any method known in the art, including, *inter alia*, those described above, will be referred to herein as "transformation." The cells into which have been introduced nucleic acids described above are meant to also include the progeny of such cells.

Large quantities of the nucleic acids of the present invention may be prepared by expressing the BRCA1 nucleic acids portions in vectors or other expression vehicles in compatible prokaryotic or eukaryotic host cells. The most commonly used prokaryotic hosts are strains of *Escherichia coli,* although other prokaryotes, such as *Bacillus subtilis* or *Pseudomonas* may also be used.

Clones are selected by using markers depending on the mode of the vector construction. The marker may be on the same or a different DNA molecule, preferably the same DNA molecule. In prokaryotic hosts, the transformant may be selected, e.g., by resistance to ampicillin, tetracycline or other antibiotics. Production of a particular product based on temperature sensitivity may also serve as an appropriate marker.

Prokaryotic or eukaryotic cells transformed with the polynucleotides of the present invention will be useful not only for the production of the nucleic acids of the present invention, but also, for example, in studying the characteristics of BRCA1 polypeptides.

Antisense polynucleotide sequences are useful in preventing or diminishing the expression of the BRCA1 locus, as will be appreciated by those skilled in the art. For example, polynucleotide vectors containing all or a portion of the BRCA1 locus or other sequences from the BRCA1 region (particularly those flanking the BRCA1 locus) may be placed under the control of a promoter in an antisense orientation and introduced into a cell. Expression of such an antisense construct within a cell will interfere with BRCA1 transcription and/or translation and/or replication.

The probes and primers based on the BRCA1 gene sequences disclosed herein are used to identify homologous BRCA1 gene sequences and proteins in other species. These BRCA1 gene sequences and proteins are used in the diagnostic/prognostic, therapeutic and drug screening methods described herein for the species from which they have been isolated.

### Methods of Use: Nucleic Acid Diagnosis and Diagnostic Kits

In order to detect the presence of a BRCA1 allele predisposing an individual to cancer, a biological sample such as blood is prepared and analyzed for the presence or absence of susceptibility alleles of BRCA1. In order to detect the presence of neoplasia, the progression toward malignancy of a precursor lesion, or as a prognostic indicator, a biological sample of the lesion is prepared and analyzed for the presence or absence of mutant alleles of BRCA1. Results of these tests and interpretive information are returned to the health care provider for communication to the tested individual. Such diagnoses may be performed by diagnostic laboratories, or, alternatively, diagnostic kits are manufactured and sold to health care providers or to private individuals for self-diagnosis.

Initially, the screening method involves amplification of the relevant BRCA1 sequences. In another preferred embodiment of the invention, the screening method involves a non-PCR based strategy. Such screening methods include two-step label amplification methodologies that are well known in the art. Both PCR and non-PCR based screening strategies can detect target sequences with a high level of sensitivity.

The most popular method used today is target amplification. Here, the target nucleic acid sequence is amplified with polymerases. One particularly preferred method using polymerase-driven amplification is the polymerise chain reaction (PCR). The polymerase chain reaction and other polymerase-driven amplification assays can achieve over a million-fold increase in copy number through the use of polymerase-driven amplification cycles. Once amplified, the resulting nucleic acid can be sequenced or used as a substrate for DNA probes.

When the probes are used to detect the presence of the target sequences (for example, in screening for cancer susceptibility), the biological sample to be analyzed, such as blood or serum, may be treated, if desired, to extract the nucleic acids. The sample nucleic acid may be prepared in various ways to facilitate detection of the target sequence; e.g. denaturation, restriction digestion, electrophoresis or dot blotting. The targeted region of the analyte nucleic acid usually must be at least partially single-stranded to form hybrids with the targeting sequence of the probe. If the sequence is naturally single-stranded, denaturation will not be required. However, if the sequence is double-stranded, the sequence will probably need to be denatured. Denaturation can be carried out by various techniques known in the art.

Analyte nucleic acid and probe are incubated under conditions which promote stable hybrid formation of the target sequence in the probe with the putative targeted sequence in the analyte. The region of the probes which is used to bind to the analyte can be made completely complementary to the targeted region of human chromosome 17q. Therefore, high stringency conditions are desirable in order to prevent false positives. However, conditions of high stringency are used only if the probes are complementary to regions of the chromosome which are unique in the genome. The stringency of hybridization is determined by a number of factors during hybridization and during the washing procedure, including temperature, ionic strength, base composition, probe length, and concentration of formamide. These factors are outlined in, for example, Maniatis *et al.,* 1982 and Sambrook *et al*., 1989. Under certain circumstances, the formation of higher order hybrids, such as triplexes, quadraplexes, etc., may be desired to provide the means of detecting target sequences.

Detection, if any, of the resulting hybrid is usually accomplished by the use of labeled probes. Alternatively, the probe may be unlabeled, but may be detectable by specific binding with a ligand which is labeled, either directly or indirectly. Suitable labels, and methods for labeling probes and ligands are known in the art, and include, for example, radioactive labels which may be incorporated by known methods (e.g., nick translation, random priming or kinasing), biotin, fluorescent groups, chemiluminescent groups (e.g., dioxetanes, particularly triggered dioxetanes), enzymes, antibodies and the like. Variations of this basic scheme are known in the art, and include those variations that facilitate separation of the hybrids to be detected from extraneous materials and/or that amplify the signal from the labeled moiety. A number of these variations are reviewed in, e.g., Matthews & Kricka, 1988; Landegren *et al.,* 1988; Mittlin, 1989; U.S. Patent 4,868,105, and in EPO Publication No. 225,807.

As noted above, non-PCR based screening assays are also contemplated in this invention. An exemplary non-PCR based procedure is provided in Example 11. This procedure hybridizes a nucleic acid probe (or an analog such as a methyl phosphonate backbone replacing the normal phosphodiester), to the low level DNA target. This probe may have an enzyme covalently linked to the probe, such that the covalent linkage does not interfere with the specificity of the hybridization. This enzyme-probe-conjugate-target nucleic acid complex can then be isolated away from the free probe enzyme conjugate and a substrate is added for enzyme detection. Enzymatic activity is observed as a change in color development or luminescent output resulting in a 10³-10⁶ increase in sensitivity. For an example relating to the preparation of oligodeoxynucleotide-alkaline phosphatase conjugates and their use as hybridization probes see Jablonski *et al.,* 1986.

Two-step label amplification methodologies are known in the art. These assays work on the principle that a small ligand (such as digoxigenin, biotin, or the like) is attached to a nucleic acid probe capable of specifically binding BRCA1. Exemplary probes are provided in Table 9 of this patent application and additionally include the nucleic acid probe corresponding to nucleotide positions 3631 to 3930 of SEQ ID NO:1. Allele specific probes are also contemplated within the scope of this example and exemplary allele specific probes include probes encompassing the predisposing mutations summarized in Tables 11 and 12 of this patent application.

In one example, the small ligand attached to the nucleic acid probe is specifically recognized by an antibody-enzyme conjugate. In one embodiment of this example, digoxigenin is attached to the nucleic acid probe. Hybridization is detected by an antibody-alkaline phosphatase conjugate which turns over a chemiluminescent substrate. For methods for labeling nucleic acid probes according to this embodiment see Martin *et al.,* 1990. In a second example, the small ligand is recognized by a second ligand-enzyme conjugate that is capable of specifically complexing to the first ligand. A well known embodiment of this example is the biotin-avidin type of interactions. For methods for labeling nucleic acid probes and their use in biotin-avidin based assays see Rigby *et al.,* 1977 and Nguyen *et al.,* 1992.

It is also contemplated within the scope of this invention that the nucleic acid probe of this invention will be employed in a cocktail of nucleic acid probes capable of detecting BRCA1. Thus, in one example to detect the presence of BRCA1 in a cell sample, more than one probe complementary to BRCA1 is employed and in particular the number of different probes is alternatively 2, 3, or 5 different nucleic acid probe sequences. In another example, to detect the presence of mutations in the BRCA1 gene sequence in a patient, more than one probe complementary to BRCA1 is employed where the cocktail includes probes capable of binding to the allele-specific mutations identified in populations of patients with alterations in BRCA1. In this embodiment, any number of probes can be used, and will preferably include probes corresponding to the major gene mutations identified as predisposing an individual to breast cancer. Some candidate probes contemplated within the scope of the present disclosure include probes that include the allele-specific mutations identified in Tables 11 and 12 and those that have the BRCA1 regions corresponding to SEQ ID NO:1 both 5' and 3' to the mutation site.

The disclosure is supported by the following Examples. Standard techniques well known in the art or the techniques specifically described below were utilized.

### EXAMPLE 1

### Ascertain and Study Kindreds Likely to Have a 17q-Linked Breast Cancer Susceptibility Locus

Extensive cancer prone kindreds were ascertained from a defined population providing a large set of extended kindreds with multiple cases of breast cancer and many relatives available to study. The large number of meioses present in these large kindreds provided the power to detect whether the BRCA1 locus was segregating, and increased the opportunity for informative recombinants to occur within the small region being investigated. This vastly improved the chances of establishing linkage to the BRCA1 region, and greatly facilitated the reduction of the BRCA1 region to a manageable size, which permits identification of the BRCA1 locus itself.

Each kindred was extended through all available connecting relatives, and to all informative first degree relatives of each proband or cancer case. For these kindreds, additional breast cancer cases and individuals with cancer at other sites of interest (e.g. ovarian) who also appeared in the kindreds were identified through the tumor registry linked files. All breast cancers reported in the kindred which were not confirmed in the Utah Cancer Registry were researched. Medical records or death certificates were obtained for confirmation of all cancers. Each key connecting individual and all informative individuals were invited to participate by providing a blood sample from which DNA was extracted. We also sampled spouses and relatives of deceased cases so that the genotype of the deceased cases could be inferred from the genotypes of their relatives.

Ten kindreds which had three or more cancer cases with inferable genotypes were selected for linkage studies to 17q markers from a set of 29 kindreds originally ascertained from the linked databases for a study of proliferative breast disease and breast cancer (Skolnick *et al.,* 1990). The criterion for selection of these kindreds was the presence of two sisters or a mother and her daughter with breast cancer. Additionally, two kindreds which have been studied since 1980 as part of our breast cancer linkage studies (K1001, K9018), six kindreds ascertained from the linked databases for the presence of clusters of breast and/or ovarian cancer (K2019, K2073, K2079, K2080, K2039, K2082) and a self-referred kindred with early onset breast cancer (K2035) were included. These kindreds were investigated and expanded in our clinic in the manner described above. Table 1 displays the characteristics of these 19 kindreds which are the subject of subsequent examples. In Table 1, for each kindred the total number of individuals in our database, the number of typed individuals, and the minimum, median, and maximum age at diagnosis of breast/ovarian cancer are reported. Kindreds are sorted in ascending order of median age at diagnosis of breast cancer. Four women diagnosed with both ovarian and breast cancer are counted in both categories.

**TABLE 1**

| Description of the 19 Kindreds | | | | | | | | | | |
|---|---|---|---|---|---|---|---|---|---|---|
| | | | Breast | | | | Ovarian | | | |
| | No. of Individuals | | | Age at Dx | | | | Age at Dx | | |
| KINDRED | Total | Sample | # Aff. | Min. | Med. | Max. | # Aff. | Min. | Med. | Max. |
| | | | | | | | | | | |
| 1910 | 15 | 10 | 4 | 27 | 34 | 49 | - | - | - | - |
| 1001 | 133 | 98 | 13 | 28 | 37 | 64 | - | - | - | - |
| 2035 | 42 | 25 | 8 | 28 | 37 | 45 | 1 | - | 60 | - |
| 2027 | 21 | 11 | 4 | 34 | 38 | 41 | - | - | - | - |
| 9018 | 54 | 17 | 9 | 30 | 40 | 72 | 2 | 46 | 48 | 50 |
| | | | | | | | | | | |
| 1925 | 50 | 27 | 4 | 39 | 42 | 53 | - | - | - | - |
| 1927 | 49 | 29 | 5 | 32 | 42 | 51 | - | - | - | - |
| 1911 | 28 | 21 | 7 | 28 | 42 | 76 | - | - | - | - |
| 1929 | 16 | 11 | 4 | 34 | 43 | 73 | - | - | - | - |
| 1901 | 35 | 19 | 10 | 31 | 44 | 76 | - | - | - | - |
| | | | | | | | | | | |
| 2082 | 180 | 105 | 20 | 27 | 47 | 67 | 10 | 45 | 52 | 66 |
| 2019 | 42 | 19 | 10 | 42 | 53 | 79 | - | - | - | - |
| 1900 | 70 | 23 | 8 | 45 | 55 | 70 | 1 | - | 78 | - |
| 2080 | 264 | 74 | 22+ | 27 | 55 | 92 | 4 | 45 | 53 | 71 |
| 2073 | 57 | 29 | 9 | 35 | 57 | 80 | - | - | - | - |
| | | | | | | | | | | |
| 1917 | 16 | 6 | 4 | 43 | 58 | 61 | - | - | - | - |
| 1920 | 22 | 14 | 3 | 62 | 63 | 68 | - | - | - | - |
| 2079 | 136 | 18 | 14 | 38 | 66 | 84 | 4 | 52 | 59 | 65 |
| 2039 | 87 | 40 | 14 | 44 | 68 | 88 | 4 | 41 | 51 | 75 |

| | | | | | | | | | | |
|---|---|---|---|---|---|---|---|---|---|---|
| + Includes one case of male breast cancer. | | | | | | | | | | |

### EXAMPLE 2

### Selection of Kindreds Which are Linked to Chromosome 17q and Localization of BRCA1 to the Interval Mfd15 - Mfd188

For each sample collected in these 19 kindreds, DNA was extracted from blood (or in two cases from paraffin-embedded tissue blocks) using standard laboratory protocols. Genotyping in this study was restricted to short tandem repeat (STR) markers since, in general, they have high heterozygosity and PCR methods offer rapid turnaround while using very small amounts of DNA. To aid in this effort, four such STR markers on chromosome 17 were developed by screening a chromosome specific cosmid library for CA positive clones. Three of these markers localized to the long arm: (46E6, Easton *et al*., 1993); (42D6, Easton *et al*., 1993); 26C2 (D17S514, Oliphant *et al.,* 1991), while the other, 12G6 (D17S513, Oliphant *et al*., 1991), localized to the short arm near the p53 tumor suppressor locus. Two of these, 42D6 and 46E6, were submitted to the Breast Cancer Linkage Consortium for typing of breast cancer families by investigators worldwide. Oligonucleotide sequences for markers not developed in our laboratory were obtained from published reports, or as part of the Breast Cancer Linkage Consortium, or from other investigators. All genotyping films were scored blindly with a standard lane marker used to maintain consistent coding of alleles. Key samples in the four kindreds presented here underwent duplicate typing for all relevant markers. All 19 kindreds have been typed for two polymorphic CA repeat markers: 42D6 (D17S588), a CA repeat isolated in our laboratory, and Mfd15 (D17S250), a CA repeat provided by J. Weber (Weber *et al.,* 1990). Several sources of probes were used to create genetic markers on chromosome 17, specifically chromosome 17 cosmid and lambda phage libraries created from sorted chromosomes by the Los Alamos National Laboratories (van Dilla *et al.,* 1986).

LOD scores for each kindred with these two markers (42D6, Mfd15) and a third marker, Mfd188 (D17S579, Hall *et al.,* 1992), located roughly midway between these two markers, were calculated for two values of the recombination fraction, 0.001 and 0.1. (For calculation of LOD scores, *see* Oh, 1985). Likelihoods were computed under the model derived by Claus *et al.,* 1991, which assumes an estimated gene frequency of 0.003, a lifetime risk in gene carriers of about 0.80, and population based age-specific risks for breast cancer in non-gene carriers. Allele frequencies for the three markers used for the LOD score calculations were calculated from our own laboratory typings of unrelated individuals in the CEPH panel (White and Lalouel, 1988). Table 2 shows the results of the pairwise linkage analysis of each kindred with the three markers 42D6, Mfd188, and Mfd15.

**TABLE 2**

| Pairwise Linkage Analysis of Kindreds | | | | | | |
|---|---|---|---|---|---|---|
| | Mfd15 (D17S250) Recombination | | Mfd188 (D17S579) Recombination | | 42D6 (D17S588) Recombination | |
| | 0.001 | 0.1 | 0.001 | 0.1 | 0.001 | 0.1 |

| KINDRED | | | | | | |
|---|---|---|---|---|---|---|
| | | | | | | |
| 1910 | 0.06 | 0.30 | 0.06 | 0.30 | 0.06 | 0.30 |
| 1001 | -0.30 | -0.09 | NT | NT | -0.52 | -0.19 |
| 2035 | 2.34 | 1.85 | 0.94 | 0.90 | 2.34 | 1.82 |
| 2027 | -1.22 | -0.33 | -1.20 | -0.42 | -1.16 | -0.33 |
| 9018 | -0.54 | -0.22 | -0.17 | -0.10 | 0.11 | 0.07 |
| 1925 | 1.08 | 0.79 | 0.55 | 0.38 | -0.11 | -0.07 |
| 1927 | -0.41 | 0.01 | -0.35 | 0.07 | -0.44 | -0.02 |
| | | | | | | |
| 1911 | -0.27 | -0.13 | -0.43 | -0.23 | 0.49 | 0.38 |
| 1929 | -0.49 | -0.25 | NT | NT | -0.49 | -0.25 |
| 1901 | 1.50 | 1.17 | 0.78 | 0.57 | 0.65 | 0.37 |
| 2082 | 4.25 | 3.36 | 6.07 | 5.11 | 2.00 | 3.56 |
| 2019 | -0.10 | -0.01 | -0.11 | -0.05 | -0.18 | -0.10 |
| 1900 | -0.14 | -0.11 | NT | NT | -0.12 | -0.05 |
| | | | | | | |
| 2080 | -0.16 | -0.04 | 0.76 | 0.74 | -1.25 | -0.58 |
| 2073 | -0.41 | -0.29 | 0.63 | 0.49 | -0.23 | -0.13 |
| 1917 | -0.02 | -0.02 | NT | NT | -0.01 | 0.00 |
| 1920 | -0.03 | -0.02 | NT | NT | 0.00 | 0.00 |
| 2079 | 0.02 | 0.01 | -0.01 | -0.01 | 0.01 | 0.01 |
| 2039 | -1.67 | -0.83 | 0.12 | 0.59 | -1.15 | 0.02 |

| | | | | | | |
|---|---|---|---|---|---|---|
| NT - Kindred not typed for Mfd188. | | | | | | |

Using a criterion for linkage to 17q of a LOD score > 1.0 for at least one locus under the CASH model (Claus *et al*., 1991), four of the 19 kindreds appeared to be linked to 17q (K1901, K1925, K2035, K2082). A number of additional kindreds showed some evidence of linkage but at this time could not be definitively assigned to the linked category. These included kindreds K1911, K2073, K2039, and K2080. Three of the 17q-linked kindreds had informative recombinants in this region and these are detailed below.

Kindred 2082 is the largest 17q-linked breast cancer family reported to date by any group. The kindred contains 20 cases of breast cancer, and ten cases of ovarian cancer. Two cases have both ovarian and breast cancer. The evidence of linkage to 17q for this family is overwhelming; the LOD score with the linked haplotype is over 6.0, despite the existence of three cases of breast cancer which appear to be sporadic, i.e., these cases share no part of the linked haplotype between Mfd15 and 42D6. These three sporadic cases were diagnosed with breast cancer at ages 46, 47, and 54. In smaller kindreds, sporadic cancers of this type greatly confound the analysis of linkage and the correct identification of key recombinants. The key recombinant in the 2082 kindred is a woman who developed ovarian cancer at age 45 whose mother and aunt had ovarian cancer at ages 58 and 66, respectively. She inherited the linked portion of the haplotype for both Mfd188 and 42D6 while inheriting unlinked alleles at Mfd15; this recombinant event placed BRCA1 distal to Mfd15.

K1901 is typical of early-onset breast cancer kindreds. The kindred contains 10 cases of breast cancer with a median age at diagnosis of 43.5 years of age; four cases were diagnosed under age 40. The LOD score for this kindred with the marker 42D6 is 1.5, resulting in a posterior probability of 17q-linkage of 0.96. Examination of haplotypes in this kindred identified a recombinant haplotype in an obligate male carrier and his affected daughter who was diagnosed with breast cancer at age 45. Their linked allele for marker Mfd15 differs from that found in all other cases in the kindred (except one case which could not be completely inferred from her children). The two haplotypes are identical for Mfd188 and 42D6. Accordingly, data from Kindred 1901 would also place the BRCA1 locus distal to Mfd15.

Kindred 2035 is similar to K1901 in disease phenotype. The median age of diagnosis for the eight cases of breast cancer in this kindred is 37. One case also had ovarian cancer at age 60. The breast cancer cases in this family descend from two sisters who were both unaffected with breast cancer until their death in the eighth decade. Each branch contains four cases of breast cancer with at least one case in each branch having markedly early onset. This kindred has a LOD score of 2.34 with Mfd15. The haplotypes segregating with breast cancer in the two branches share an identical allele at Mfd15 but differ for the distal loci Mfd188 and NM23 (a marker typed as part of the consortium which is located just distal to 42D6 (Hall *et al.,* 1992)). Although the two haplotypes are concordant for marker 42D6, it is likely that the alleles are shared identical by state (the same allele but derived from different ancestors), rather than identical by descent (derived from a common ancestor) since the shared allele is the second most common allele observed at this locus. By contrast the linked allele shared at Mfd15 has a frequency of 0.04. This is a key recombinant in our dataset as it is the sole recombinant in which BRCA1 segregated with the proximal portion of the haplotype, thus setting the distal boundary to the BRCA1 region. For this event not to be a key recombinant requires that a second mutant BRCA1 gene be present in a spouse marrying into the kindred who also shares the rare Mfd15 allele segregating with breast cancer in both branches of the kindred. This event has a probability of less than one in a thousand. The evidence from this kindred therefore placed the BRCA1 locus proximal to Mfd188.

### EXAMPLE 3

### Creation of a Fine Structure Map and Refinement of the BRCA1 Region to Mfd191-Mfd188 using Additional STR Polymorphisms

In order to improve the characterization of our recombinants and define closer flanking markers, a dense map of this relatively small region on chromosome 17q was required. The chromosome 17 workshop has produced a consensus map of this region (Figure 1) based on a combination of genetic and physical mapping studies (Fain, 1992). This map contains both highly polymorphic STR polymorphisms, and a number of nonpolymorphic expressed genes. Because this map did not give details on the evidence for this order nor give any measure of local support for inversions in the order of adjacent loci, we viewed it as a rough guide for obtaining resources to be used for the development of new markers and construction of our own detailed genetic and physical map of a small region containing BRCA1. Our approach was to analyze existing STR markers provided by other investigators and any newly developed markers from our laboratory with respect to both a panel of meiotic (genetic) breakpoints identified using DNA from the CEPH reference families and a panel of somatic cell hybrids (physical breakpoints) constructed for this region. These markers included 26C2 developed in our laboratory which maps proximal to Mfd15, Mfd191 (provided by James Weber), THRA1 (Futreal *et al.,* 1992a), and three polymorphisms kindly provided to us by Dr. Donald Black, NM23 (Hall *et al.* 1992), SCG40 (D17S181), and 6C1 (D17S293).

Genetic localization of markers. In order to localize new markers genetically within the region of interest, we have identified a number of key meiotic breakpoints within the region, both in the CEPH reference panel and in our large breast cancer kindred (K2082). Given the small genetic distance in this region, they are likely to be only a relatively small set of recombinants which can be used for this purpose, and they are likely to group markers into sets. The orders of the markers within each set can only be determined by physical mapping. However the number of genotypings necessary to position a new marker is minimized. These breakpoints are illustrated in Tables 3 and 4. Using this approach we were able to genetically order the markers THRA1, 6C1, SCG40, and Mfd191. As can be seen from Tables 3 and 4, THRA1 and MFD191 both map inside the Mfd15-Mfd188 region we had previously identified as containing the BRCA1 locus. In Tables 3 and 4, M/P indicates a maternal or paternal recombinant. A "1" indicates inherited allele is of grandpaternal origin, while a "0" indicates grandmaternal origin, and "-" indicates that the locus was untyped or uninformative.

**TABLE 3**

| CEPH Recombinants | | | | | | | | | |
|---|---|---|---|---|---|---|---|---|---|
| Family | ID | M/P | Mfd15 | THRA1 | Mfd191 | Mfd188 | SCG40 | 6C1 | 42D6 |
| 13292 | 4 | M | 1 | 1 | 1 | 0 | 0 | 0 | 0 |
| 13294 | 4 | M | 1 | 1 | 1 | 0 | 0 | 0 | 0 |
| 13294 | 6 | M | 0 | 0 | 1 | 1 | - | - | - |
| 1334 | 3 | M | 1 | 1 | 1 | 1 | 1 | 0 | 0 |
| 1333 | 4 | M | 1 | 1 | 1 | 0 | - | - | 0 |
| 1333 | 6 | M | 0 | 0 | 1 | 1 | - | - | 1 |
| 1333 | 8 | P | 1 | 0 | 0 | 0 | - | - | 0 |
| 1377 | 8 | M | 0 | - | 0 | 0 | 0 | 0 | 1 |

**TABLE 4**

| Kindred 2082 Recombinants | | | | | | | | |
|---|---|---|---|---|---|---|---|---|
| Family | ID | M/P | Mfd15 | Mfd191 | Mfd188 | SCG40 | 6C1 | 42D6 |
| 75 | | M | 0 | 0 | 1 | 1 | - | - |
| 63 | | M | 0 | 0 | 1 | 1 | - | 1 |
| 125 | | M | 1 | 1 | 1 | 0 | - | 0 |
| 40 | | M | 1 | 1 | 0 | 0 | - | 0 |

Analysis of markers Mfd15, Mfd188, Mfd191, and THRA1 in our recombinant families. Mfd15, Mfd188, Mfd191 and THRA1 were typed in our recombinant families and examined for additional information to localize the BRCA1 locus. In kindred 1901, the Mfd15 recombinant was recombinant for THRA1 but uninformative for Mfdl91, thus placing BRCA1 distal to THRA1. In K2082, the recombinant with Mfd15 also was recombinant with Mfd191, thus placing the BRCA1 locus distal to Mfd191 (Goldgar *et al.,* 1994). Examination of THRA1 and Mfd191 in kindred K2035 yielded no further localization information as the two branches were concordant for both markers. However, SCG40 and 6C1 both displayed the same pattern as Mfd188, thus increasing our confidence in the localization information provided by the Mfd188 recombinant in this family. The BRCA1 locus, or at least a portion of it, therefore lies within an interval bounded by Mfd191 on the proximal side and Mfd188 on the distal side.

### EXAMPLE 4

### Development of Genetic and Physical Resources in the Region of Interest

To increase the number of highly polymorphic loci in the Mfd191-Mfd188 region, we developed a number of STR markers in our laboratory from cosmids and YACs which physically map to the region. These markers allowed us to further refine the region.

STSs were identified from genes known to be in the desired region to identify YACs which contained these loci, which were then used to identify subclones in cosmids, P1s or BACs. These subclones were then screened for the presence of a CA tandem repeat using a (CA)ₙ oligonucleotide (Pharmacia). Clones with a strong signal were selected preferentially, since they were more likely to represent CA-repeats which have a large number of repeats and/or are of near-perfect fidelity to the (CA)ₙ pattern. Both of these characteristics are known to increase the probability of polymorphism (Weber, 1990). These clones were sequenced directly from the vector to locate the repeat. We obtained a unique sequence on one side of the CA-repeat by using one of a set of possible primers complementary to the end of a CA-repeat, such as (GT)₁₀T. Based on this unique sequence, a primer was made to sequence back across the repeat in the other direction, yielding a unique sequence for design of a second primer flanking the CA-repeat. STRs were then screened for polymorphism on a small group of unrelated individuals and tested against the hybrid panel to confirm their physical localization. New markers which satisfied these criteria were then typed in a set of 40 unrelated individuals from the Utah and CEPH families to obtain allele frequencies appropriate for the study population. Many of the other markers reported in this study were tested in a smaller group of CEPH unrelated individuals to obtain similarly appropriate allele frequencies.

Using the procedure described above, a total of eight polymorphic STRs was found from these YACS. Of the loci identified in this manner, four were both polymorphic and localized to the BRCA1 region. Four markers did not localize to chromosome 17, reflecting the chimeric nature of the YACs used. The four markers which were in the region were denoted AA1, ED2, 4-7, and YM29. AA1 and ED2 were developed from YACs positive for the RNU2 gene, 4-7 from an EPB3 YAC and YM29 from a cosmid which localized to the region by the hybrid panel. A description of the number of alleles, heterozygosity and source of these four and all other STR polymorphisms analyzed in the breast cancer kindreds is given below in Table 5.

**TABLE 5**

| Polymorphic Short Tandem Repeat Markers Used for Fine Structure Mapping of the BRCA1 Locus | | | | | | | | | |
|---|---|---|---|---|---|---|---|---|---|
| | | | | Allele^{*} Frequency (%) | | | | | |
| Clone | Gene | Na** | Heterozygosity | 1 | 2 | 3 | 4 | 5 | 6 |
| Mfd15 | D17S250 | 10 | 0.82 | 26 | 22 | 15 | 7 | 7 | 23 |
| THRA1 | THRA1 | 5 | | | | | | | |
| Mfd191 | D17S776 | 7 | 0.55 | 48 | 20 | 11 | 7 | 7 | 7 |
| ED2 | D17S1327 | 12 | 0.55 | 62 | 9 | 8 | 5 | 5 | 11 |
| AA1 | D17S1326 | 7 | 0.83 | 28 | 28 | 25 | 8 | 6 | 5 |
| CA375 | D17S184 | 10 | 0.75 | 26 | 15 | 11 | 9 | 9 | 20 |
| 4-7 | D17S1183 | 9 | 0.50 | 63 | 15 | 8 | 6 | 4 | 4 |
| YM29 | -- | 9 | 0.62 | 42 | 24 | 12 | 7 | 7 | 8 |
| Mfd188 | D17S579 | 12 | 0.92 | 33 | 18 | 8 | 8 | 8 | 25 |
| SCG40 | D17S181 | 14 | 0.90 | 20 | 18 | 18 | 10 | 8 | 35 |
| 42D6 | D17S588 | 11 | 0.86 | 21 | 17 | 11 | 10 | 9 | 32 |
| 6C1 | D17S293 | 7 | 0.75 | 30 | 30 | 11 | 11 | 9 | 9 |
| Z109 | D17S750 | 9 | 0.70 | 33 | 27 | 7 | 7 | 7 | 19 |
| tdj1475 | D17S1321 | 13 | 0.84 | 21 | 16 | 11 | 11 | 8 | 33 |
| CF4 | D17S1320 | 6 | 0.63 | 50 | 27 | 9 | 7 | 4 | 3 |
| tdj1239 | D17S1328 | 10 | 0.80 | 86 | 10 | 9 | 7 | 4 | 14 |
| U5 | D17S1325 | 13 | 0.83 | 19 | 16 | 12 | 10 | 9 | 34 |

| | | | | | | | | | |
|---|---|---|---|---|---|---|---|---|---|
| * Allele codes 1-5 are listed in decreasing frequency; allele numbers do not correspond to fragment sizes. Allele 6 frequency is the joint frequency of all other alleles for each locus. ** Number of alleles seen in the genetically independent DNA samples used for calculating allele frequencies. | | | | | | | | | |

The four STR polymorphisms which mapped physically to the region (4-7, ED2, AA1, YM29) were analyzed in the meiotic, breakpoint panel shown initially in Tables 3 and 4. Tables 6 and 7 contain the relevant CEPH data and Kindred 2082 data for localization of these four markers. In the tables, M/P indicates a maternal or paternal recombinant. A "1" indicates inherited allele is of grandpatemal origin, while a "0" indicates grandmatemal origin, and "-" indicates that the locus was untyped or uninformative.

**TABLE 6**

| Key Recombinants Used for Genetic Ordering of New STR Loci Developed in Our Laboratory Within the BRCA1 Region of 17g | | | | | | | | | | | | | |
|---|---|---|---|---|---|---|---|---|---|---|---|---|---|
| CEPH Family | ID | M/P | Mfd15 | THRA1 | Mfd191 | ED2 | AA1 | Z109 | 4-7 | YM292 | Mfd188 | SCG40 | 42D6 |
| 13292 | 4 | M | 1 | 1 | 1 | 1 | 1 | 0 | 0 | 0 | 0 | 0 | 0 |
| 13294 | 4 | M | 1 | 0 | 0 | - | 0 | - | - | - | 0 | - | - |
| 13294 | 6 | M | 0 | 0 | 1 | - | 1 | - | - | - | 1 | - | - |
| 1333 | 4 | M | 1 | 1 | 1 | - | 0 | - | - | 0 | 0 | - | 0 |
| 1333 | 6 | M | 0 | 0 | 1 | - | 1 | - | - | 1 | 1 | - | 1 |
| 1333 | 3 | M | 0 | 0 | 1 | - | - | - | 1 | 1 | 1 | - | 1 |

**TABLE 7**

| Kindred 2082 Recombinants | | | | | | | | | | |
|---|---|---|---|---|---|---|---|---|---|---|
| ID | M/P | Mfd15 | Mfd191 | ED2 | AA1 | 4-7 | YM29 | Mfd188 | SCG40 | 42D6 |
| 63 | M | 0 | 0 | 1 | - | 1 | 1 | 1 | 1 | 1 |
| 125 | M | 1 | 1 | 1 | - | 1 | 1 | 1 | 0 | 0 |
| 40 | M | 1 | 1 | 0 | - | 0 | - | 0 | 0 | 0 |
| 22 | P | 0 | 0 | 1 | 1 | 1 | 1 | 1 | 1 | 1 |

From CEPH 1333-04, we see that AA1 and YM29 must lie distal to Mfd191. From 13292, it can be inferred that both AA1 and ED2 are proximal to 4-7, YM29, and Mfd188. The recombinants found in K2082 provide some additional ordering information. Three independent observations (individual numbers 22, 40, & 63) place AA1, ED2, 4-7, and YM29, and Mfd188 distal to Mfd191, while ID 125 places 4-7, YM29, and Mfd188 proximal to SCG40. No genetic information on the relative ordering within the two clusters of markers AA1/ED2 and 4-7/YM29/Mfd188 was obtained from the genetic recombinant analysis. Although ordering loci with respect to hybrids which are known to contain "holes" in which small pieces of interstitial human DNA may be missing is problematic, the hybrid patterns indicate that 4-7 lies above both YM29 and Mfdl 88.

### EXAMPLE 5

### Genetic Analyses of Breast Cancer Kindreds with Markers AA1, 4-7, ED2, and YM29

In addition to the three kindreds containing key recombinants which have been discussed previously, kindred K2039 was shown through analysis of the newly developed STR markers to be linked to the region and to contain a useful recombinant.

Table 8 defines the haplotypes (shown in coded form) of the kindreds in terms of specific marker alleles at each locus and their respective frequencies. In Table 8, alleles are listed in descending order of frequency; frequencies of alleles 1-5 for each locus are given in Table 5. Haplotypes coded H are BRCA1 associated haplotypes, P designates a partial H haplotype, and an R indicates an observable recombinant haplotype. As evident in Table 8, not all kindreds were typed for all markers; moreover, not all individuals within a kindred were typed for an identical set of markers, especially in K2082. With one exception, only haplotypes inherited from affected or at-risk kindred members are shown; haplotypes from spouses marrying into the kindred are not described. Thus in a given sibship, the appearance of haplotypes X and Y indicates that both haplotypes from the affected/at-risk individual were seen and neither was a breast cancer associated haplotype.

**TABLE 8**

| Breast Cancer Linked Haplotypes Found in the Three Kindreds | | | | | | | | | | | | | | | |
|---|---|---|---|---|---|---|---|---|---|---|---|---|---|---|---|
| Kin. | HAP | Mfd 15 | THRA1 | Mfd 191 | tdj 1475 | ED2 | AA1 | Z109 | CA375 | 4-7 | YM29 | Mfd 188 | SCG40 | 6C1 | 42D6 |
| 1901 | H1 | 1 | 5 | 5 | 3 | 1 | 4 | NI | NI | 1 | 1 | 3 | NI | NI | 1 |
| | R2 | 9 | 2 | 5 | 6 | 1 | 4 | NI | NI | 1 | 1 | 3 | NI | NI | 1 |
| | | | | | | | | | | | | | | | |
| 2082 | H1 | 3 | NI | 4 | 6 | 6 | 1 | NI | NI | 2 | 1 | 4 | 2 | NI | 1 |
| | P1 | 3 | NI | 4 | NI | NI | NI | NI | NI | NI | NI | 4 | 2 | NI | 1 |
| | P2 | 3 | NI | NI | NI | NI | NI | NI | NI | NI | NI | 4 | NI | NI | NI |
| | R1 | 6 | NI | 1 | 5 | 6 | 1 | NI | NI | 2 | 1 | 4 | 2 | NI | 1 |
| | R2 | 6 | NI | 4 | 6 | 6 | 1 | NI | NI | 2 | 1 | 4 | 2 | NI | 1 |
| | R3 | 3 | NI | 4 | NI | 6 | 1 | NI | NI | 2 | 1 | 4 | 1 | NI | 7 |
| | R4 | 7 | NI | 1 | NI | 1 | 5 | NI | NI | 4 | 6 | 1 | 2 | NI | 1 |
| | R5 | 3 | NI | 4 | NI | NI | NI | NI | NI | NI | 2 | 1 | NI | NI | NI |
| | R6 | 3 | NI | 4 | 3 | 1 | 2 | NI | NI | 1 | 2 | 2 | 6 | NI | 6 |
| | R7 | 3 | NI | 4 | 3 | 7 | 1 | NI | NI | 1 | 1 | 3 | 7 | NI | 4 |
| | | | | | | | | | | | | | | | |
| 2035 | HI | 8 | 2 | 1 | NI | 5 | 1 | 1 | 4 | 3 | 1 | 6 | 8 | 2 | 4 |
| | H2 | 8 | 2 | 1 | NI | 5 | 1 | 1 | 2 | 1 | 1 | 2 | 3 | 1 | 4 |
| | R2 | 8 | 2 | 1 | NI | 5 | 1 | 1 | 2 | 1 | 1 | 2 | 3 | 6 | 1 |

In kindred K1901, the new markers showed no observable recombination with breast cancer susceptibility, indicating that the recombination event in this kindred most likely took place between THRA1 and ED2. Thus, no new BRCA1 localization information was obtained based upon studying the four new markers in this kindred. In kindred 2082 the key recombinant individual has inherited the linked alleles for ED2, 4-7, AA1, and YM29, and was recombinant for tdj1474 indicating that the recombination event occurred in this individual between tdj1474 and ED2/AA1.

There are three haplotypes of interest in kindred K2035, H1, H2, and R2 shown in Table 8. H1 is present in the four cases and one obligate male carrier descendant from individual 17 while H2 is present or inferred in two cases and two obligate male carriers in descendants of individual 10. R2 is identical to H2 for loci between and including Mfd15 and SCG40, but has recombined between SCG40 and 42D6. Since we have established that BRCA1 is proximal to 42D6, this H2/R2 difference adds no further localization information. H1 and R2 share an identical allele at Mfd15, THRA1, AA1, and ED2 but differ for loci presumed distal to ED2, i.e., 4-7, Mfd188, SCG40, and 6C1. Although the two haplotypes are concordant for the 5th allele for marker YM29, a marker which maps physically between 4-7 and Mfd188, it is likely that the alleles are shared identical by state rather than identical by descent since this allele is the most common allele at this locus with a frequency estimated in CEPH parents of 0.42. By contrast, the linked alleles shared at the Mfd15 and ED2 loci have frequencies of 0.04 and 0.09, respectively. They also share more common alleles at Mfd191 (frequency = 0.52), THRA1, and AA1 (frequency = 0.28). This is the key recombinant in the set as it is the sole recombinant in which breast cancer segregated with the proximal portion of the haplotype, thus setting the distal boundary. The evidence from this kindred therefore places the BRCA1 locus proximal to 4-7.

The recombination event in kindred 2082 which places BRCA1 distal to tdj1474 is the only one of the four events described which can be directly inferred; that is, the affected mother's genotype can be inferred from her spouse and offspring, and the recombinant haplotype can be seen in her affected daughter. In this family the odds in favor of affected individuals carrying BRCA1 susceptibility alleles are extremely high; the only possible interpretations of the data are that BRCA1 is distal to Mfd191 or alternatively that the purported recombinant is a sporadic case of ovarian cancer at age 44. Rather than a directly observable or inferred recombinant, interpretation of kindred 2035 depends on the observation of distinct 17q-haplotypes segregating in different and sometimes distantly related branches of the kindred. The observation that portions of these haplotypes have alleles in common for some markers while they differ at other markers places the BRCA1 locus in the shared region. The confidence in this placement depends on several factors: the relationship between the individuals carrying the respective haplotypes, the frequency of the shared allele, the certainty with which the haplotypes can be shown to segregate with the BRCA1 locus, and the density of the markers in the region which define the haplotype. In the case of kindred 2035, the two branches are closely related, and each branch has a number of early onset cases which carry the respective haplotype. While two of the shared alleles are common, (Mfd191, THRA1), the estimated frequencies of the shared alleles at Mfd15, AA1, and ED2 are 0.04, 0.28, and 0.09, respectively. It is therefore highly likely that these alleles are identical by descent (derived from a common ancestor) rather than identical by state (the same allele but derived from the general population).

### EXAMPLE 6

### Refined Physical Mapping Studies Place the BRCA1 Gene in a Region Flanked by tdj1474 and USR

Since its initial localization to chromosome 17q in 1990 (Hall *et al.,* 1990) a great deal of effort has gone into localizing the BRCA1 gene to a region small enough to allow implementation of effective positional cloning strategies to isolate the gene. The BRCA1 locus was first localized to the interval Mfd15 (D17S250) - 42D6 (D17S588) by multipoint linkage analysis (Easton *et al.,* 1993) in the collaborative Breast Cancer Linkage Consortium dataset consisting of 214 families collected worldwide. Subsequent refinements of the localization have been based upon individual recombinant events in specific families. The region THRA1 - D17S183 was defined by Bowcock *et al.,* 1993; and the region THRA1 - D17S78 was defined by Simard *et al*., 1993.

We further showed that the BRCA1 locus must lie distal to the marker Mfd191 (D17S776) (Goldgar *et al*., 1994). This marker is known to lie distal to THRA1 and RARA. The smallest published region for the BRCA1 locus is thus between D17S776 and D17S78. This region still contains approximately 1.5 million bases of DNA, making the isolation and testing of all genes in the region a very difficult task. We have therefore undertaken the tasks of constructing a physical map of the region, isolating a set of polymorphic STR markers located in the region, and analyzing these new markers in a set of informative families to refine the location of the BRCA1 gene to a manageable interval.

Four families provide important genetic evidence for localization of BRCA1 to a sufficiently small region for the application of positional cloning strategies. Two families (K2082, K1901) provide data relating to the proximal boundary for BRCA1 and the other two (K2035, K1813) fix the distal boundary. These families are discussed in detail below. A total of 15 Short Tandem Repeat markers assayable by PCR were used to refine this localization in the families studied. These markers include DS17S7654, DS17S975, tdj1474, and tdj1239. Primer sequences for these markers are provided in SEQ ID NO:3 and SEQ ID NO:4 for DS17S754; in SEQ ID NO:5 and SEQ ID NO:6 for DS17S975; in SEQ ID NO:7 and SEQ ID N0:8 for tdj1474; and, in SEQ ID NO:9 and SEQ ID NO:10 for tdj1239.

### Kindred 2082

Kindred 2082 is the largest BRCA1-linked breast/ovarian cancer family studied to date. It has a LOD score of 8.6, providing unequivocal evidence for 17q linkage. This family has been previously described and shown to contain a critical recombinant placing BRCA1 distal to MFD191 (D17S776). This recombinant occurred in a woman diagnosed with ovarian cancer at age 45 whose mother had ovarian cancer at age 63. The affected mother was deceased; however, from her children, she could be inferred to have the linked haplotype present in the 30 other linked cases in the family in the region between Mfd15 and Mfd188. Her affected daughter received the linked allele at the loci ED2, 4-7, and Mfd188, but received the allele on the non-BRCA1 chromosome at Mfd15 and Mfd191. In order to further localize this recombination breakpoint, we tested the key members of this family for the following markers derived from physical mapping resources: tdj1474, tdj1239, CF4, D17S855. For the markers tdj 1474 and CF4, the affected daughter did not receive the linked allele. For the STR locus tdj1239, however, the mother could be inferred to be informative and her daughter did receive the BRCA1-associated allele. D17S855 was not informative in this family. Based on this analysis, the order is 17q centromere - Mfd191 - 17HSD - CF4 - tdj1474 - tdj1239 - D17S855 - ED2 - 4-7 - Mfd188 17q telomere. The recombinant described above therefore places BRCA1 distal to tdj1474, and the breakpoint is localized to the interval between tdj1474 and tdj1239. The only alternative explanation for the data in this family other than that of BRCA1 being located distal to tdj1474, is that the ovarian cancer present in the recombinant individual is caused by reasons independent of the BRCA1 gene. Given that ovarian cancer diagnosed before age 50 is rare, this alternate explanation is exceedingly unlikely.

### Kindred 1901

Kindred 1901 is an early-onset breast cancer family with 7 cases of breast cancer diagnosed before 50, 4 of which were diagnosed before age 40. In addition, there were three cases of breast cancer diagnosed between the ages of 50 and 70. One case of breast cancer also had ovarian cancer at age 61. This family currently has a LOD score of 1.5 with D17S855. Given this linkage evidence and the presence of at lease one ovarian cancer case, this family has a posterior probability of being due to BRCA1 of over 0.99. In this family, the recombination comes from the fact that an individual who is the brother of the ovarian cancer case from which the majority of the other cases descend, only shares a portion of the haplotype which is cosegregating with the other cases in the family. However, he passed this partial haplotype to his daughter who developed breast cancer at age 44. If this case is due to the BRCA1 gene, then only the part of the haplotype shared between this brother and his sister can contain the BRCA1 gene. The difficulty in interpretation of this kind of information is that while one can be sure of the markers which are not shared and therefore recombinant, markers which are concordant can either be shared because they are non-recombinant, or because their parent was homozygous. Without the parental genotypic data it is impossible to discriminate between these alternatives. Inspection of the haplotype in K1901, shows that he does not share the linked allele at Mfd15 (D17S250), THRA1, CF4 (D17S1320), and tdj1474 (17DS1321). He does share the linked allele at Mfd191 (D17S776), ED2 (D17S1327), tdj1239 (D17S1328), and Mfdl88 (D17S579). Although the allele shared at Mfd191 is relatively rare (0.07), we would presume that the parent was homozygous since they are recombinant with markers located nearby on either side, and a double recombination event in this region would be extremely unlikely. Thus the evidence in this family would also place the BRCA1 locus distal to tdj1474. However, the lower limit of this breakpoint is impossible to determine without parental genotype information. It is intriguing that the key recombinant breakpoint in this family confirms the result in Kindred 2082. As before, the localization information in this family is only meaningful if the breast cancer was due to the BRCA1 gene. However, her relatively early age at diagnosis (44) makes this seem very likely since the risk of breast cancer before age 45 in the general population is low (approximately 1%).

### Kindred 2035

This family is similar to K1901 in that the information on the critical recombinant events is not directly observed but is inferred from the observation that the two haplotypes which are cosegregating with the early onset breast cancer in the two branches of the family appear identical for markers located in the proximal portion of the 17q BRCA1 region but differ at more distal loci. Each of these two haplotypes occurs in at least four cases of early-onset or bilateral breast cancer. The overall LOD score with ED2 in this family is 2.2, and considering that there is a case of ovarian cancer in the family (indicating a prior probability of BRCA1 linkage of 80%), the resulting posterior probability that this family is linked to BRCA1 is 0.998. The haplotypes are identical for the markers Mfd15, THRA1, Mfd191, ED2, AA1, D17S858 and D17S902. The common allele at Mfd15 and ED2 are both quite rare, indicating that this haplotype is shared identical by descent. The haplotypes are discordant, however, for CA375, 4-7, and Mfd188, and several more distal markers. This indicates that the BRCA1 locus must lie above the marker CA-375. This marker is located approximately 50 kb below D17S78, so it serves primarily as additional confirmation of this previous lower boundary as reported in Simard *et al.* (1993).

### Kindred 1813

Kindred 1813 is a small family with four cases of breast cancer diagnosed under the age of 40 whose mother had breast cancer diagnosed at age 45 and ovarian cancer at age 61. This situation is somewhat complicated by the fact the four cases appear to have three different fathers, only one of whom has been genotyped. However, by typing a number of different markers in the BRCA1 region as well as highly polymorphic markers elsewhere in the genome, the paternity of all children in the family has been determined with a high degree of certainty. This family yields a maximum multipoint LOD score of 0.60 with 17q markers and, given that there is at least one case of ovarian cancer, results in a posterior probability of being a BRCA1 linked family of 0.93. This family contains a directly observable recombination event in individual 18 (see Figure 5 in Simard et al., Human Mol. Genet. 2:1193-1199 (1993)), who developed breast cancer at age 34. The genotype of her affected mother at the relevant 17q loci can be inferred from her genotypes, her affected sister's genotypes, and the genotypes of three other unaffected siblings. Individual 18 inherits the BRCA1-linked alleles for the following loci: Mfd15, THRA1, D17S800, D17S855, AA1, and D17S931. However, for markers below D17S931, i.e., U5R, vrs31, D17S858, and D17S579, she has inherited the alleles located on the non-disease bearing chromosome. The evidence from this family therefore would place the BRCA1 locus proximal to the marker U5R. Because of her early age at diagnosis (34) it is extremely unlikely that the recombinant individual's cancer is not due to the gene responsible for the other cases of breast/ovarian cancer in this family; the uncertainty in this family comes from our somewhat smaller amount of evidence that breast cancer in this family is due to BRCA1 rather than a second, as yet unmapped, breast cancer susceptibility locus.

### Size of the region containing BRCA1

Based on the genetic data described in detail above, the BRCA1 locus must lie in the interval between the markers tdj1474 and U5R, both of which were isolated in our laboratory. Based upon the physical maps shown in Figures 2 and 3, we can try to estimate the physical distance between these two loci. It takes approximately 14 P1 clones with an average insert size of approximately 80 kb to span the region. However, because all of these P1s overlap to some unknown degree, the physical region is most likely much smaller than 14 times 80 kb. Based on restriction maps of the clones covering the region, we estimate the size of the region containing BRCA1 to be approximately 650 kb.

### EXAMPLE 7

### Identification of Candidate cDNA Clones for the BRCA1 Locus by Genomic Analysis of the Contig Region

Complete screen of the plausible region. The first method to identify candidate cDNAs, although labor intensive, used known techniques. The method comprised the screening of cosmids and P1 and BAC clones in the contig to identify putative coding sequences. The clones containing putative coding sequences were then used as probes on filters of cDNA libraries to identify candidate cDNA clones for future analysis. The clones were screened for putative coding sequences by either of two methods.

Zoo blots. The first method for identifying putative coding sequences was by screening the cosmid and P1 clones for sequences conserved through evolution across several species. This technique is referred to as "zoo blot analysis" and is described by Monaco, 1986. Specifically, DNAs from cow, chicken, pig, mouse and rat were digested with the restriction enzymes EcoRI and HindIII (8 µg of DNA per enzyme). The digested DNAs were separated overnight on an 0.7% gel at 20 volts for 16 hours (14 cm gel), and the DNA transferred to Nylon membranes using standard Southern blot techniques. For example, the zoo blot filter was treated at 65°C in 0.1 x SSC, 0.5% SDS, and 0.2M Tris, pH 8.0, for 30 minutes and then blocked overnight at 42°C in 5x SSC, 10% PEG 8000, 20 mM NaPO₄ pH 6.8, 100 µg/ml Salmon Sperm DNA, lx Denhardt's, 50% formamide, 0.1% SDS, and 2 µg/ml Cₒt-1 DNA.

The cosmid and P1 clones to be analyzed were digested with a restriction enzyme to release the human DNA from the vector DNA. The DNA was separated on a 14 cm, 0.5% agarose gel run overnight at 20 volts for 16 hours. The human DNA bands were cut out of the gel and electroeluted from the gel wedge at 100 volts for at least two hours in 0.5x Tris Acetate buffer (Maniatis *et al*., 1982). The eluted Not I digested DNA (∼15 kb to 25 kb) was then digested with EcoRI restriction enzyme to give smaller fragments (∼0.5 kb to 5.0 kb) which melt apart more easily for the next step of labeling the DNA with radionucleotides. The DNA fragments were labeled by means of the hexamer random prime labeling method (Boehringer-Mannheim, Cat. #1004760). The labeled DNA was spermine precipitated (add 100 µl TE, 5 µl 0.1 M spermine, and 5 µl of 10 mg/ml salmon sperm DNA) to remove unincorporated radionucleotides. The labeled DNA was then resuspended in 100 µl TE, 0.5 M NaCl at 65°C for 5 minutes and then blocked with Human Cₒt-1 DNA for 2-4 hrs. as per the manufacturer's instructions (Gibco/BRL, Cat. #5279SA). The Cₒt-1 blocked probe was incubated on the zoo blot filters in the blocking solution overnight at 42°C. The filters were washed for 30 minutes at room temperature in 2 x SSC, 0.1% SDS, and then in the same buffer for 30 minutes at 55°C. The filters were then exposed 1 to 3 days at -70°C to Kodak XAR-5 film with an intensifying screen. Thus, the zoo blots were hybridized with either the pool of Eco-R1 fragments from the insert, or each of the fragments individually.

HTF island analysis. The second method for identifying cosmids to use as probes on the cDNA libraries was HTF island analysis. Since the pulsed-field map can reveal HTF islands, cosmids that map to these HTF island regions were analyzed with priority. HTF islands are segments of DNA which contain a very high frequency of unmethylated CpG dinucleotides (Tonolio *et al*., 1990) and are revealed by the clustering of restriction sites of enzymes whose recognition sequences include CpG dinucleotides. Enzymes known to be useful in HTF-island analysis are AscI, NotI, BssHII, EagI, SacII, NaeI, NarI, SmaI, and MluI (Anand, 1992). A pulsed-field map was created using the enzymes NotI, NruI, EagI, SacII, and SalI, and two HTF islands were found. These islands are located in the distal end of the region, one being distal to the GP2B locus, and the other being proximal to the same locus, both outside the BRCA1 region. The cosmids derived from the YACs that cover these two locations were analyzed to identify those that contain these restriction sites, and thus the HTF islands.

cDNA screening. Those clones that contain HTF islands or show hybridization to other species DNA besides human are likely to contain coding sequences. The human DNA from these clones was isolated as whole insert or as EcoRl fragments and labeled as described above. The labeled DNA was used to screen filters of various cDNA libraries under the same conditions as the zoo blots except that the cDNA filters undergo a more stringent wash of 0.1 x SSC, 0.1% SDS at 65°C for 30 minutes twice.

Most of the cDNA libraries used to date in our studies (libraries from normal breast tissue, breast tissue from a woman in her eighth month of pregnancy and a breast malignancy) were prepared at Clonetech, Inc. The cDNA library generated from breast tissue of an 8 month pregnant woman is available from Clonetech (Cat. #HL1037a) in the Lambda gt-10 vector, and is grown in C600Hfl bacterial host cells. Normal breast tissue and malignant breast tissue samples were isolated from a 37 year old Caucasian female and one-gram of each tissue was sent to Clonetech for mRNA processing and cDNA library construction. The latter two libraries were generated using both random and oligo-dT priming, with size selection of the final products which were then cloned into the Lambda Zap II vector, and grown in XL1-blue strain of bacteria as described by the manufacturer. Additional tissue-specific cDNA libraries include human fetal brain (Stratagene, Cat. 936206), human testis (Clonetech Cat. HL3024), human thymus (Clonetech Cat. HL1127n), human brain (Clonetech Cat. HL11810), human placenta (Clonetech Cat 1075b), and human skeletal muscle (Clonetech Cat. HL1124b).

The cDNA libraries were plated with their host cells on NZCYM plates, and filter lifts are made in duplicate from each plate as per Maniatis *et al.* (1982). Insert (human) DNA from the candidate genomic clones was purified and radioactively labeled to high specific activity. The radioactive DNA was then hybridized to the cDNA filters to identify those cDNAs which correspond to genes located within the candidate cosmid clone. cDNAs identified by this method were picked, replated, and screened again with the labeled clone insert or its derived EcoR1 fragment DNA to verify their positive status. Clones that were positive after this second round of screening were then grown up and their DNA purified for Southern blot analysis and sequencing. Clones were either purified as plasmid through *in vivo* excision of the plasmid from the Lambda vector as described in the protocols from the manufacturers, or isolated from the Lambda vector as a restriction fragment and subcloned into plasmid vector.

The Southern blot analysis was performed in duplicate, one using the original genomic insert DNA as a probe to verify that cDNA insert contains hybridizing sequences. The second blot was hybridized with cDNA insert DNA from the largest cDNA clone to identify which clones represent the same gene. All cDNAs which hybridize with the genomic clone and are unique were sequenced and the DNA analyzed to determine if the sequences represent known or unique genes. All cDNA clones which appear to be unique were further analyzed as candidate BRCA1 loci. Specifically, the clones are hybridized to Northern blots to look for breast specific expression and differential expression in normal versus breast tumor RNAs. They are also analyzed by PCR on clones in the BRCA1 region to verify their location. To map the extent of the locus, full length cDNAs are isolated and their sequences used as PCR probes on the YACs and the clones surrounding and including the original identifying clones. Intron-exon boundaries are then further defined through sequence analysis.

We have screened the normal breast, 8 month pregnant breast and fetal brain cDNA libraries with zoo blot-positive Eco R1 fragments from cosmid BAC and P1 clones in the region. Potential BRCA1 cDNA clones were identified among the three libraries. Clones were picked, replated, and screened again with the original probe to verify that they were positive.

Analysis of hybrid-selected cDNA. cDNA fragments obtained from direct selection were checked by Southern blot hybridization against the probe DNA to verify that they originated from the contig. Those that passed this test were sequenced in their entirety. The set of DNA sequences obtained in this way were then checked against each other to find independent clones that overlapped. For example, the clones 694-65, 1240-1 and 1240-33 were obtained independently and subsequently shown to derive from the same contiguous cDNA sequence which has been named EST:489:1.

Analysis of candidate clones. One or more of the candidate genes generated from above were sequenced and the information used for identification and classification of each expressed gene. The DNA sequences were compared to known genes by nucleotide sequence comparisons and by translation in all frames followed by a comparison with known amino acid sequences. This was accomplished using Genetic Data Environment (GDE) version 2.2 software and the Basic Local Alignment Search Tool (Blast) series of client/server software packages (e.g., BLASTN 1.3.13MP), for sequence comparison against both local and remote sequence databases (e.g., GenBank), running on Sun SPARC workstations. Sequences reconstructed from collections of cDNA clones identified with the cosmids and P1s have been generated. All candidate genes that represented new sequences were analyzed further to test their candidacy for the putative BRCA1 locus.

Mutation screening. To screen for mutations in the affected pedigrees, two different approaches were followed. First, genomic DNA isolated from family members known to carry the susceptibility allele of BRCA1 was used as a template for amplification of candidate gene sequences by PCR. If the PCR primers flank or overlap an intron/exon boundary, the amplified fragment will be larger than predicted from the cDNA sequence or will not be present in the amplified mixture. By a combination of such amplification experiments and sequencing of P1, BAC or cosmid clones using the set of designed primers it is possible to establish the intron/exon structure and ultimately obtain the DNA sequences of genomic DNA from the pedigrees.

A second approach that is much more rapid if the intron/exon structure of the candidate gene is complex involves sequencing fragments amplified from pedigree lymphocyte cDNA. cDNA synthesized from lymphocyte mRNA extracted from pedigree blood was used as a substrate for PCR amplification using the set of designed primers. If the candidate gene is expressed to a significant extent in lymphocytes, such experiments usually produce amplified fragments that can be sequenced directly without knowledge of intron/exon junctions.

The products of such sequencing reactions were analyzed by gel electrophoresis to determine positions in the sequence that contain either mutations such as deletions or insertions, or base pair substitutions that cause amino acid changes or other detrimental effects.

Any sequence within the BRCA1 region that is expressed in breast is considered to be a candidate gene for BRCA1. Compelling evidence that a given candidate gene corresponds to BRCA1 comes from a demonstration that pedigree families contain defective alleles of the candidate.

### EXAMPLE 8

### Identification of BRCA1

Identification of BRCA1. Using several strategies, a detailed map of transcripts was developed for the 600 kb region of 17q21 between D17S1321 and D17S1324. Candidate expressed sequences were defined as DNA sequences obtained from: 1) direct screening of breast, fetal brain, or lymphocyte cDNA libraries, 2) hybrid selection of breast, lymphocyte or ovary cDNAs, or 3) random sequencing of genomic DNA and prediction of coding exons by XPOUND (Thomas and Skolnick, 1994). These expressed sequences in many cases were assembled into contigs composed of several independently identified sequences. Candidate genes may comprise more than one of these candidate expressed sequences. Sixty-five candidate expressed sequences within this region were identified by hybrid selection, by direct screening of cDNA libraries, and by random sequencing of P1 subclones. Expressed sequences were characterized by transcript size, DNA sequence, database comparison, expression pattern, genomic structure, and, most importantly, DNA sequence analysis in individuals from kindreds segregating 17q-linked breast and ovarian cancer susceptibility.

Three independent contigs of expressed sequence, 1141:1 (649 bp), 694:5 (213 bp) and 754:2 (1079 bp) were isolated and eventually shown to represent portions of BRCA1. When ESTs for these contigs were used as hybridization probes for Northern analysis, a single transcript of approximately 7.8 kb was observed in normal breast mRNA, suggesting that they encode different portions of a single gene. Screens of breast, fetal brain, thymus, testes, lymphocyte and placental cDNA libraries and PCR experiments with breast mRNA linked the 1141:1, 694:5 and 754:2 contigs. 5' RACE experiments with thymus, testes, and breast mRNA extended the contig to the putative 5' end, yielding a composite full length sequence. PCR and direct sequencing of P1s and BACs in the region were used to identify the location of introns and allowed the determination of splice donor and acceptor sites. These three expressed sequences were merged into a single transcription unit that proved in the final analysis to be BRCA1. This transcription unit is located adjacent to D17S855 in the center of the 600 kb region (Fig. 4).

Combination of sequences obtained from cDNA clones, hybrid selection sequences, and amplified PCR products allowed construction of a composite full length BRCA1 cDNA (SEQ ID NO:1). The sequence of the BRCA1 cDNA (up through the stop codon) has also been deposited with GenBank and assigned accession number U-14680. This deposited sequence is incorporated herein by reference. The cDNA clone extending farthest in the 3' direction contains a poly(A) tract preceded by a polyadenylation signal. Conceptual translation of the cDNA revealed a single long open reading frame of 208 kilodaltons (amino acid sequence: SEQ ID NO:2) with a potential initiation codon flanked by sequences resembling the Kozak consensus sequence (Kozak, 1987). Smith-Waterman (Smith and Waterman, 1981) and BLAST (Altschul *et al*., 1990) searches identified a sequence near the amino terminus with considerable homology to zinc-finger domains (Fig. 5). This sequence contains cysteine and histidine residues present in the consensus C3HC4 zinc-finger motif and shares multiple other residues with zinc-finger proteins in the databases. The BRCA1 gene is composed of 23 coding exons arrayed over more than 100 kb of genomic DNA (Fig. 6). Northern blots using fragments of the BRCA1 cDNA as probes identified a single transcript of about 7.8 kb, present most abundantly in breast, thymus and testis, and also present in ovary (Fig. 7). Four alternatively spliced products were observed as independent cDNA clones; 3 of these were detected in breast and 2 in ovary mRNA (Fig. 6). A PCR survey from tissue cDNAs further supports the idea that there is considerable heterogeneity near the 5' end of transcripts from this gene; the molecular basis for the heterogeneity involves differential choice of the first splice donor site, and the changes detected all alter the transcript in the region 5' of the identified start codon. We have detected six potential alternate splice donors in this 5' untranslated region, with the longest deletion being 1,155 bp. The predominant form of the BRCA1 protein in breast and ovary lacks exon 4. The nucleotide sequence for BRCA1 exon 4 is shown in SEQ ID NO:11, with the predicted amino acid sequence shown in SEQ ID NO:12.

Additional 5' sequence of BRCA1 genomic DNA is set forth in SEQ ID NO:13. The G at position 1 represents the potential start site in testis. The A in position 140 represents the potential start site in somatic tissue. There are six alternative splice forms of this 5' sequence as shown in Figure 8. The G at position 356 represents the canonical first splice donor site. The G at position 444 represents the first splice donor site in two clones (testis 1 and testis 2). The G at position 889 represents the first splice donor site in thymus 3. A fourth splice donor site is the G at position 1230. The T at position 1513 represents the splice acceptor site for all of the above splice donors. A fifth alternate splice form has a first splice donor site at position 349 with a first acceptor site at position 591 and a second splice donor site at position 889 and a second acceptor site at position 1513. A sixth alternate form is unspliced in this 5' region. The A at position 1532 is the canonical start site, which appears at position 120 of SEQ ID NO:1. Partial genomic DNA sequences determined for BRCA1 are set forth in Figures 10A-1OH and SEQ ID Numbers:14-34. The lower case letters (in figures 10A-10H) denote intron sequence while the upper case letters denote exon sequence. Indefinite intervals within introns are designated with vvvvvvvvvvvvv in Figures 10A-10H. The intron/exon junctions are shown in Table 9. The CAG found at the 5' end of exons 8 and 14 is found in some cDNAs but not in others. Known polymorphic sites are shown in Figures 10A-10H in boldface type and are underlined. The known polymorphisms are listed in Tables 18 and 19.

**TABLE 9**

| Base | | | | | |
|---|---|---|---|---|---|
| Exon | Position^{*} | | | Intron Borders | |
| No. | 5' | 3' | Length | 5' | 3' |
| e1 | 1 | 100 | 100 | GATAAATTAAAACTGCGACTGCGCGGCGTG^{35*} | GTAGTAGAGTCCCGGGAAAGGGACAGGGGG³⁶ |
| e2 | 101 | 199 | 99 | ATATATATATGTTTTTCTAATGTGTTAAAG³⁷ | GTAAGTCAGCACAAGAGTGTATTAATTTGG³⁸ |
| e3 | 200 | 253 | 54 | TTTCTTTTTCTCCCCCCCCTACCCTGCTAG³⁹ | GTAAGTTTGAATGTGTTATGTGGCTCCATT⁴⁰ |
| e4 | *** | *** | 111 | AGCTACTTTTTTTTTTTTTTTTTGAGACAG⁴¹ | GTAAGTGCACACCACCATATCCAGCTAAAT⁴² |
| e5 | 254 | 331 | 78 | AATTGTTCTTTCTTTCTTTATAATTTATAG⁴³ | GTATATAATTTGGTAATGATGCTAGGTTGG⁴⁴ |
| e6 | 332 | 420 | 89 | GAGTGTGTTTCTCAAACAATTTAATTTCAG⁴⁵ | GTAAGTGTTGAATATCCCAAGAATGACACT⁴⁶ |
| e7 | 421 | 560 | 140 | AAACATAATGTTTTCCCTTGTATTTTACAG⁴⁷ | GTAAAACCATTTGTTTTCTTCTTCTTCTTC⁴⁸ |
| e8 | 561 | 666 | 106 | TGCTTGACTGTTCTTTACCATACTGTTTAG⁴⁹ | GTAAGGGTCTCAGGTTTTTTAAGTATTTAA⁵⁰ |
| e9 | 667 | 712 | 46 | TGATTTATTTTTTGGGGGGAAATTTTTTAG⁵¹ | GTGAGTCAAAGAGAACCTTTGTCTATGAAG⁵² |
| e10 | 713 | 789 | 77 | TCTTATTAGGACTCTGTCTTTTCCCTATAG⁵³ | GTAATGGCAAAGTTTOCCAACTTAACAGGC⁵⁴ |
| e11 | 790 | 4215 | 3426 | GAGTACCTTGTTATTTTTGTATATTTTCAG⁵⁵ | GTATTGGAACCAGGTTTTTGTGTTTGCCCC⁵⁶ |
| e12 | 4216 | 4302 | 87 | ACATCTGAACCTCTGTTTTTGTTATTTAAG⁵⁷ | AGGTAAAAAGCGTGTGTGTGTGTGCACATG⁵⁸ |
| e13 | 4303 | 4476 | 174 | CATTTTCTTGGTACCATTTATCGTTTTTGA⁵⁹ | GTGTGTATTGTTGGCCAAACACTGATATCT⁶⁰ |
| e14 | 4477 | 4603 | 127 | AGTAGATTTGTTTTCTCATTCCATTTAAAG⁶¹ | GTAAGAAACATCAATGTAAAGATGCTGTGG⁶² |
| e15 | 4604 | 4794 | 191 | ATGGTTTTCTCCTTCCATTTATCTTTCTAG^{63**} | GTAATATTTCATCTGCTGTATTGGAACAAA⁶⁴ |
| e16 | 4795 | 5105 | 311 | TGTAAATTAAACTTCTCCCATTCCTTTCAG⁶⁵ | GTGAGTGTATCCATATGTATCTCCCTAATG⁶⁶ |
| e17 | 5106 | 5193 | 88 | ATGATAATGGAATATTTGATTTAATTTCAG⁶⁷ | GTATACCAAGAACCTTTACAGAATACCTTG⁶⁸ |
| e18 | 5194 | 5271 | 78 | CTAATCCTTTGAGTGTTTTTCATTCTGCAG⁶⁹ | GTAAGTATAATACTATTTCTCCCCTCCTCC⁷⁰ |
| e19 | 5272 | 5312 | 41 | TGTAACCTGTCTTTTCTATGATCTCTTTAG⁷¹ | GTAAGTACTTGATGTTACAAACTAACCAGA⁷² |
| e20 | 5313 | 5396 | 84 | TCCTGATGGGTTGTGTTTCGTTTCTTTCAG⁷³ | GTAAAGCTCCCTCCCTCAAGTTGACAAAAA⁷⁴ |
| e21 | 5397 | 5451 | 55 | CTGTCCCTCTCTCTTCCTCTCTTCTTCCAG⁷⁵ | GTAAGAGCCTGGGAGAACCCCAGAGTTCCA⁷⁶ |
| e22 | 5452 | 5525 | 74 | AGTGATTTTACATGTAAATGTCCATTTTAG⁷⁷ | GTAAGTATTGGGTGCCCTGTCAGTGTGGGA⁷⁸ |
| e23 | 5526 | 5586 | 61 | TTGAATGCTCTTTCCTTCCTGGGGATCCAG⁷⁹ | GTAAGGTGCCTCGCATGTACCTGTGCTATT⁸⁰ |
| e24 | 5587 | 5914 | 328 | CTAATCTCTGCTTGTGTTCTCTGTCTCCAG⁸¹ | |

| | | | | | |
|---|---|---|---|---|---|
| * Base numbers in SEQ ID NO:1. ** Numbers in superscript refer to SEQ ID NOS. *** e4 from SEQ ID NO:11. | | | | | |

Low stringency blots in which genomic DNA from organisms of diverse phylogenetic background were probed with BRCA1 sequences that lack the zinc-finger region revealed strongly hybridizing fragments in human, monkey, sheep and pig, and very weak hybridization signals in rodents. This result indicates that, apart from the zinc-finger domain, BRCA1 is conserved only at a moderate level through evolution.

Germline BRCA1 mutations in 17q-linked kindreds. The most rigorous test for BRCA1 candidate genes is to search for potentially disruptive mutations in carrier individuals from kindreds that segregate 17q-linked susceptibility to breast and ovarian cancer. Such individuals must contain BRCA1 alleles that differ from the wildtype sequence. The set of DNA samples used in this analysis consisted of DNA from individuals representing 8 different BRCA1 kindreds (Table 10).

**TABLE 10**

| KINDRED DESCRIPTIONS AND ASSOCIATED LOD SCORES | | | | | | |
|---|---|---|---|---|---|---|
| Kindred | Cases (n) | | | Sporadic Cases¹ (n) | LOD Score | Marker(s) |
| | Br | Br<50 | Ov | | | |
| 2082 | 31 | 20 | 22 | 7 | 9.49 | D17S1327 |
| 2099 | 22 | 14 | 2* | 0 | 2.36 | D17S800/D17S855² |
| 2035 | 10 | 8 | 1* | 0 | 2.25 | D17S1327 |
| 1901 | 10 | 7 | 1* | 0 | 1.50 | D17S855 |
| 1925 | 4 | 3 | 0 | 0 | 0.55 | D17S579 |
| 1910 | 5 | 4 | 0 | 0 | 0.36 | D17S579/D17S250² |
| 1927 | 5 | 4 | 0 | 1 | -0.44 | D17S250 |
| 1911 | 8 | 5 | 0 | 2 | -0.20 | D17S250 |

| | | | | | | |
|---|---|---|---|---|---|---|
| ¹ Number of women with breast cancer (diagnosed under age 50) or ovarian cancer (diagnosed at any age) who do not share the BRCA1-linked haplotype segregating in the remainder of the cases in the kindred. | | | | | | |
| ² Multipoint LOD score calculated using both markers | | | | | | |
| * kindred contains one individual who had both breast and ovarian cancer; this individual is counted as a breast cancer case and as an ovarian cancer case. | | | | | | |

The logarithm of the odds (LOD) scores in these kindreds range from 9.49 to -0.44 for a set of markers in 17q21. Four of the families have convincing LOD scores for linkage, and 4 have low positive or negative LOD scores. The latter kindreds were included because they demonstrate haplotype sharing at chromosome 17q21 for at least 3 affected members. Furthermore, all kindreds in the set display early age of breast cancer onset and 4 of the kindreds include at least one case of ovarian cancer, both hallmarks of BRCA1 kindreds. One kindred, 2082, has nearly equal incidence of breast and ovarian cancer, an unusual occurrence given the relative rarity of ovarian cancer in the population. All of the kindreds except two were ascertained in Utah. K2035 is from the midwest. K2099 is an African-American kindred from the southern USA.

In the initial screen for predisposing mutations in BRCA1, DNA from one individual who carries the predisposing haplotype in each kindred was tested. The 23 coding exons and associated splice junctions were amplified either from genomic DNA samples or from cDNA prepared from lymphocyte mRNA. When the amplified DNA sequences were compared to the wildtype sequence, 4 of the 8 kindred samples were found to contain sequence variants (Table 11).

**TABLE 11**

| PREDISPOSING MUTATIONS | | | |
|---|---|---|---|
| Kindred Number | Mutation | Coding Effect | Location* |
| 2082 | C→T | Gln→Stop | 4056 |
| 1910 | extra C | frameshift | 5385 |
| 2099 | T→G | Met→Arg | 5443 |
| 2035 | ? | loss of transcript | |
| 1901 | 11 bp deletion | frameshift | 189 |

| | | | |
|---|---|---|---|
| * In Sequence ID NO:1 | | | |

All four sequence variants are heterozygous and each appears in only one of the kindreds. Kindred 2082 contains a nonsense mutation in coding exon 10 (Fig. 9A), Kindred 1910 contains a single nucleotide insertion in coding exon 19 (Fig. 9B), and Kindred 2099 contains a missense mutation in coding exon 20, resulting in a Met→Arg substitution (Fig. 9C). The frameshift and nonsense mutations are likely disruptive to the function of the BRCA1 product. The peptide encoded by the frameshift allele in Kindred 1910 would contain an altered amino acid sequence beginning 107 residues from the wildtype C-terminus. The peptide encoded by the frameshift allele in Kindred 1901 would contain an altered amino acid sequence beginning with the 24th residue from the wildtype N-terminus. The mutant allele in Kindred 2082 would encode a protein missing 548 residues from the C-terminus. The missense substitution observed in Kindred 2099 is potentially disruptive as it causes the replacement of a small hydrophobic amino acid (Met), by a large charged residue (Arg). Eleven common polymorphisms were also identified, 8 in coding sequence and 3 in introns.

The individual studied in Kindred 2035 evidently contains a regulatory mutation in BRCA1. In her cDNA, a polymorphic site (A→G at base 3667) appeared homozygous, whereas her genomic DNA revealed heterozygosity at this position (Fig. 9C). A possible explanation for this observation is that mRNA from her mutated BRCA1 allele is absent due to a mutation that affects its production or stability. This possibility was explored further by examining 5 polymorphic sites in the BRCA1 coding region, which are separated by as much as 3.5 kb in the BRCA1 transcript. In all cases where her genomic DNA appeared heterozygous for a polymorphism, cDNA appeared homozygous. In individuals from other kindreds and in non-haplotype carriers in Kindred 2035, these polymorphic sites could be observed as heterozygous in cDNA, implying that amplification from cDNA was not biased in favor of one allele. This analysis indicates that a BRCA1 mutation in Kindred 2035 either prevents transcription or causes instability or aberrant splicing of the BRCAI transcript.

Cosegregation of BRCA1 mutations with BRCA1 haplotypes and population frequency analysis. In addition to potentially disrupting protein function, two criteria must be met for a sequence variant to qualify as a candidate predisposing mutation. The variant must: 1) be present in individuals from the kindred who carry the predisposing BRCA1 haplotype and absent in other members of the kindred, and 2) be rare in the general population.

Each mutation was tested for cosegregation with BRCA1. For the frameshift mutation in Kindred 1910, two other haplotype carriers and one non-carrier were sequenced (Fig. 9B). Only the carriers exhibited the frameshift mutation. The C to T change in Kindred 2082 created a new AvrII restriction site. Other carriers and non-carriers in the kindred were tested for the presence of the restriction site (Fig. 9A). An allele-specific oligonucleotide (ASO) was designed to detect the presence of the sequence variant in Kindred 2099. Several individuals from the kindred, some known to carry the haplotype associated with the predisposing allele, and others known not to carry the associated haplotype, were screened by ASO for the mutation previously detected in the kindred. In each kindred, the corresponding mutant allele was detected in individuals carrying the BRCA1-associated haplotype, and was not detected in noncarriers. In the case of the potential regulatory mutation observed in the individual from Kindred 2035, cDNA and genomic DNA from carriers in the kindred were compared for heterozygosity at polymorphic sites. In every instance, the extinguished allele in the cDNA sample was shown to lie on the chromosome that carries the BRCA1 predisposing allele (Fig. 9C).

To exclude the possibility that the mutations were simply common polymorphisms in the population, ASOs for each mutation were used to screen a set of normal DNA samples. Gene frequency estimates in Caucasians were based on random samples from the Utah population. Gene frequency estimates in African-Americans were based on 39 samples provided by M. Peracek-Vance which originate from African-Americans used in her linkage studies and 20 newborn Utah African-Americans. None of the 4 potential predisposing mutations was found in the appropriate control population, indicating that they are rare in the general population. Thus, two important requirements for BRCA1 susceptibility alleles were fulfilled by the candidate predisposing mutations: 1) cosegregation of the mutant allele with disease, and 2) absence of the mutant allele in controls, indicating a low gene frequency in the general population.

Phenotypic Expression of BRCA1 Mutations. The effect of the mutations on the BRCA1 protein correlated with differences in the observed phenotypic expression in the BRCA1 kindreds. Most BRCA1 kindreds have a moderately increased ovarian cancer risk, and a smaller subset have high risks of ovarian cancer, comparable to those for breast cancer (Easton *et al.,* 1993). Three of the four kindreds in which BRCA1 mutations were detected fall into the former category, while the fourth (K2082) falls into the high ovarian cancer risk group. Since the BRCA1 nonsense mutation found in K2082 lies closer to the amino terminus than the other mutations detected, it might be expected to have a different phenotype. In fact, Kindred K2082 mutation has a high incidence of ovarian cancer, and a later mean age at diagnosis of breast cancer cases than the other kindreds (Goldgar *et al.,* 1994). This difference in age of onset could be due to an ascertainment bias in the smaller, more highly penetrant families, or it could reflect tissue-specific differences in the behavior of BRCA1 mutations. The other 3 kindreds that segregate known BRCA1 mutations have, on average, one ovarian cancer for every 10 cases of breast cancer, but have a high proportion of breast cancer cases diagnosed in their late 20's or early 30's. Kindred 1910, which has a frameshift mutation, is noteworthy because three of the four affected individuals had bilateral breast cancer, and in each case the second tumor was diagnosed within a year of the first occurrence. Kindred 2035, which segregates a potential regulatory BRCA1 mutation, might also be expected to have a dramatic phenotype. Eighty percent of breast cancer cases in this kindred occur under age 50. This figure is as high as any in the set, suggesting a BRCA1 mutant allele of high penetrance (Table 10).

Although the mutations described above clearly are deleterious, causing breast cancer in women at very young ages, each of the four kindreds with mutations includes at least one woman who carries the mutation who lived until age 80 without developing a malignancy. It will be of utmost importance in the studies that follow to identify other genetic or environmental factors that may ameliorate the effects of BRCA1 mutations.

In four of the eight putative BRCA1-linked kindreds, potential predisposing mutations were not found. Three of these four have LOD scores for BRCA1-linked markers of less than 0.55. Thus, these kindreds may not in reality segregate BRCA1 predisposing alleles. Alternatively, the mutations in these four kindreds may lie in regions of BRCA1 that, for example, affect the level of transcript and therefore have thus far escaped detection.

Role of BRCA1 in Cancer. Most tumor suppressor genes identified to date give rise to protein products that are absent, nonfunctional, or reduced in function. The majority of TP53 mutations are missense; some of these have been shown to produce abnormal p53 molecules that interfere with the function of the wildtype product (Shaulian *et al*. 1992; Srivastava *et al.,* 1993). A similar dominant negative mechanism of action has been proposed for some adenomatous polyposis coli (APC) alleles that produce truncated molecules (Su *et al.,* 1993), and for point mutations in the Wilms' tumor gene (WT1) that alter DNA binding of the protein (Little *et al.,* 1993). The nature of the mutations observed in the BRCA1 coding sequence is consistent with production of either dominant negative proteins or nonfunctional proteins. The regulatory mutation inferred in Kindred 2035 cannot be a dominant negative; rather, this mutation likely causes reduction or complete loss of BRCA1 expression from the affected allele.

The BRCA1 protein contains a C₃HC₄ zinc-finger domain, similar to those found in numerous DNA binding proteins and implicated in zinc-dependent binding to nucleic acids. The first 180 amino acids of BRCA1 contain five more basic residues than acidic residues. In contrast, the remainder of the molecule is very acidic, with a net excess of 70 acidic residues. The excess negative charge is particularly concentrated near the C-terminus. Thus, one possibility is that BRCA1 encodes a transcription factor with an N-terminal DNA binding domain and a C-terminal transactivational "acidic blob" domain. Interestingly, another familial tumor suppressor gene, WT1, also contains a zinc-finger motif (Haber *et al.,* 1990). Many cancer predisposing mutations in WT1 alter zinc-finger domains (Little *et al.,* 1993; Haber *et al.,* 1990; Little *et al.,* 1992). WT1 encodes a transcription factor, and alternative splicing of exons that encode parts of the zinc-finger domain alter the DNA binding properties of WT1 (Bickmore *et al.,* 1992). Some alternatively spliced forms of WT1 mRNA generate molecules that act as transcriptional repressors (Drummond *et al.,* 1994). Some BRCA1 splicing variants may alter the zinc-finger motif, raising the possibility that a regulatory mechanism similar to that which occurs in WT1 may apply to BRCA1.

### EXAMPLE 9

### Analysis of Tumors for BRCA1 Mutations

To focus the analysis on tumors most likely to contain BRCA1 mutations, primary breast and ovarian carcinomas were typed for LOH in the BRCA1 region. Three highly polymorphic, simple tandem repeat markers were used to assess LOH: D17S1323 and D17S855, which are intragenic to BRCA1, and D17S1327, which lies approximately 100 kb distal to BRCA1. The combined LOH frequency in informative cases (i.e., where the germline was heterozygous) was 32/72 (44%) for the breast carcinomas and 12/21 (57%) for the ovarian carcinomas, consistent with previous measurements of LOH in the region (Futreal *et al*., 1992b; Jacobs *et al.,* 1993; Sato *et al.,* 1990; Eccles *et al.,* 1990; Cropp *et al*., 1994). The analysis thus defined a panel of 32 breast tumors and 12 ovarian tumors of mixed race and age of onset to be examined for BRCA mutations. The complete 5,589 bp coding region and intron/exon boundary sequences of the gene were screened in this tumor set by direct sequencing alone or by a combination of single-strand conformation analysis (SSCA) and direct sequencing.

A total of six mutations was found, one in an ovarian tumor, four in breast tumors and one in a male unaffected haplotype carrier (Table 12). One mutation, Glu1541Ter, introduced a stop codon that would create a truncated protein missing 273 amino acids at the carboxy terminus. In addition, two missense mutations were identified. These are Ala1708Glu and Met1775Arg and involve substitutions of small, hydrophobic residues by charged residues. Patients 17764 and 19964 are from the same family. In patient OV24 nucleotide 2575 is deleted and in patients 17764 and 19964 nucleotides 2993-2996 are deleted.

**TABLE 12**

| Predisposing Mutations | | | | | |
|---|---|---|---|---|---|
| Patient | Codon | Nucleotide Change | Amino Acid Change | Age of Onset | Family History |
| BT098 | 1541 | GAG → TAG | Glu → Stop | 39 | - |
| OV24 | 819 | 1 bp deletion | frameshift | 44 | - |
| BT106 | 1708 | GCG → GAG | Ala → Glu | 24 | + |
| MC44 | 1775 | ATG → AGG | Met → Arg | 42 | + |
| 17764 | 958 | 4 bp deletion | frameshift | 31 | + |
| 19964 | 958 | 4 bp deletion | frameshift | | +* |

| | | | | | |
|---|---|---|---|---|---|
| * Unaffected haplotype carrier, male | | | | | |

Several lines of evidence suggest that all five mutations represent BRCA1 susceptibility alleles:
(i) all mutations are present in the germline;
(ii) all are absent in appropriate control populations, suggesting they are not common polymorphisms;
(iii) each mutant allele is retained in the tumor, as is the case in tumors from patients belonging to kindreds that segregate BRCA1 susceptibility alleles (Smith *et al*., 1992; Kelsell *et al*., 1993) (if the mutations represented neutral polymorphisms, they should be retained in only 50% of the cases);
(iv) the age of onset in the four breast cancer cases with mutations varied between 24 and 42 years of age, consistent with the early age of onset of breast cancer in individuals with BRCA1 susceptibility; similarly, the ovarian cancer case was diagnosed at 44, an age that falls in the youngest 13% of all ovarian cancer cases; and finally,
(v) three of the five cases have positive family histories of breast or ovarian cancer found retrospectively in their medical records, although the tumor set was not selected with regard to this criterion.

BT106 was diagnosed at age 24 with breast cancer. Her mother had ovarian cancer, her father had melanoma, and her paternal grandmother also had breast cancer. Patient MC44, an African-American, had bilateral breast cancer at age 42. This patient had a sister who died of breast cancer at age 34, another sister who died of lymphoma, and a brother who died of lung cancer. Her mutation (Met1775Arg) had been detected previously in Kindred 2099, an African-American family that segregates a BRCA1 susceptibility allele, and was absent in African-American and Caucasian controls. Patient MC44, to our knowledge, is unrelated to Kindred 2099. The detection of a rare mutant allele, once in a BRCA1 kindred and once in the germline of an apparently unrelated early-onset breast cancer case, suggests that the Met1775Arg change may be a common predisposing mutation in African-Americans. Collectively, these observations indicate that all four BRCA1 mutations in tumors represent susceptibility alleles; no somatic mutations were detected in the samples analyzed.

The paucity of somatic BRCA1 mutations is unexpected, given the frequency of LOH on 17q, and the usual role of susceptibility genes as tumor suppressors in cancer progression. There are three possible explanations for this result: (i) some BRCA1 mutations in coding sequences were missed by our screening procedure; (ii) BRCA1 somatic mutations fall primarily outside the coding exons; and (iii) LOH events in 17q do not reflect BRCA1 somatic mutations.

If somatic BRCA1 mutations truly are rare in breast and ovary carcinomas, this would have strong implications for the biology of BRCA1. The apparent lack of somatic BRCA1 mutations implies that there may be some fundamental difference in the genesis of tumors in genetically predisposed BRCA1 carriers, compared with tumors in the general population. For example, mutations in BRCA1 may have an effect only on tumor formation at a specific stage early in breast and ovarian development. This possibility is consistent with a primary function for BRCA1 in premenopausal breast cancer. Such a model for the role of BRCA1 in breast and ovarian cancer predicts an interaction between reproductive hormones and BRCA1 function. However, no clinical or pathological differences in familial versus sporadic breast and ovary tumors, other than age of onset, have been described (Lynch *et al.,* 1990). On the other hand, the recent finding of increased TP53 mutation and microsatellite instability in breast tumors from patients with a family history of breast cancer (Glebov *et al.,* 1994) may reflect some difference in tumors that arise in genetically predisposed persons. The involvement of BRCA1 in this phenomenon can now be addressed directly. Alternatively, the lack of somatic BRCA1 mutations may result from the existence of multiple genes that function in the same pathway of tumor suppression as BRCA1, but which collectively represent a more favored target for mutation in sporadic tumors. Since mutation of a single element in a genetic pathway is generally sufficient to disrupt the pathway, BRCA1 might mutate at a rate that is far lower than the sum of the mutational rates of the other elements.

A separate study to analyze tumors for BRCA1 mutations was performed in Japan. A panel of 103 patients representing early-onset cases (<35 years of age) (46 patients), members of multiply-affected families (12 patients), and/or had developed bilateral breast cancers (59 patients) were screened for mutations in BRCA1. Primary breast tumors from these patients were screened for mutations in coding exons of BRCA1 using single-strand conformation polymorphism (SSCP) analysis. For exon 11, which is 3425 bp long, PCR primers were designed to amplify eleven overlapping segments of this exon separately. Each of the other 22 exons was amplified individually in a single PCR. Thus 33 PCR-SSCP analyses were carried out for each case. Mutations were detected in tumors from four patients, all of whom had developed breast cancers bilaterally (Table 12A). One mutation resulted in a frame shift due to a 2 bp deletion (deletion of AA) at codon 797. This gives rise to a truncated protein missing 1065 amino acids at the COOH terminus. A second mutation was a nonsense mutation at codon 1214 due to a G → T transversion of the first nucleotide of the codon. This results in a premature stop codon in place of glutamic acid at this site and results in a truncated protein missing 649 amino acids at the COOH terminus. There were also two missense mutations. One was a G → A transition at the first nucleotide of codon 271 resulting in a Val → Met substitution. The second was at codon 1150 (a C → T transition in the first nucleotide of the codon) causing a Pro → Ser substitution, a replacement of a hydrophobic nonpolar amino acid with a polar uncharged amino acid. These mutations were all found to be germline mutations. The mean age of onset in these four patients was 49. These studies also found a common neutral polymorphism of either C or T at the first nucleotide of codon 771.

**TABLE 12A**

| Predisposing Mutations | | | | |
|---|---|---|---|---|
| Patient | Codon | Nucleotide Change | Amino Acid Change | Age of Onset |
| 23 | 1150 | CCT→TCT | Pro→Ser | 49 & 64 |
| 44 | 1214 | GAG→TAG | Glu→Stop | 51 & 51 |
| 98 | 271 | GTG→ATG | Val→Met | 45 & 45 |
| 100 | 797 | 2bp deletion | frameshift | 50 & 71 |
| 5 | 482-483 | 4bp deletion | frameshift | 45 |
| 6 | 856 | TAT→CAT | Tyr→His | 54 |
| 7 | 271 | GTG→ATG | Val→Met | 49 & 49 |
| 8 | 852 | 1bp deletion | frameshift | 62 |

Although patients 98 and 7 show the same mutation, they are not related to each other.

### EXAMPLE 10

### Analysis of the BRCA1 Gene

The structure and function BRCA 1 gene are determined according to the following methods.

Biological Studies. Mammalian expression vectors containing BRCA1 cDNA are constructed and transfected into appropriate breast carcinoma cells with lesions in the gene. Wild-type BRCA1 cDNA as well as altered BRCA1 cDNA are utilized. The altered BRCA1 cDNA can be obtained from altered BRCA1 alleles or produced as described below. Phenotypic reversion in cultures (e.g., cell morphology, doubling time, anchorage-independent growth) and in animals (e.g., tumorigenicity) is examined. The studies will employ both wild-type and mutant forms (Section B) of the gene.

Molecular Genetics Studies. *In vitro* mutagenesis is performed to construct deletion mutants and missense mutants (by single base-pair substitutions in individual codons and cluster charged → alanine scanning mutagenesis). The mutants are used in biological, biochemical and biophysical studies.

Mechanism Studies. The ability of BRCA1 protein to bind to known and unknown DNA sequences is examined. Its ability to transactivate promoters is analyzed by transient reporter expression systems in mammalian cells. Conventional procedures such as particle-capture and yeast two-hybrid system are used to discover and identify any functional partners. The nature and functions of the partners are characterized. These partners in turn are targets for drug discovery.

Structural Studies. Recombinant proteins are produced in *E. coli,* yeast, insect and/or mammalian cells and are used in crystallographical and NMR studies. Molecular modeling of the proteins is also employed. These studies facilitate structure-driven drug design.

### EXAMPLE 11

### Two Step Assay to Detect the Presence of BRCA1 in a Sample

Patient sample is processed according to the method disclosed by Antonarakis, *et al.* (1985), separated through a 1% agarose gel and transferred to nylon membrane for Southern blot analysis. Membranes are UV cross linked at 150 mJ using a GS Gene Linker (Bio-Rad). BRCAI probe corresponding to nucleotide positions 3631-3930 of SEQ ID NO:1 is subcloned into pTZ18U. The phagemids are transformed into *E. coli* MV1190 infected with M13K07 helper phage (Bio-Rad, Richmond, CA). Single stranded DNA is isolated according to standard procedures (see Sambrook, *et al*., 1989).

Blots are prehybridized for 15-30 min at 65°C in 7% sodium dodecyl sulfate (SDS) in 0.5 M NaPO₄. The methods follow those described by Nguyen, *et al.,* 1992. The blots are hybridized overnight at 65°C in 7% SDS, 0.5 M NaPO₄ with 25-50 ng/ml single stranded probe DNA. Post-hybridization washes consist of two 30 min washes in 5% SDS, 40 mM NaPO₄ at 65°C, followed by two 30-min washes in 1% SDS, 40 mM NaPO₄ at 65°C.

Next the blots are rinsed with phosphate buffered saline (pH 6.8) for 5 min at room temperature and incubated with 0.2% casein in PBS for 30-60 min at room temperature and rinsed in PBS for 5 min. The blots are then preincubated for 5-10 minutes in a shaking water bath at 45°C with hybridization buffer consisting of 6 M urea, 0.3 M NaCl, and 5X Denhardt's solution (see Sambrook, *et al.,* 1989). The buffer is removed and replaced with 50-75 µl/cm² fresh hybridization buffer plus 2.5 nM of the covalently cross-linked oligonucleotide-alkaline phosphatase conjugate with the nucleotide sequence complementary to the universal primer site (UP-AP, Bio-Rad). The blots are hybridized for 20-30 min at 45°C and post hybridization washes are incubated at 45°C as two 10 min washes in 6 M urea, 1x standard saline citrate (SSC), 0.1% SDS and one 10 min wash in lx SSC, 0.1% Triton® X-100. The blots are rinsed for 10 min at room temperature with 1x SSC.

Blots are incubated for 10 min at room temperature with shaking in the substrate buffer consisting of 0.1 M diethanolamine, 1 mM MgCl₂, 0.02% sodium azide, pH 10.0. Individual blots are placed in heat sealable bags with substrate buffer and 0.2 mM AMPPD ( 3-(2'-spiroadamantane)-4-methoxy-4-(3'-phosphoryloxy)phenyl-1,2-dioxetane, disodium salt, Bio-Rad). After a 20 min incubation at room temperature with shaking, the excess AMPPD solution is removed. The blot is exposed to X-ray film overnight. Positive bands indicate the presence of BRCA1.

### EXAMPLE 12

### Generation of Polyclonal Antibody against BRCA1

Segments of BRCA1 coding sequence were expressed as fusion protein in *E. coli*. The overexpressed protein was purified by gel elution and used to immunize rabbits and mice using a procedure similar to the one described by Harlow and Lane, 1988. This procedure has been shown to generate Abs against various other proteins (for example, see Kraemer, *et al.,* 1993).

Briefly, a stretch of BRCA1 coding sequence was cloned as a fusion protein in plasmid PET5A (Novagen, Inc., Madison, WI). The BRCA1 incorporated sequence includes the amino acids corresponding to #1361-1554 of SEQ ID NO:2. After induction with IPTG, the overexpression of a fusion protein with the expected molecular weight was verified by SDS/PAGE. Fusion protein was purified from the gel by electroelution. The identification of the protein as the BRCA1 fusion product was verified by protein sequencing at the N-terminus. Next, the purified protein was used as immunogen in rabbits. Rabbits were immunized with 100 µg of the protein in complete Freund's adjuvant and boosted twice in 3 week intervals, first with 100 µg of immunogen in incomplete Freund's adjuvant followed by 100 µg of immunogen in PBS. Antibody containing serum is collected two weeks thereafter.

This procedure is repeated to generate antibodies against the mutant forms of the BRCA1 gene. These antibodies, in conjunction with antibodies to wild type BRCA1, are used to detect the presence and the relative level of the mutant forms in various tissues and biological fluids.

### EXAMPLE 13

### Generation of Monoclonal Antibodies Specific for BRCA1

Monoclonal antibodies are generated according to the following protocol. Mice are immunized with immunogen comprising intact BRCA1 or BRCA1 peptides (wild type or mutant) conjugated to keyhole limpet hemocyanin using glutaraldehyde or EDC as is well known.

The immunogen is mixed with an adjuvant. Each mouse receives four injections of 10 to 100 µg of immunogen and after the fourth injection blood samples are taken from the mice to determine if the serum contains antibody to the immunogen. Serum titer is determined by ELISA or RIA. Mice with sera indicating the presence of antibody to the immunogen are selected for hybridoma production.

Spleens are removed from immune mice and a single cell suspension is prepared (see Harlow and Lane, 1988). Cell fusions are performed essentially as described by Kohler and Milstein, 1975. Briefly, P3.65.3 myeloma cells (American Type Culture Collection, Rockville, MD) are fused with immune spleen cells using polyethylene glycol as described by Harlow and Lane, 1988. Cells are plated at a density of 2x10⁵ cells/well in 96 well tissue culture plates. Individual wells are examined for growth and the supernatants of wells with growth are tested for the presence of BRCA1 specific antibodies by ELISA or RIA using wild type or mutant BRCA1 target protein. Cells in positive wells are expanded and subcloned to establish and confirm monoclonality.

Clones with the desired specificities are expanded and grown as ascites in mice or in a hollow fiber system to produce sufficient quantities of antibody for characterization and assay development.

### EXAMPLE 14

### Sandwich Assay for BRCA1

Monoclonal antibody is attached to a solid surface such as a plate, tube, bead, or particle. Preferably, the antibody is attached to the well surface of a 96-well ELISA plate. 100 µl sample (e.g., serum, urine, tissue cytosol) containing the BRCA1 peptide/protein (wild-type or mutant) is added to the solid phase antibody. The sample is incubated for 2 hrs at room temperature. Next the sample fluid is decanted, and the solid phase is washed with buffer to remove unbound material. 100 µl of a second monoclonal antibody (to a different determinant on the BRCA1 peptide/protein) is added to the solid phase. This antibody is labeled with a detector molecule (e.g., 125-I, enzyme, fluorophore, or a chromophore) and the solid phase with the second antibody is incubated for two hrs at room temperature. The second antibody is decanted and the solid phase is washed with buffer to remove unbound material.

The amount of bound label, which is proportional to the amount of BRCA1 peptide/protein present in the sample, is quantitated. Separate assays are performed using monoclonal antibodies which are specific for the wild-type BRCA1 as well as monoclonal antibodies specific for each of the mutations identified in BRCA1.

### EXAMPLE 15

### Analysis of BRCA1 Mutations

The DNA samples which were screened for BRCA1 mutations were extracted from blood or tumor samples from patients with breast or ovarian cancer (or known carriers by haplotype analysis) who were participating in research studies on the genetics of breast cancer. All subjects signed appropriate informed consent. Table 13 details the number of samples, ascertainment criteria, and screening method for each set of samples screened.

**TABLE 13**

| Sets of DNA Samples Screened for Mutations in RRCA1 | | | | |
|---|---|---|---|---|
| Source of Samples | Description of Samples¹ | Screening Method² | No. Samples Screened | No. Mutations Found to Date |
| UTAH-2 | Br/Ov Families | SEQ | 10 | 2 |
| MONTREAL | Br/Ov Families | SEQ | 30 | 13 |
| MSKCC-1 | Br and Br/Ov Families | SEQ | 14 | 2 |
| MSK/UT-1 | Early Onset Br Cases | SEQ | 24 | 1 |
| STRANG | Br and Br/Ov Families | SEQ | 12 | 4 |
| STOCKHOLM | Br and Br/Ov Families | SEQ | 15 | 4 |
| USC-1 | Bilat Br Proband, High-Risk | SEQ | 7 | 3 |
| TUMOR-3 | Early Onset Br Tumors | SEQ | 14 | 1 |
| USC-2 | Bilat Br<50 + 1° rel Br | ASO | 59 | 5 |
| MSK/UT-2 | Early Onset Br Cases | ASO | 109 | 3 |
| YN | Bilateral; Early Onset | SSCA | 103 | 4 |
| Texas | Br/Ov Families | SEQ | 15 | 2 |
| Utah | Br/Ov Families | SEQ | 10 | 1 |
| Pisa | Br/Ov Families | SEQ | 21 | 4 |
| Tumor1mod | | SEQ | | 1 |
| MSKCC-2 | Early Onset Br Cases | SEQ | 21 | 3 |

| | | | | |
|---|---|---|---|---|
| 1 Most sample groups contained a heterogeneous mixture of samples. The most representative description of each set is given. 2 SEQ - Direct sequencing of PCR products; SSCA - Single Strand Conformation Assay; ASO - Allele-Specific Oligo | | | | |

Although the original mutations described in Miki *et al.,* 1994 were detected through screening of cDNA, 25 pairs of intronic PCR primers were used to amplify the complete coding sequence and splice junctions from genomic DNA for the majority of the remaining samples. Updated primer information is publicly available via anonymous ftp from morgan.med.utah.edu in the directory pub/BRCA1. Where possible, DNA sequence variations were tested for cosegregation with breast or ovarian cancer in the family. Further evidence of a causal role of a sequence variant in cancer was provided by proving the absence of the putative mutation in a set of control individuals. Screening for specific, previously-identified mutations in large sets of selected samples was performed using ASO hybridization.

Table 14 describes many of the mutations found screening the entire BRCA1 coding sequence as well as the intron/exon boundaries and by finding polymorphic sites in genomic DNA reduced to monomorphic sites in cDNA. Two common mutations were found and their frequencies in other samples were examined by ASO analysis (Table 15). Tables 16 and 17 describe the distribution of mutations by type and by location within the BRCA1 coding sequence, respectively. By far, the majority of mutations identified were frameshifts. Globally, no statistically significant departure from a random distribution across the coding sequence of BRCA1 was found (χ² = 2.00, 2 df, p = 0.37) among the distinct mutations found in the coding sequence of BRCA1 to date.

**TABLE 14**

| | Mutations Identified by Complete Screening of the RRCA1 Gene | | | | | | |
|---|---|---|---|---|---|---|---|
| Sample Set | Family | # Cases Mutation | | Description | | | |
| | | BR | OV | Type¹ | Exon | Codon | Mutation² |
| TEXAS | 132-000 | | | FS | 2 | 23 | 185 ins A -> ter 40 |
| MONTREAL | 180 | 2 | 2 | FS | 2 | 23 | 185 del AG -> ter 39 |
| MONTREAL | 235 | 4 | 2 | FS | 2 | 23 | 185 del AG -> ter 39 |
| MONTREAL | 253 | 1 | 3 | FS | 2 | 23 | 185 del AG -> ter 39 |
| MONTREAL | 255 | 0 | 7 | FS | 2 | 23 | 185 del AG -> ter 39 |
| MSKCC | 210311 | 3 | 0 | FS | 2 | 23 | 185 del AG -> ter 39 |
| USC-1 | 008 | 2 | 1 | FS | 2 | 23 | 185 del AG -> ter 39 |
| PISA | 27 | 8 | 5 | MS | 5 | 64 | Cys 64 Arg |
| UTAH | | | | SP | 1-5 | 1-5 | T->G ins 59 -> ter 75 |
| MSKCC | 19921 | | | SP | 1-6 | 1-6 | del A at -2 of 3' splice |
| TUMOR-3⁴ | - | 1 | 0 | FS | 11 | 270 | 926 ins 10 -> ter 289 |
| MSK/UT-1 | - | 1 | 0 | FS | 11 | 270 | 926 ins 10 -> ter 289 |
| YN98 | - | 1 | 0 | MS | 11 | 271 | Val 271 Met |
| YN7 | - | 1 | 0 | MS | 11 | 271 | Val 271 Met |
| MONTREAL | 270 | 4 | 3 | FS | 11 | 339 | 1128 ins A -> ter 345 |
| STRANG | 2903 | 1 | 2 | FS | 11 | 339 | 1128 ins A -> ter 345 |
| MONTREAL | 185 | 1 | 3 | FS | 11 | 392 | 1294 del 40 -> ter 396 |
| PISA | 6 | | | FS | 11 | 461 | 1499 ins A -> ter 479 |
| PISA | 17 | | | FS | 11 | 461 | 1499 ins A -> ter 479 |
| PISA | 31 | | | FS | 11 | 461 | 1499 ins A -> ter 479 |
| YN5 | - | 1 | 0 | FS | 11 | 482 | del 4 -> ter |
| USC-1 | 052 | 5 | 1 - | FS | 11 | 655 | 2080 ins A -> ter 672 |
| USC-1 | 068 | 2 | 1 | FS | 11 | 655 | 2080 ins A -> ter 672 |
| PISA | | | | MS | 11 | 667 | Gln 667 His |
| STRANG | 2802 | 2 | 2 | FS | 11 | 725 | 2293 del G -> ter 735 |
| YN100 | - | 1 | 0 | FS | 11 | 797 | 2509 del AA -> ter 799 |
| TUMOR1mod | OV24 | 0 | 1 | FS | 11 | 819 | 2575 del C -> ter 845 |
| MONTREAL | 179 | 2 | 3 | MS | 11 | 826 | Thr 826 Lys |
| STOCKHOLM | AL48 | 3 | 1 | FS | 11 | 826 | 2596 del C -> ter 845 |
| STOCKHOLM | BR33 | 5 | 1 | FS | 11 | 826 | 2596 del C -> ter 845 |
| YN8 | | 1 | 0 | FS | 11 | 852 | del 1 -> ter 891 |
| YN6 | | 1 | 0 | MS | 11 | 856 | Tyr 856 His |
| UTAH-2 | 2305 | 2 | 7 | FS | 11 | 958 | 2993 del 4 -> ter 998 |
| MONTREAL | 218 | 5 | 1 | FS | 11 | 1002 | 3121 del A -> ter 1023 |
| MSK17572 | | | | MS | 11 | 1008 | Met 1008 Ile |
| STOCKHOLM | BR24 | 2 | 1 | FS | 11 | 1016 | 3166 ins 5 -> ter 1025 |
| MONTREAL | | | | FS | 11 | 1110 | 3447 del 4 -> ter 1115 |
| MONTREAL | 448 | | | FS | 11 | 1110 | 3449 del 4 -> ter 1115 |
| TEXAS | BC110-001 | | | FS | 11 | 1111 | 3450 del 4 -> ter 1115 |
| YN23 | - | 1 | 0 | MS | 11 | 1150 | Pro 1150 Ser |
| STOCKHOLM | PAL33 | 1 | 0 | FS | 11 | 1209 | 3745 del T -> ter 1209 |
| YN44 | - | 1 | 0 | NS | 11 | 1214 | Glu 1214 ter |
| MSK12871 | | | | MS | 11 | 1219 | Glu 1219 Asp |
| TEXAS | BC215-000 | | | FS | 11 | 1252 | 3873 del 4 -> ter 1262 |
| UTAH-2 | 2039 | 3 | 2 | MS | 11 | 1347 | Arg 1347 Gly |
| MONTREAL | 183 | 4 | 1 | FS | 11 | 1355 | 4184 del 4 -> ter 1364 |
| STRANG | 1900³ | 3 | 1 | NS | 13 | 1443 | Arg 1443 ter |
| TUMOR-2 | | 1 | 0 | NS | 15 | 1541 | Glu 1541 ter |
| PISA | #8 | | | FS | 16 | 1585 | 4873 del CA -> ter 1620 |
| MSK9646 | | | | MS | 16 | 1628 | Met 1628 Val |
| STRANG | 8622³ | 4 | 1 | FS | 16 | 1656 | 5085 del 19 -> ter 1670 |
| MONTREAL | 101 | 2 | 2 | FS | 20 | 1756 | 5382 ins C -> ter 1829 |
| MONTREAL | 162 | 3 | 1 | FS | 20 | 1756 | 5382 ins C -> ter 1829 |
| MONTREAL | 166 | 5 | 2 | FS | 20 | 1756 | 5382 ins C -> ter 1829 |
| MONTREAL | 279 | 4 | 0 | FS | 20 | 1756 | 5382 ins C -> ter 1829 |
| MSKCC | 193549 | 0 | 3 | FS | 20 | 1756 | 5382 ins C -> ter 1829 |
| MSK7542 | | | | MS | 24 | 1852 | Thr 1852 Ser |

| | | | | | | | |
|---|---|---|---|---|---|---|---|
| ¹ FS-Frameshift; NS-Nonsense; MS-Missense; SP-Splice Site. ² For Missense and Nonsense mutations, the mutation description contains: wild type amino acid, affected codon, altered amino acid (or ter). For frameshift mutations, the format is: nucleotide, insertion or deletion, specific nucleotides changed (if <3) or number inserted or deleted (if >2) and the amino acid (accounting for the insertion or deletion) in which the frameshift results in a termination signal. Nucleotides refer to the BRCA1 cDNA sequence in GENBANK under Accession No. U-14680. ³ The mutation in this family was independently identified in both the Myriad and University of Pennsylvania Labs. ⁴ The mutation identified in this tumor was also found in the germline of the individual. | | | | | | | |

**TABLE 15**

| Frequency of Two Common BRCA1 Mutations | | | |
|---|---|---|---|
| Set | Number Studied | Number of Mutations Found | |
| | | *185 del AG* | *5382 ins C* |
| USC-1 | 59 | 4 | 1 |
| MSK/UT-2 | 109 | 3 | 0 |
| GLASGOW-2 | 100 | Not tested | 3 |
| GLASGOW-3 | 100 | Not tested | 2 |
| CRC-OV | 250 | Not tested | 1 |

**TABLE 16**

| Observed Frequency of Different Types of Mutations | | |
|---|---|---|
| | Number (Percent) | |
| Mutation Type | Distinct Mutations¹ | All Mutations² |
| Frameshift | 42 (65) | 81 (72) |
| Nonsense | 10 (16) | 13 (12) |
| Missense | 9 (14) | 14 (12) |
| Other | 3 (5) | 5 (4) |

| | | |
|---|---|---|
| ¹ Identical mutations are counted only once in this column. | | |
| ² Each sample in which a mutation has been identified is counted in this column. | | |

**TABLE 17**

| Distribution of Identified Mutations in BRCA1 Coding Sequence | | | |
|---|---|---|---|
| Mutations | 1-621 | Amino Acids 622-1242 | 1243-1863 |
| Distinct | 18 | 23 | 21 |
| All | 44 | 28 | 39 |

Mutations have been found in many different regions of the gene - phenotypically severe mutations have been found both in the extreme 5' end of BRCA1 as well as in the extreme 3' portion of the gene. One such mutation found in a family with seven early-onset breast cancer cases produces a protein that is only missing the terminal 10 amino acids, indicating that this region of BRCA1 plays a role in normal gene function. It is noteworthy the overwhelming majority of alterations in BRCA1 have been either frameshift or nonsense mutations resulting in an unstable or truncated protein product.

In BRCA1, to date, two mutations appear to be relatively common. The *5382 ins C* BRCA1 mutation in codon 1756 and the *185 del AG* mutation in codon 23 were identified by direct sequencing in seven (10%) and eight (12%) of the 68 probands studied in the initial studies in which mutations were identified, respectively. In addition to these common mutations, additional mutations have been found in more than one family by a complete screen of the cDNA. Many of the probands screened to date for BRCA1 mutations were selected for having a high prior probability of having such mutations. Thus the mutations found in this set may not be representative of those which would be identified in other sets of patients. However, the two most frequent BRCA1 mutations (*5382 ins C* and *185 del AG*) have been found multiple times in targeted screening in sets of probands who were either unselected for family history or ascertained with minimal family history.

Besides the mutations shown above, many polymorphisms were also detected during the screening of samples. These polymorphisms are listed in Tables 18 and 19.

### Industrial Utility

As previously described above, the present invention provides materials and methods for use in testing BRCA1 alleles of an individual and an interpretation of the normal or predisposing nature of the alleles. Individuals at higher than normal risk might modify their lifestyles appropriately. In the case of BRCA1, the most significant non-genetic risk factor is the protective effect of an early, full term pregnancy. Therefore, women at risk could consider early childbearing or a therapy designed to simulate the hormonal effects of an early full-term pregnancy. Women at high risk would also strive for early detection and would be more highly motivated to learn and practice breast self examination. Such women would also be highly motivated to have regular mammograms, perhaps starting at an earlier age than the general population. Ovarian screening could also be undertaken at greater frequency. Diagnostic methods based on sequence analysis of the BRCA1 locus could also be applied to tumor detection and classification. Sequence analysis could be used to diagnose precursor lesions.

**TABLE 18**

| | **Polymorphisms in BRCA1 Genomic DNA Exons** | | | | |
|---|---|---|---|---|---|
| Name | Exon # | Codon | Base Position¹ | Base Change | Effect |
| | | | | | |
| PMO1 | 11 | 356 | 1186 | A ↔ G | gln ↔ arg |
| PM02 | 13 | 1436 | 4427 | T ↔ C | ser ↔ ser |
| PM03 | 16 | 1613 | 4956 | A ↔ G | ser ↔ gly |
| PM06 | 11 | 871 | 2731 | C ↔ T | pro ↔ leu |
| PM07 | 11 | 1183 | 3667 | A ↔ G | lys ↔ arg |
| PM09 | 11 | 694 | 2201 | C ↔ T | ser ↔ ser |
| PM10 | 11 | 771 | 2430 | T ↔ C | leu ↔ leu |
| PM12 | 16 | 1561 | 4801 | C ↔ T | thr ↔ ile |
| PM14 | 11 | 1038 | 3233 | A ↔ G | glu ↔ glu |
| PM17 | 9 | 197 | 710 | C ↔ T | cys ↔ cys |
| PM18 | 11 | 693 | 2196 | G ↔ A | asp ↔ asn |
| PM19 | 11 | 841 | 2640 | C ↔ T | arg ↔ trp |
| PM20 | 11 | 1040 | 3238 | G ↔ A | ser ↔ asn |
| PM21 | 4 | 61² | 48³ | C ↔ T | ala ↔ val |
| PM22 | 11 | 327 | 1100 | A ↔ G | thr ↔ thr |
| PM23 | 11 | 1316 | 4067 | C ↔ A | pre ↔ leu |
| PM24 | 11 | 1008 | 3143 | G ↔ A | met ↔ ile |
| PM25 | 11 | 1316 | 4067 | C ↔ G | phe ↔ leu |
| PM26 | 11 | 1322 | 4083 | A ↔ G | lys ↔ glu |
| PM27 | 11 | 1347 | 4158 | A ↔ G | arg ↔ gly |
| PM28 | 11 | 707 | 2240 | T ↔ C | gly ↔ gly |
| PM29 | 11 | 675 | 2144 | A ↔ C | ala ↔ ala |

| | | | | | |
|---|---|---|---|---|---|
| ¹ Base position as shown in SEQ ID NO:1 ² Codon number with exon 4 included in the coding region ³ Base position as shown in SEQ ID NO:11 (exon 4 alone) | | | | | |

**TABLE 19**

| **Polymorphisms in BRCA1 Genomic DNA Introns** | | | | |
|---|---|---|---|---|
| Name | Intron # | Base Position¹ | Base Change | Effect |
| PM04 | 11 | 15284 | C ↔ A | unknown |
| PM05 | 18 | 20334 | A ↔ G | unknown |
| PM11 | 16 | 19231 | G ↔ A | unknown |
| PM15 | 8 | 9106 | del T | unknown |
| PM16 | 22 | 22914 | T ↔ C | unknown |
| PMA02.1 | 1 | 1295 | G ↔ A | unknown |
| PMA03.1 | 2 | 2141 | G ↔ C | unknown |
| PMA06.1 | 5 | 3653 | A ↔ G | unknown |
| PMA07.1 | 7 | insert between 4391-4392 | TTC | unknown |
| PMA08.1 | 7 | 6538 | C ↔ T | unknown |
| PMA08.2 | 8 | 6823 | A ↔ T | unknown |
| PMA09.2 | 9 | 9376 | T ↔ C | unknown |
| PMA13.1 | 13 | 16243 | G ↔ A | unknown |
| PMA15.1 | 14 | insert between 17335-17336 | CCAAC | unknown |
| PMA15.2 | 14 | 17399 | A ↔ T | unknown |
| PMA15.3 | 14 | 17473 | C ↔ G | unknown |
| PMA18.1 | 17 | 20138 | C ↔ T | unknown |
| PMA22.1 | 21 | 22680 | A ↔ G | unknown |

| | | | | |
|---|---|---|---|---|
| ¹ Base position as shown in Figures 10A-H | | | | |

With the evolution of the method and the accumulation of information about BRCA1 and other causative loci, it could become possible to separate cancers into benign and malignant.

Women with breast cancers may follow different surgical procedures if they are predisposed, and therefore likely to have additional cancers, than if they are not predisposed. Other therapies may be developed, using either peptides or small molecules (rational drug design). Peptides could be the missing gene product itself or a portion of the missing gene product. Alternatively, the therapeutic agent could be another molecule that mimics the deleterious gene's function, either a peptide or a nonpeptidic molecule that seeks to counteract the deleterious effect of the inherited locus. The therapy could also be gene based, through introduction of a normal BRCA1 allele into individuals to make a protein which will counteract the effect of the deleterious allele. These gene therapies may take many forms and may be directed either toward preventing the tumor from forming, curing a cancer once it has occurred, or stopping a cancer from metastasizing.

### LIST OF REFERENCES

Altschul, S.F. et al. (1990). J. Mol. Biol. 215: 195-197.
American Cancer Society, Cancer Facts & Figures - 1992. (American Cancer Society, Atlanta, GA).
Anand, R. (1992). Techniques for the Analysis of Complex Genomes, (Academic Press).
Anderson, et al. (1980). Proc. Natl. Acad. Sci. USA 77:5399-5403.
Anderson, D.E. (1972). J. Natl. Cancer Inst. 48:1029-1034.
Anderson, J.A., et al. (1992). J. Otolaryngology 21:321.
Antonarakis, S.E., et al. (1985). New Eng. J. Med. 313:842-848.
Ausubel, F.M., et al. (1992). Current Protocols in Molecular Biology, (J. Wiley and Sons, N.Y.)
Beaucage & Carruthers (1981). Tetra. Letts. 22:1859-1862.
Berkner (1992). Curr. Top. Microbiol. Immunol. 158:39-61.
Berkner, et al. (1988). BioTechniques 6:616-629.
Bickmore, W.A., et al. (1992). Science 257:235-7.
Bishop, D.T., et al. (1988). Genet. Epidemiol. 5:151-169.
Bishop, D.T. and Gardner, E.J. (1980). In: Banbury Report 4: Cancer Incidence in Defined Populations (J. Cairns, J.L. Lyon, M. Skolnick, eds.), Cold Spring Harbor Laboratory, Cold Spring Harbor, N.Y., 309-408.
Botstein, et al. (1980). Am. J. Hum. Genet. 32:314-331.
Bowcock, A.M., et al. (1993). Am. J. Hum. Genet. 52:718.
Brandyopadhyay and Temin (1984). Mol. Cell. Biol. 4:749-754.
Breakfield and Geller (1987). Mol. Neurobiol. 1:337-371.
Brinster, et al. (1981). Cell 27:223-231.
Buchschacher and Panganiban (1992). J. Virol. 66:2731-2739.
Buckler, et al. (1991). Proc. Natl. Acad. Sci. USA 88:4005-4009.
Cannon-Albright, L., et al. (1994). Cancer Research 54:2378-2385.
Capecchi, M.R. (1989). Science 244:1288.
Cariello (1988). Human Genetics 42:726.
Claus, E., et al. (1991). Am. J. Hum. Genet. 48:232-242.
Conner, B.J., et al. (1983). Proc. Natl. Acad. Sci. USA 80:278-282.
Constantini and Lacy (1981). Nature 294:92-94.
Cotten, et al. (1990). Proc. Natl. Acad. Sci. USA 87:4033-4037.
Cotton, et al. (1988). Proc. Natl. Acad. Sci. USA 85:4397.
Cropp, C.S., et al. (1994). Cancer Res. 54:2548-2551.
Culver, et al. (1992). Science 256:1550-1552.
Curiel, et al. (1991a). Proc. Natl. Acad. Sci. USA 88:8850-8854.
Curiel, et al. (1991b). Hum. Gene Ther. 3:147-154.
Deutscher, M. (1990). Meth. Enzymology 182 (Academic Press, San Diego, Cal.).
Donehower, L.A., et al. (1992). Nature 356:215.
Drummond, I. A., et al. (1994). Mol. Cell Biol. 14:3800-9.
Easton, D., et al. (1993). Am. J. Hum. Genet. 52:678-701.
Eccles, D.M., et al. (1990). Oncogene 5:1599-1601.
Enhancers and Eurkaryotic Gene Expression, Cold Spring Harbor Press, Cold Spring Harbor, New York (1983).
Erickson, J. et al., (1990). Science 249:527-533.
Fain, P.R. (1992). Cytogen. Cell Genet. 60:178.
Felgner, et al. (1987). Proc. Natl. Acad. Sci. USA 84:7413-7417.
Fiers, et al. (1978). Nature 273:113.
Fink, et al. (1992). Hum. Gene Ther. 3:11-19.
Finkelstein, J., et al. (1990). Genomics 7:167-172.
Freese, et al. (1990). Biochem. Pharmacol. 40:2189-2199.
Friedman, T. (1991). In Therapy for Genetic Diseases, T. Friedman, ed., Oxford University Press, pp. 105-121.
Futreal (1993). Ph.D. Thesis, University of North Carolina, Chapel Hill.
Futreal, A., et al. (1992a). Hum. Molec. Genet. 1:66.
Futreal, P.A., et al. (1992b). Cancer Res. 52:2624-2627.
Glebov, O.K., et al. (1994). Cancer Res. 54:3703-3709.
Glover, D. (1985). DNA Cloning, I and II (Oxford Press).
Go, R.C.P., et al. (1983). J. Natl. Cancer Inst. 71:455-461.
Goding (1986). Monoclonal Antibodies: Principles and Practice, 2d ed. (Academic Press, N.Y.).
Godowski, et al. (1988). Science 241:812-816.
Goldgar, D.E., et al. (1994). J. Natl. Can. Inst. 86:3:200-209.
Gordon, et al. (1980). Proc. Natl. Acad. Sci. USA 77:7380-7384.
Gorziglia and Kapikian (1992). J. Virol. 66:4407-4412.
Graham and van der Eb (1973). Virology 52:456-467.
Grompe, M., (1993). Nature Genetics 5:111-117.
Grompe, M., et al., (1989). Proc. Natl. Acad. Sci. USA 86:5855-5892.
Guthrie, G. & Fink, G.R. (1991). Guide to Yeast Genetics and Molecular Biology (Academic Press).
Haber, D. A., et al. (1990). Cell 61:1257-69.
Hall, J.M., et al. (1990). Science 250:1684-1689.
Hall, J.M., et al. (1992). Am. J. Hum. Genet. 50:1235-1241.
Harlow & Lane (1988). Antibodies: A Laboratory Manual (Cold Spring Harbor Laboratory, Cold Spring Harbor, N.Y.
Hasty, P., K., et al. (1991). Nature 350:243.
Helseth, et al. (1990). J. Virol. 64:2416-2420.
Hodgson, J. (1991). Bio/Technology 9:19-21.
Huse, et al. (1989). Science 246:1275-1281.
Innis et al. (1990). PCR Protocols: A Guide to Methods and Applications (Academic Press, San Diego, Cal.).
Jablonski, E., et al. (1986). Nuc. Acids Res. 14:6115-6128.
Jacobs, I.J., et al. (1993). Cancer Res. 53:1218-1221.
Jakoby, W.B. and Pastan, I.H. (eds.) (1979). Cell Culture. Methods in Enzymology, volume 58 (Academic Press, Inc., Harcourt Brace Jovanovich (New York)).
Jeffreys, et al. (1985). Nature 314:67-73.
Johnson, et al. (1992). J. Virol. 66:2952-2965.
Kamb, A. et al. (1994). Science 264:436-440.
Kandpal, et al. (1990). Nucl. Acids Res. 18:1789-1795.
Kaneda, et al. (1989). J. Biol. Chem. 264:12126-12129.
Kanehisa (1984). Nucl. Acids Res. 12:203-213.
Kelsell, D.P., et al. (1993). Human Mol. Genet. 2:1823-1828.
Kinszler, K.W., et al. (1991). Science 251:1366-1370.
Knudson, A.G. (1993). Nature Genet. 5:103.
Kohler, G. and Milstein, C. (1975). Nature 256:495-497.
Kozak, M. (1987). Nucleic Acids Res. 15:8125-8148.
Kraemer, F.B. et al. (1993). J. Lipid Res. 34:663-672.
Kubo, T., et al. (1988). FEBS Letts. 241:119.
Landegren, et al. (1988). Science 242:229.
Lim, et al. (1992). Circulation 83:2007-2011.
Lindsay, S., et al. (1987). Nature 327:336-368.
Litt, et al. (1989). Am. J. Hum. Genet. 44:397-401.
Little, M.H., et al. (1992). Proc. Natl. Acad Sci. USA 89:4791.
Little, M.H., et al. (1993). Hum. Mol. Genet. 2:259.
Lovett, et al. (1991). Proc. Natl. Acad. Sci. USA 88:9628-9632.
Lynch, H.T., et al. (1990). Gynecol. Oncol. 36:48-55.
Madzak, et al. (1992). J. Gen. Virol. 73:1533-1536.
Malkin, D., et al. (1990). Science 250:1233-1238.
Maniatis. T., et al. (1982). Molecular Cloning: A Laboratory Manual (Cold Spring Harbor Laboratory, Cold Spring Harbor, N.Y.).
Mann and Baltimore (1985). J. Virol. 54:401-407.
Margaritte, et al. (1992). Am. J. Hum. Genet. 50:1231-1234.
Margolskee (1992). Curr. Top. Microbiol. Immunol. 158:67-90.
Martin, R., et al. (1990). BioTechniques 9:762-768.
Matteucci, M.D. and Caruthers, M.H. (1981). J. Am. Chem. Soc. 103:3185.
Matthews & Kricka (1988). Anal. Biochem. 169:1.
Merrifield (1963). J. Am. Chem. Soc. 85:2149-2156.
Mettlin. C.. et al. (1990). American Journal of Epidemiology 131:973-983.
Metzger, et al. (1988). Nature 334:31-36.
Miki et al. (1994). Science 266:66.
Miller (1992). Curr. Top. Microbiol. Immunol. 158:1-24.
Miller, et al. (1985). Mol. Cell. Biol. 5:431-437.
Miller, et al. (1988). J. Virol. 62:4337-4345.
Mittlin (1989). Clinical Chem. 35:1819.
Modrich, P. (1991). Ann. Rev. Genet. 25:229-253.
Mombaerts, P., et al. (1992). Cell 68:869.
Monaco, et al. (1986). Nature 323:646.
Moss (1992). Curr. Top. Microbiol. Immunol. 158:25-38.
Muzyczka (1992). Curr. Top. Microbiol. Immunol. 158:97-123.
Nabel (1992). Hum. Gene Ther. 3:399-410.
Nabel, et al. (1990). Science 249:1285-1288.
Nakamura, et al. (1987). Science 235:1616-1622.
Narod, S.A., et al. (1991). The Lancet 338:82-83.
Newman, B., et al. (1988). Proc. Natl. Acad. Sci. USA 85:3044-3048.
Newton, C.R., Graham, A., Heptinstall, L.E., Powell, S.J., Summers, C., Kalsheker, N., Smith, J.C., and Markham, A.F. (1989). Nucl. Acids Res. 17:2503-2516.
Nguyen, Q., et al. (1992). BioTechniques 13:116-123.
Novack, et al. (1986). Proc. Natl. Acad. Sci. USA 83:586.
Oh, J. (1985). Analysis of Human Genetic Linkage, Johns Hopkins University Press, Baltimore, Md, pp. 1-216.
Ohi, et al. (1990). Gene 89:279-282.
Oliphant, A., et al. (1991). Nucleic Acid Res. 19:4794.
Oliphant, A., et al. (1991). Nucleic Acid Res. 19:4795.
Orita, et al. (1989). Proc. Natl. Acad. Sci. USA 86:2776-2770.
Page, et al. (1990). J. Virol. 64:5370-5276.
Pellicer, et al. (1980). Science 209:1414-1422.
Petropoulos, et al. (1992). J. Virol. 66:3391-3397.
Philpott, K.L., et al. (1992). Science 256:1448.
Pierce, et al. (1992). Proc. Natl. Acad. Sci. USA 89:2056-2060.
Quantin, et al. (1992). Proc. Natl. Acad. Sci. USA 89:2581-2584.
Rano & Kidd (1989). Nucl. Acids Res. 17:8392.
Rigby, P.W.J., et al. (1977). J. Mol. Biol. 113:237-251.
Rosenfeld, et al. (1992). Cell 68:143-155.
Sambrook, J., et al. (1989). Molecular Cloning: A Laboratory Manual, 2nd Ed. (Cold Spring Harbor Laboratory, Cold Spring Harbor, N.Y.).
Sato, T., et al. (1990). Cancer Res. 50:7184-7189.
Scharf(1986). Science 233:1076.
Scopes, R. (1982). Protein Purification: Principles and Practice, (Springer-Verlag, N.Y.).
Shaulian, E., et al. (1992). Mol. Cell Biol. 12:5581-92.
Sheffield, V.C., et al. (1989). Proc. Natl. Acad. Sci. USA 86:232-236.
Sheffield, V.C., et al. (1991). Am. J. Hum. Genet. 49:699-706.
Shenk, et al. (1975). Proc. Natl. Acad. Sci. USA 72:989.
Shimada, et al. (1991). J. Clin. Invest. 88:1043-1047.
Shinkai, Y., et al. (1992). Cell 68:855.
Shizuya, H., et al. (1992). Proc. Natl. Acad. Sci. USA 89:8794-8797.
Simard, J., et al. (1993). Human Mol. Genet. 2:1193-1199.
Skolnick, M.H. and Wallace, B.R. (1988). Genomics 2:273-279.
Skolnick, M.H., et al. (1990). Science 250:1715-1720.
Smith, S.A., et al. (1992). Nature Genetics 2:128-131.
Smith, T.F. and Waterman, M.S. (1981). J. Mol. Biol. 147:195-197.
Snouwaert, J.N., et al. (1992). Science 257:1083.
Sorge, et al. (1984). Mol. Cell. Biol. 4:1730-1737.
Srivastava, S., et al. (1993). Cancer Res. 53:4452-5.
Stemberg (1990). Proc. Natl. Acad. Sci. USA 87:103-107.
Sternberg, et al. (1990). The New Biologist 2:151-162.
Stewart, et al. (1992). Hum. Gene Ther. 3:267-275.
Stratford-Perricaudet, et al. (1990). Hum. Gene Ther. 1:241-256.
Swift, M., et al. (1991). N. Engl. J. Med. 325:1831-1836.
Swift, M., et al. (1976). Cancer Res. 36:209-215.
Su, L. K., et al. (1993). Cancer Res. 53:2728-31.
Thomas, A. and Skolnick, M.H. (1994). IMA Journal of Mathematics Applied in Medicine and Biology (in press).
Tonolio, D., *et al*. (1990). Cold Spring Harbor Conference.
Valancius, V. & Smithies, O. (1991). Mol. Cell Biol. 11:1402.
van Dilla, et al. (1986). Biotechnology 4:537-552.
Wagner, et al. (1990). Proc. Natl. Acad. Sci. USA 87:3410-3414.
Wagner, et al. (1991). Proc. Natl. Acad. Sci. USA 88:4255-4259.
Wang and Huang (1989). Biochemistry 28:9508-9514.
Wartell, R.M., et al. (1990). Nucl. Acids Res. 18:2699-2705.
Weber, J.L. (1990). Genomics 7:524-530.
Weber and May (1989). Am. J. Hum. Genet. 44:388-396.
Weber, J.L., et al. (1990). Nucleic Acid Res. 18:4640.
Wells, J.A. (1991). Methods in Enzymol. 202:390-411.
Wetmur & Davidson (1968). J. Mol. Biol. 31:349-370.
White, M.B., et al., (1992). Genomics 12:301-306.
White and Lalouel (1988). Ann. Rev. Genet. 22:259-279.
Wilkinson, et al. (1992). Nucleic Acids Res. 20:2233-2239.
Willams and Anderson (1984). Genet. Epidemiol. 1:7-20.
Wolff, et al. (1990). Science 247:1465-1468.
Wolff, et al. (1991). BioTechniques 11:474-485.
Wooster, R., et al. (1994). Science 265:2088.
Wu, et al. (1989a). Genomics 4:560-569.
Wu, et al. (1989b). J. Biol. Chem. 264:16985-16987.
Wu, et al. (1991). J. Biol. Chem. 266:14338-14342.
Zenke, et al. (1990). Proc. Natl. Acad. Sci. USA 87:3655-3659.
List of Patents and Patent Applications:
U.S. Patent No. 3,817,837
U.S. Patent No. 3,850,752
U.S. Patent No. 3,939,350
U.S. Patent No. 3,996,345
U.S. Patent No. 4,275,149
U.S. Patent No. 4,277,437
U.S. Patent No. 4,366,241
U.S. Patent No. 4,376,110
U.S. Patent No. 4,486,530
U.S. Patent No. 4,683,195
U.S. Patent No. 4,683,202
U.S. Patent No. 4,816,567
U.S. Patent No. 4,868,105
U.S. Patent No. 5,252,479
EPO Publication No. 225,807
European Patent Application Publication No. 0332435
Geysen, H., PCT published application WO 84/03564, published 13 September 1984
Hitzeman *et al.,* EP 73,675A
PCT published application WO 93/07282

## Claims

1. A method for diagnosing a predisposition for breast and ovarian cancer in a human subject which comprises determining whether there is germline alteration 185delAG→ter39 in the BRCA1 gene in a tissue sample of said subject, said alteration indicating a predisposition to said cancer.

2. A method for diagnosing a breast or ovarian lesion of a human subject for neoplasia associated with the BRCA1 gene locus which comprises determining whether there is mutation 185delAG→ter39 in the BRCA1 gene in a sample from said lesion.

3. A method as claimed in claim 1 or 2, which comprises analyzing mRNA of said sample to determine whether an expression product is present indicative of expression of a mutant BRCA1 allele, wherein said mRNA from said sample is contacted with an oligonucleotide BRCA1 gene probe, being allele-specific for said alteration/mutation, under conditions suitable for hybridization of said probe to an RNA corresponding to said BRCA1 gene and hybridization of said probe is determined.

4. A method as claimed in claim 1 or claim 2 wherein an oligonucleotide BRCA1 gene probe, being allele-specific for said alteration/mutation, is contacted with genomic DNA isolated from said sample under conditions suitable for hybridization of said probe to said gene and hybridization of said probe is determined.

5. A method as claimed in claim 1 or claim 2 wherein oligonucleotide primers are employed which are specific for the mutant BRCA1 allele as defined in claim 1 to determine whether said allele is present in said sample by nucleic acid amplification.

6. A method as claimed in claim 1 or claim 2 wherein all or part of the BRCA1 gene in said sample is amplified.

7. A nucleic acid probe having 15 to 30 nucleotides of SEQ ID NO:1 and containing the mutation 185delAG→ter39.

8. A replicative cloning vector which comprises an isolated nucleic acid according to claim 7 and a replicon operative in a host cell for said vector.

9. Host cells in vitro transformed with a vector as claimed in claim 8.

## Patentansprüche

1. Verfahren zur Diagnose einer Prädisposition für Brust- und Eierstockkrebs bei einem menschlichen Individuum, welches das Bestimmen umfasst, ob es eine 185delAG→ter39-Keimbahn-Veränderung im BRCA1-Gen in einer Gewebeprobe des Individuums gibt, wobei die Veränderung eine Prädisposition für den Krebs anzeigt.

2. Verfahren zur Diagnose einer Brust- oder Eierstockläsion bei einem menschlichen Individuum auf Neoplasie, assoziiert mit dem BRCA1-Gen-Locus, welches das Bestimmen umfasst, ob es eine 185delAG→ter39-Mutation im BRCA1-Gen in einer Probe der Läsion gibt.

3. Verfahren wie in Anspruch 1 oder 2 beansprucht, welches das Analysieren von mRNA in der Probe umfasst, um zu bestimmen, ob ein Expressionsprodukt vorliegt, das die Expression einer BRCA1-Allel-Mutante anzeigt, wobei die mRNA von der Probe unter Bedingungen, die zur Hybridisierung der Sonde an eine dem BRCA1-Gen entsprechende RNA geeignet sind, mit einer BRCA1-Oligonucleotid-Gensonde in Kontakt gebracht wird, die für die Veränderung/Mutation allelspezifisch ist, und wobei die Hybridisierung der Sonde bestimmt wird.

4. Verfahren wie in Anspruch 1 oder 2 beansprucht, wobei eine BRCA1-Oligonucleotid-Gensonde, die für die Veränderung/Mutation allelspezifisch ist, mit aus der Probe isolierter genomischer DNA in Kontakt gebracht wird, unter Bedingungen, die für die Hybridisierung der Sonde an das Gen geeignet sind, und wobei die Hybridisierung der Sonde bestimmt wird.

5. Verfahren wie in Anspruch 1 oder 2 beansprucht, wobei Oligonucleotidprimer eingesetzt werden, welche spezifisch für die BRCA1-Allel-Mutante gemäß der Definition in Anspruch 1 sind, um durch Nucleinsäureamplifikation zu bestimmen, ob das Allel in der Probe vorliegt.

6. Verfahren wie in Anspruch 1 oder 2 beansprucht, wobei das ganze oder ein Teil des BRCA1-Gens in der Probe amplifiziert wird.

7. Nucleinsäuresonde, die 15 bis 30 Nucleotide von SEQ ID NO:1 aufweist und die Mutation 185delAG→ter39 enthält.

8. Replikativer Clonierungsvektor, welcher eine isolierte Nucleinsäure gemäß Anspruch 7 und ein Replikon umfasst, das in einer Wirtszelle für den Vektor funktionsfähig ist.

9. Wirtszellen, die in vitro mit einem Vektor wie in Anspruch 8 beansprucht transformiert wurden.

## Revendications

1. Méthode pour le diagnostic d'une prédisposition à un cancer du sein ou de l'ovaire chez un sujet humain qui comprend l'étape consistant à déterminer s'il y a une altération 185delAG→ter39 de la lignée germinale dans le gène BRCA1 dans un échantillon dudit sujet, ladite altération étant indicative d'une prédisposition audit cancer.

2. Méthode pour le diagnostic d'une lésion du sein ou de l'ovaire d'un sujet humain pour une néoplasie associée au locus du gène BRCA1 qui comprend l'étape consistant à déterminer s'il y a une mutation 185delAG→ter39 dans le gène BRCA1 dans un échantillon provenant de ladite lésion.

3. Méthode telle que revendiquée à la revendication 1 ou 2, qui comprend l'analyse de l'ARNm dudit échantillon pour déterminer si un produit d'expression est présent, indicatif de l'expression d'un allèle mutant de BRCA1, dans laquelle ledit ARNm provenant dudit échantillon est mis en contact avec une sonde oligonucléotidique du gène BRCA1, qui est allèle spécifique pour ladite altération/mutation, dans des conditions appropriées pour l'hybridation de ladite sonde à un ARN correspondant audit gène BRCA1 et l'hybridation de ladite sonde est déterminée.

4. Méthode telle que revendiquée à la revendication 1 ou la revendication 2 dans laquelle une sonde oligonucléotidique du gène BRCA1, qui est allèle spécifique pour ladite altération/mutation, est mise en contact avec l'ADN génomique isolé à partir dudit échantillon dans des conditions appropriées pour l'hybridation de ladite sonde audit gène et l'hybridation de ladite sonde est déterminée.

5. Méthode telle que revendiquée à la revendication 1 ou la revendication 2 dans laquelle des amorces oligonucléotidiques sont utilisées qui sont spécifiques de l'allèle mutant de BRCA1 tel que défini à la revendication 1 pour déterminer si ledit allèle est présent dans ledit échantillon par amplification d'acide nucléique.

6. Méthode telle que revendiquée à la revendication 1 ou la revendication 2 dans laquelle tout ou une partie du gène BRCA1 dans ledit échantillon est amplifié.

7. Sonde d'acide nucléique ayant 15 à 30 nucléotides de SEQ ID NO : 1 et contenant la mutation 185delAG→ter39.

8. Vecteur de clonage réplicatif qui comprend un acide nucléique isolé selon la revendication 7 et un réplicon fonctionnel dans une cellule hôte pour ledit vecteur.

9. Cellules hôtes transformées in vitro avec un vecteur tel que revendiqué à la revendication 8.


### SEQUENCE LISTING

(1) GENERAL INFORMATION:
   (i) APPLICANT:
      (A) NAME: MYRIAD GENETICS INC.
      (B) STREET: 300 WAKARA WAY
      (C) CITY: SALT LAKE CITY
      (D) STATE: UTAH
      (E) COUNTRY: UNITED STATES OF AMERICA
      (F) POSTAL CODE (ZIP): 84108

      (A) NAME: CENTRE DU RECHERCHE DU CHUL
      (B) STREET: 2705 LAURIER BOULEVARD
      (C) CITY: SAINTE-FOY
      (D) STATE: QUEBEC
      (E) COUNTRY: CANADA
      (F) POSTAL CODE (ZIP): G1V 4G2

      (A) NAME: CANCER INSTITUTE
      (B) STREET: 1-31-1, KAMI-IKEBUKURO, TOSHIMO-KU
      (C) CITY: TOKYO
      (E) COUNTRY: JAPAN
      (F) POSTAL CODE (ZIP): 170
   (ii) TITLE OF INVENTION: IN VIVO MUTATIONS AND POLYMORPHISMS IN THE 17q-LINKED BREAST AND OVARIAN CANCER SUSCEPTIBILITY GENE
   (iii) NUMBER OF SEQUENCES: 85
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO:1:
   (i) SEQUENCE CHARACTERISTICS:
      (A) LENGTH: 5914 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Homo sapiens
   (ix) FEATURE:
      (A) NAME/KEY: CDS
      (B) LOCATION: 120..5711
   (xi) SEQUENCE DESCRIPTION: SEQ ID NO:1:
AGCTCGCTGA GACTTCCTGG ACCCCGCACC AGGCTGTGGG GTTTCTCAGA TAACTGGGCC       60
   CCTGCGCTCA GGAGGCCTTC ACCCTCTGCT CTGGGTAAAG TTCATTGGAA CAGAAAGAA       119
   ATG GAT TTA TCT GCT CTT CGC GTT GAA GAA GTA CAA AAT GTC ATT AAT       167
   GCT ATG CAG AAA ATC TTA GAG TGT CCC ATC TGT CTG GAG TTG ATC AAG       215
   GAA CCT GTC TCC ACA AAG TGT GAC CAC ATA TTT TGC AAA TTT TGC ATG       263
   CTG AAA CTT CTC AAC CAG AAG AAA GGG CCT TCA CAG TGT CCT TTA TGT    311
   AAG AAT GAT ATA ACC AAA AGG AGC CTA CAA GAA AGT ACG AGA TTT AGT    359
   CAA CTT GTT GAA GAG CTA TTG AAA ATC ATT TGT GCT TTT CAG CTT GAC    407
   ACA GGT TTG GAG TAT GCA AAC AGC TAT AAT TTT GCA AAA AAG GAA AAT    455
   AAC TCT CCT GAA CAT CTA AAA GAT GAA GTT TCT ATC ATC CAA AGT ATG    503
   GGC TAC AGA AAC CGT GCC AAA AGA CTT CTA CAG AGT GAA CCC GAA AAT    551
   CCT TCC TTG CAG GAA ACC AGT CTC AGT GTC CAA CTC TCT AAC CTT GGA    599
   ACT GTG AGA ACT CTG AGG ACA AAG CAG CGG ATA CAA CCT CAA AAG ACG    647
   TCT GTC TAC ATT GAA TTG GGA TCT GAT TCT TCT GAA GAT ACC GTT AAT    695
   AAG GCA ACT TAT TGC AGT GTG GGA GAT CAA GAA TTG TTA CAA ATC ACC    743
   CCT CAA GGA ACC AGG GAT GAA ATC AGT TTG GAT TCT GCA AAA AAG GCT    791
   GCT TGT GAA TTT TCT GAG ACG GAT GTA ACA AAT ACT GAA CAT CAT CAA    839
   CCC AGT AAT AAT GAT TTG AAC ACC ACT GAG AAG CGT GCA GCT GAG AGG    887
   CAT CCA GAA AAG TAT CAG GGT AGT TCT GTT TCA AAC TTG CAT GTG GAG    935
   CCA TGT GGC ACA AAT ACT CAT GCC AGC TCA TTA CAG CAT GAG AAC AGC      983
   AGT TTA TTA CTC ACT AAA GAC AGA ATG AAT GTA GAA AAG GCT GAA TTC     1031
   TGT AAT AAA AGC AAA CAG CCT GGC TTA GCA AGG AGC CAA CAT AAC AGA     1079
   TGG GCT GGA AGT AAG GAA ACA TGT AAT GAT AGG CGG ACT CCC AGC ACA     1127
   GAA AAA AAG GTA GAT CTG AAT GCT GAT CCC CTG TGT GAG AGA AAA GAA     1175
   TGG AAT AAG CAG AAA CTG CCA TGC TCA GAG AAT CCT AGA GAT ACT GAA     1223
   GAT GTT CCT TGG ATA ACA CTA AAT AGC AGC ATT CAG AAA GTT AAT GAG     1271
   TGG TTT TCC AGA AGT GAT GAA CTG TTA GGT TCT GAT GAC TCA CAT GAT     1319
   GGG GAG TCT GAA TCA AAT GCC AAA GTA GCT GAT GTA TTG GAC GTT CTA     1367
   AAT GAG GTA GAT GAA TAT TCT GGT TCT TCA GAG AAA ATA GAC TTA CTG     1415
   GCC AGT GAT CCT CAT GAG GCT TTA ATA TGT AAA AGT GAA AGA GTT CAC     1463
   TCC AAA TCA GTA GAG AGT AAT ATT GAA GAC AAA ATA TTT GGG AAA ACC     1511
   TAT CGG AAG AAG GCA AGC CTC CCC AAC TTA AGC CAT GTA ACT GAA AAT     1559
   CTA ATT ATA GGA GCA TTT GTT ACT GAG CCA CAG ATA ATA CAA GAG CGT     1607
   CCC CTC ACA AAT AAA TTA AAG CGT AAA AGG AGA CCT ACA TCA GGC CTT      1655
   CAT CCT GAG GAT TTT ATC AAG AAA GCA GAT TTG GCA GTT CAA AAG ACT      1703
   CCT GAA ATG ATA AAT CAG GGA ACT AAC CAA ACG GAG CAG AAT GGT CAA      1751
   GTG ATG AAT ATT ACT AAT AGT GGT CAT GAG AAT AAA ACA AAA GGT GAT      1799
   TCT ATT CAG AAT GAG AAA AAT CCT AAC CCA ATA GAA TCA CTC GAA AAA      1847
   GAA TCT GCT TTC AAA ACG AAA GCT GAA CCT ATA AGC AGC AGT ATA AGC      1895
   AAT ATG GAA CTC GAA TTA AAT ATC CAC AAT TCA AAA GCA CCT AAA AAG      1943
   AAT AGG CTG AGG AGG AAG TCT TCT ACC AGG CAT ATT CAT GCG CTT GAA      1991
   CTA GTA GTC AGT AGA AAT CTA AGC CCA CCT AAT TGT ACT GAA TTG CAA      2039
   ATT GAT AGT TGT TCT AGC AGT GAA GAG ATA AAG AAA AAA AAG TAC AAC      2087
   CAA ATG CCA GTC AGG CAC AGC AGA AAC CTA CAA CTC ATG GAA GGT AAA      2135
   GAA CCT GCA ACT GGA GCC AAG AAG AGT AAC AAG CCA AAT GAA CAG ACA      2183
   AGT AAA AGA CAT GAC AGC GAT ACT TTC CCA GAG CTG AAG TTA ACA AAT      2231
   GCA CCT GGT TCT TTT ACT AAG TGT TCA AAT ACC AGT GAA CTT AAA GAA      2279
   TTT GTC AAT CCT AGC CTT CCA AGA GAA GAA AAA GAA GAG AAA CTA GAA        2327
   ACA GTT AAA GTG TCT AAT AAT GCT GAA GAC CCC AAA GAT CTC ATG TTA        2375
   AGT GGA GAA AGG GTT TTG CAA ACT GAA AGA TCT GTA GAG AGT AGC AGT        2423
   ATT TCA TTG GTA CCT GGT ACT GAT TAT GGC ACT CAG GAA AGT ATC TCG        2471
   TTA CTG GAA GTT AGC ACT CTA GGG AAG GCA AAA ACA GAA CCA AAT AAA        2519
   TGT GTG AGT CAG TGT GCA GCA TTT GAA AAC CCC AAG GGA CTA ATT CAT        2567
   GGT TGT TCC AAA GAT AAT AGA AAT GAC ACA GAA GGC TTT AAG TAT CCA        2615
   TTG GGA CAT GAA GTT AAC CAC AGT CGG GAA ACA AGC ATA GAA ATG GAA        2663
   GAA AGT GAA CTT GAT GCT CAG TAT TTG CAG AAT ACA TTC AAG GTT TCA        2711
   AAG CGC CAG TCA TTT GCT CCG TTT TCA AAT CCA GGA AAT GCA GAA GAG        2759
   GAA TGT GCA ACA TTC TCT GCC CAC TCT GGG TCC TTA AAG AAA CAA AGT        2807
   CCA AAA GTC ACT TTT GAA TGT GAA CAA AAG GAA GAA AAT CAA GGA AAG        2855
   AAT GAG TCT AAT ATC AAG CCT GTA CAG ACA GTT AAT ATC ACT GCA GGC        2903
   TTT CCT GTG GTT GGT CAG AAA GAT AAG CCA GTT GAT AAT GCC AAA TGT        2951
   AGT ATC AAA GGA GGC TCT AGG TTT TGT CTA TCA TCT CAG TTC AGA GGC 2999
   AAC GAA ACT GGA CTC ATT ACT CCA AAT AAA CAT GGA CTT TTA CAA AAC 3047
   CCA TAT CGT ATA CCA CCA CTT TTT CCC ATC AAG TCA TTT GTT AAA ACT 3095
   AAA TGT AAG AAA AAT CTG CTA GAG GAA AAC TTT GAG GAA CAT TCA ATG 3143
   TCA CCT GAA AGA GAA ATG GGA AAT GAG AAC ATT CCA AGT ACA GTG AGC 3191
   ACA ATT AGC CGT AAT AAC ATT AGA GAA AAT GTT TTT AAA GAA GCC AGC 3239
   TCA AGC AAT ATT AAT GAA GTA GGT TCC AGT ACT AAT GAA GTG GGC TCC 3287
   AGT ATT AAT GAA ATA GGT TCC AGT GAT GAA AAC ATT CAA GCA GAA CTA 3335
   GGT AGA AAC AGA GGG CCA AAA TTG AAT GCT ATG CTT AGA TTA GGG GTT 3383
   TTG CAA CCT GAG GTC TAT AAA CAA AGT CTT CCT GGA AGT AAT TGT AAG 3431
   CAT CCT GAA ATA AAA AAG CAA GAA TAT GAA GAA GTA GTT CAG ACT GTT 3479
   AAT ACA GAT TTC TCT CCA TAT CTG ATT TCA GAT AAC TTA GAA CAG CCT 3527
   ATG GGA AGT AGT CAT GCA TCT CAG GTT TGT TCT GAG ACA CCT GAT GAC 3575
   CTG TTA GAT GAT GGT GAA ATA AAG GAA GAT ACT AGT TTT GCT GAA AAT 3623
   GAC ATT AAG GAA AGT TCT GCT GTT TTT AGC AAA AGC GTC CAG AAA GGA 3671
   GAG CTT AGC AGG AGT CCT AGC CCT TTC ACC CAT ACA CAT TTG GCT CAG 3719
   GGT TAC CGA AGA GGG GCC AAG AAA TTA GAG TCC TCA GAA GAG AAC TTA 3767
   TCT AGT GAG GAT GAA GAG CTT CCC TGC TTC CAA CAC TTG TTA TTT GGT 3815
   AAA GTA AAC AAT ATA CCT TCT CAG TCT ACT AGG CAT AGC ACC GTT GCT 3863
   ACC GAG TGT CTG TCT AAG AAC ACA GAG GAG AAT TTA TTA TCA TTG AAG 3911
   AAT AGC TTA AAT GAC TGC AGT AAC CAG GTA ATA TTG GCA AAG GCA TCT 3959
   CAG GAA CAT CAC CTT AGT GAG GAA ACA AAA TGT TCT GCT AGC TTG TTT 4007
   TCT TCA CAG TGC AGT GAA TTG GAA GAC TTG ACT GCA AAT ACA AAC ACC 4055
   CAG GAT CCT TTC TTG ATT GGT TCT TCC AAA CAA ATG AGG CAT CAG TCT 4103
   GAA AGC CAG GGA GTT GGT CTG AGT GAC AAG GAA TTG GTT TCA GAT GAT 4151
   GAA GAA AGA GGA ACG GGC TTG GAA GAA AAT AAT CAA GAA GAG CAA AGC 4199
   ATG GAT TCA AAC TTA GGT GAA GCA GCA TCT GGG TGT GAG AGT GAA ACA 4247
   AGC GTC TCT GAA GAC TGC TCA GGG CTA TCC TCT CAG AGT GAC ATT TTA 4295
   ACC ACT CAG CAG AGG GAT ACC ATG CAA CAT AAC CTG ATA AAG CTC CAG      4343
   CAG GAA ATG GCT GAA CTA GAA GCT GTG TTA GAA CAG CAT GGG AGC CAG      4391
   CCT TCT AAC AGC TAC CCT TCC ATC ATA AGT GAC TCT TCT GCC CTT GAG      4439
   GAC CTG CGA AAT CCA GAA CAA AGC ACA TCA GAA AAA GCA GTA TTA ACT      4487
   TCA CAG AAA AGT AGT GAA TAC CCT ATA AGC CAG AAT CCA GAA GGC CTT      4535
   TCT GCT GAC AAG TTT GAG GTG TCT GCA GAT AGT TCT ACC AGT AAA AAT      4583
   AAA GAA CCA GGA GTG GAA AGG TCA TCC CCT TCT AAA TGC CCA TCA TTA      4631
   GAT GAT AGG TGG TAC ATG CAC AGT TGC TCT GGG AGT CTT CAG AAT AGA      4679
   AAC TAC CCA TCT CAA GAG GAG CTC ATT AAG GTT GTT GAT GTG GAG GAG      4727
   CAA CAG CTG GAA GAG TCT GGG CCA CAC GAT TTG ACG GAA ACA TCT TAC      4775
   TTG CCA AGG CAA GAT CTA GAG GGA ACC CCT TAC CTG GAA TCT GGA ATC      4823
   AGC CTC TTC TCT GAT GAC CCT GAA TCT GAT CCT TCT GAA GAC AGA GCC      4871
   CCA GAG TCA GCT CGT GTT GGC AAC ATA CCA TCT TCA ACC TCT GCA TTG      4919
   AAA GTT CCC CAA TTG AAA GTT GCA GAA TCT GCC CAG AGT CCA GCT GCT      4967
   GCT CAT ACT ACT GAT ACT GCT GGG TAT AAT GCA ATG GAA GAA AGT GTG      5015
   AGC AGG GAG AAG CCA GAA TTG ACA GCT TCA ACA GAA AGG GTC AAC AAA      5063
   AGA ATG TCC ATG GTG GTG TCT GGC CTG ACC CCA GAA GAA TTT ATG CTC      5111
   GTG TAC AAG TTT GCC AGA AAA CAC CAC ATC ACT TTA ACT AAT CTA ATT      5159
   ACT GAA GAG ACT ACT CAT GTT GTT ATG AAA ACA GAT GCT GAG TTT GTG      5207
   TGT GAA CGG ACA CTG AAA TAT TTT CTA GGA ATT GCG GGA GGA AAA TGG      5255
   GTA GTT AGC TAT TTC TGG GTG ACC CAG TCT ATT AAA GAA AGA AAA ATG      5303
   CTG AAT GAG CAT GAT TTT GAA GTC AGA GGA GAT GTG GTC AAT GGA AGA      5351
   AAC CAC CAA GGT CCA AAG CGA GCA AGA GAA TCC CAG GAC AGA AAG ATC      5399
   TTC AGG GGG CTA GAA ATC TGT TGC TAT GGG CCC TTC ACC AAC ATG CCC      5447
   ACA GAT CAA CTG GAA TGG ATG GTA CAG CTG TGT GGT GCT TCT GTG GTG      5495
   AAG GAG CTT TCA TCA TTC ACC CTT GGC ACA GGT GTC CAC CCA ATT GTG      5543
   GTT GTG CAG CCA GAT GCC TGG ACA GAG GAC AAT GGC TTC CAT GCA ATT      5591
   GGG CAG ATG TGT GAG GCA CCT GTG GTG ACC CGA GAG TGG GTG TTG GAC      5639
   AGT GTA GCA CTC TAC CAG TGC CAG GAG CTG GAC ACC TAC CTG ATA CCC      5687
   CAG ATC CCC CAC AGC CAC TAC TGA CTGCAGCCAG CCACAGGTAC AGAGCCACAG     5741
   GACCCCAAGA ATGAGCTTAC AAAGTGGCCT TTCCAGGCCC TGGGAGCTCC TCTCACTCTT    5801
   CAGTCCTTCT ACTGTCCTGG CTACTAAATA TTTTATGTAC ATCAGCCTGA AAAGGACTTC    5861
   TGGCTATGCA AGGGTCCCTT AAAGATTTTC TGCTTGAAGT CTCCCTTGGA AAT           5914
(2) INFORMATION FOR SEQ ID NO:2:
   (i) SEQUENCE CHARACTERISTICS:
      (A) LENGTH: 1864 amino acids
      (B) TYPE: amino acid
      (D) TOPOLOGY: linear
   (ii) MOLECULE TYPE: protein
   (xi) SEQUENCE DESCRIPTION: SEQ ID NO:2:
(2) INFORMATION FOR SEQ ID NO:3:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (vii) IMMEDIATE SOURCE:
      (B) CLONE: s754 A
   (xi) SEQUENCE DESCRIPTION: SEQ ID NO:3:
      CTAGCCTGGG CAACAAACGA 20
(2) INFORMATION FOR SEQ ID NO:4:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (vii) IMMEDIATE SOURCE:
      (B) CLONE: s754 B
   (xi) SEQUENCE DESCRIPTION: SEQ ID NO:4:
      GCAGGAAGCA GGAATGGAAC 20
(2) INFORMATION FOR SEQ ID NO:5:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (vii) IMMEDIATE SOURCE:
      (B) CLONE: s975 A
   (xi) SEQUENCE DESCRIPTION: SEQ ID NO:5:
      TAGGAGATGG ATTATTGGTG 20
(2) INFORMATION FOR SEQ ID NO:6:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (vii) IMMEDIATE SOURCE:
      (B) CLONE: s975 B
   (xi) SEQUENCE DESCRIPTION: SEQ ID NO:6:
      AGGCAACTTT GCAATGAGTG 20
(2) INFORMATION FOR SEQ ID NO:7:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (vii) IMMEDIATE SOURCE:
      (B) CLONE: tdj1474 A
   (xi) SEQUENCE DESCRIPTION: SEQ ID NO:7:
      CAGAGTGAGA CCTTGTCTCA AA 22
(2) INFORMATION FOR SEQ ID NO:8:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (vii) IMMEDIATE SOURCE:
      (B) CLONE: tdj1474 B
   (xi) SEQUENCE DESCRIPTION: SEQ ID NO:8:
      TTCTGCAAAC ACCTTAAACT CAG 23
(2) INFORMATION FOR SEQ ID NO:9:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (vii) IMMEDIATE SOURCE:
      (B) CLONE: tdj1239 A
   (xi) SEQUENCE DESCRIPTION: SEQ ID NO:9:
      AACCTGGAAG GCAGAGGTTG 20
(2) INFORMATION FOR SEQ ID NO:10:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (vii) IMMEDIATE SOURCE:
      (B) CLONE: tdj1239 B
   (xi) SEQUENCE DESCRIPTION: SEQ ID NO:10:
      TCTGTACCTG CTAAGCAGTG G 21
(2) INFORMATION FOR SEQ ID NO:11:
   (i) SEQUENCE CHARACTERISTICS:
      (A) LENGTH: 111 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (vi) ORIGINAL SOURCE:
      (A) ORGANISM: Homo sapiens
   (ix) FEATURE:
      (A) NAME/KEY: CDS
      (B) LOCATION: 2..111
   (xi) SEQUENCE DESCRIPTION: SEQ ID NO:11:
   G GKC TTA CTC TGT TGT CCC AGC TGG AGT ACA GWG TGC GAT CAT GAG          46
     Xaa Leu Leu Cys Cys Pro Ser Trp Ser Thr Xaa Cys Asp His Glu
     1865                1870                1875
   GCT TAC TGT TGC TTG ACT CCT AGG CTC AAG CGA TCC TAT CAC CTC AGT        94
   Ala Tyr Cys Cys Leu Thr Pro Arg Leu Lys Arg Ser Tyr His Leu Ser
   1880                1885                1890                1895
   CTC CAA GTA GCT GGA  CT                                               111
   Leu Gln Val Ala Gly
                   1900
(2) INFORMATION FOR SEQ ID NO:12:
   (i) SEQUENCE CHARACTERISTICS:
      (A) LENGTH: 36 amino acids
      (B) TYPE: amino acid
      (D) TOPOLOGY: linear
   (ii) MOLECULE TYPE: protein
   (xi) SEQUENCE DESCRIPTION: SEQ ID NO:12:
   Xaa Leu Leu Cys Cys Pro Ser Trp Ser Thr Xaa Cys Asp His Glu Ala
     1               5                  10                  15
   Tyr Cys Cys Leu Thr Pro Arg Leu Lys Arg Ser Tyr His Leu Ser Leu
               20                  25                  30
   Gln Val Ala Gly
           35
(2) INFORMATION FOR SEQ ID NO:13:
   (i) SEQUENCE CHARACTERISTICS:
      (A) LENGTH: 1534 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:13:
   GAGGCTAGAG GGCAGGCACT TTATGGCAAA CTCAGGTAGA ATTCTTCCTC TTCCGTCTCT 60
   TTCCTTTTAC GTCATCGGGG AGACTGGGTG GCAATCGCAG CCCGAGAGAC GCATGGCTCT 120
   TTCTGCCCTC CATCCTCTGA TGTACCTTGA TTTCGTATTC TGAGAGGCTG CTGCTTAGCG 180
   GTAGCCCCTT GGTTTCCGTG GCAACGGAAA AGCGCGGGAA TTACAGATAA ATTAAAACTG 240
   CGACTGCGCG GCGTGAGCTC GCTGAGACTT CCTGGACCCC GCACCAGGCT GTGGGGTTTC 300
   TCAGATAACT GGGCCCCTGC GCTCAGGAGG CCTTCACCCT CTGCTCTGGG TAAAGGTAGT 360
   AGAGTCCCGG GAAAGGGACA GGGGGCCCAA GTGATGCTCT GGGGTACTGG CGTGGGAGAG 420
   TGGATTTCCG AAGCTGACAG ATGGGTATTC TTTGACGGGG GGTAGGGGCG GAACCTGAGA 480
   GGCGTAAGGC GTTGTGAACC CTGGGGAGGG GGGCAGTTTG TAGGTCGCGA GGGAAGCGCT 540
   GAGGATCAGG AAGGGGGCAC TGAGTGTCCG TGGGGGAATC CTCGTGATAG GAACTGGAAT 600
   ATGCCTTGAG GGGGACACTA TGTCTTTAAA AACGTCGGCT GGTCATGAGG TCAGGAGTTC 660
   CAGACCAGCC TGACCAACGT GGTGAAACTC CGTCTCTACT AAAAATACNA AAATTAGCCG 720
   GGCGTGGTGC CGCTCCAGCT ACTCAGGAGG CTGAGGCAGG AGAATCGCTA GAACCCGGGA 780
   GGCGGAGGTT GCAGTGAGCC GAGATCGCGC CATTGCACTC CAGCCTGGGC GACAGAGCGA 840
   GACTGTCTCA AAACAAAACA AAACAAAACA AAACAAAAAA CACCGGCTGG TATGTATGAG 900
   AGGATGGGAC CTTGTGGAAG AAGAGGTGCC AGGAATATGT CTGGGAAGGG GAGGAGACAG 960
   GATTTTGTGG GAGGGAGAAC TTAAGAACTG GATCCATTTG CGCCATTGAG AAAGCGCAAG 1020
   AGGGAAGTAG AGGAGCGTCA GTAGTAACAG ATGCTGCCGG CAGGGATGTG CTTGAGGAGG 1080
   ATCCAGAGAT GAGAGCAGGT CACTGGGAAA GGTTAGGGGC GGGGAGGCCT TGATTGGTGT 1140
   TGGTTTGGTC GTTGTTGATT TTGGTTTTAT GCAAGAAAAA GAAAACAACC AGAAACATTG 1200
   GAGAAAGCTA AGGCTACCAC CACCTACCCG GTCAGTCACT CCTCTGTAGC TTTCTCTTTC 1260
   TTGGAGAAAG GAAAAGACCC AAGGGGTTGG CAGCGATATG TGAAAAAATT CAGAATTTAT 1320
   GTTGTCTAAT TACAAAAAGC AACTTCTAGA ATCTTTAAAA ATAAAGGACG TTGTCATTAG 1380
   TTCTTCTGGT TTGTATTATT CTAAAACCTT CCAAATCTTC AAATTTACTT TATTTTAAAA 1440
   TGATAAAATG AAGTTGTCAT TTTATAAACC TTTTAAAAAG ATATATATAT ATGTTTTTCT 1500
   AATGTGTTAA AGTTCATTGG AACAGAAAGA AATG 1534
(2) INFORMATION FOR SEQ ID NO:14:
   (i) SEQUENCE CHARACTERISTICS
      (A) LENGTH: 1924 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:14:
   GAGGCTAGAG GGCAGGCACT TTATGGCAAA CTCAGGTAGA ATTCTTCCTC TTCCGTCTCT       60
   TTCCTTTTAC GTCATCGGGG AGACTGGGTG GCAATCGCAG CCCGAGAGAC GCATGGCTCT      120
   TTCTGCCCTC CATCCTCTGA TGTACCTTGA TTTCGTATTC TGAGAGGCTG CTGCTTAGCG      180
   GTAGCCCCTT GGTTTCCGTG GCAACGGAAA AGCGCGGGAA TTACAGATAA ATTAAAACTG      240
   CGACTGCGCG GCGTGAGCTC GCTGAGACTT CCTGGACCCC GCACCAGGCT GTGGGGTTTC   300
   TCAGATAACT GGGCCCCTGC GCTCAGGAGG CCTTCACCCT CTGCTCTGGG TAAAGGTAGT   360
   AGAGTCCCGG GAAAGGGACA GGGGGCCCAA GTGATGCTCT GGGGTACTGG CGTGGGAGAG   420
   TGGATTTCCG AAGCTGACAG ATGGGTATTC TTTGACGGGG GGTAGGGGCG GAACCTGAGA   480
   GGCGTAAGGC GTTGTGAACC CTGGGGAGGG GGGCAGTTTG TAGGTCGCGA GGGAAGCGCT   540
   GAGGATCAGG AAGGGGGCAC TGAGTGTCCG TGGGGGAATC CTCGTGATAG GAACTGGAAT   600
   ATGCCTTGAG GGGGACACTA TGTCTTTAAA AACGTCGGCT GGTCATGAGG TCAGGAGTTC   660
   CAGACCAGCC TGACCAACGT GGTGAAACTC CGTCTCTACT AAAAATACNA AAATTAGCCG   720
   GGCGTGGTGC CGCTCCAGCT ACTCAGGAGG CTGAGGCAGG AGAATCGCTA GAACCCGGGA   780
   GGCGGAGGTT GCAGTGAGCC GAGATCGCGC CATTGCACTC CAGCCTGGGC GACAGAGCGA   840
   GACTGTCTCA AAACAAAACA AAACAAAACA AAACAAAAAA CACCGGCTGG TATGTATGAG   900
   AGGATGGGAC CTTGTGGAAG AAGAGGTGCC AGGAATATGT CTGGGAAGGG GAGGAGACAG   960
   GATTTTGTGG GAGGGAGAAC TTAAGAACTG GATCCATTTG CGCCATTGAG AAAGCGCAAG  1020
   AGGGAAGTAG AGGAGCGTCA GTAGTAACAG ATGCTGCCGG CAGGGATGTG CTTGAGGAGG  1080
   ATCCAGAGAT GAGAGCAGGT CACTGGGAAA GGTTAGGGGC GGGGAGGCCT TGATTGGTGT  1140
   TGGTTTGGTC GTTGTTGATT TTGGTTTTAT GCAAGAAAAA GAAAACAACC AGAAACATTG  1200
   GAGAAAGCTA AGGCTACCAC CACCTACCCG GTCAGTCACT CCTCTGTAGC TTTCTCTTTC  1260
   TTGGAGAAAG GAAAAGACCC AAGGGGTTGG CAGCGATATG TGAAAAAATT CAGAATTTAT  1320
   GTTGTCTAAT TACAAAAAGC AACTTCTAGA ATCTTTAAAA ATAAAGGACG TTGTCATTAG  1380
   TTCTTCTGGT TTGTATTATT CTAAAACCTT CCAAATCTTC AAATTTACTT TATTTTAAAA  1440
   TGATAAAATG AAGTTGTCAT TTTATAAACC TTTTAAAAAG ATATATATAT ATGTTTTTCT  1500
   AATGTGTTAA AGTTCATTGG AACAGAAAGA AATGGATTTA TCTGCTCTTC GCGTTGAAGA  1560
   AGTACAAAAT GTCATTAATG CTATGCAGAA AATCTTAGAG TGTCCCATCT GGTAAGTCAG  1620
   CACAAGAGTG TATTAATTTG GGATTCCTAT GATTATCTCC TATGCAAATG AACAGAATTG  1680
   ACCTTACATA CTAGGGAAGA AAAGACATGT CTAGTAAGAT TAGGCTATTG TAATTGCTGA  1740
   TTTTCTTAAC TGAAGAACTT TAAAAATATA GAAAATGATT CCTTGTTCTC CATCCACTCT  1800
   GCCTCTCCCA CTCCTCTCCT TTTCAACACA ATCCTGTGGT CCGGGAAAGA CAGGGCTCTG  1860
   TCTTGATTGG TTCTGCACTG GGCAGGATCT GTTAGATACT GCATTTGCTT TCTCCAGCTC  1920
   TAAA 1924
(2) INFORMATION FOR SEQ ID NO:15:
   (i) SEQUENCE CHARACTERISTICS:
      (A) LENGTH: 631 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:15:
   AAATGCTGAT GATAGTATAG AGTATTGAAG GGATCAATAT AATTCTGTTT TGATATCTGA    60
   AAGCTCACTG AAGGTAAGGA TCGTATTCTC TGCTGTATTC TCAGTTCCTG ACACAGCAGA   120
   CATTTAATAA ATATTGAACG AACTTGAGGC CTTATGTTGA CTCAGTCATA ACAGCTCAAA   180
   GTTGAACTTA TTCACTAAGA ATAGCTTTAT TTTTAAATAA ATTATTGAGC CTCATTTATT   240
   TTCTTTTTCT CCCCCCCCTA CCCTGCTAGT CTGGAGTTGA TCAAGGAACC TGTCTCCACA   300
   AAGTGTGACC ACATATTTTG CAAGTAAGTT TGAATGTGTT ATGTGGCTCC ATTATTAGCT   360
   TTTGTTTTTG TCCTTCATAA CCCAGGAAAC ACCTAACTTT ATAGAAGCTT TACTTTCTTC   420
   AATTAAGTGA GAACGAAAAT CCAACTCCAT TTCATTCTTT CTCAGAGAGT ATATAGTTAT   480
   CAAAAGTTGG TTGTAATCAT AGTTCCTGGT AAAGTTTTGA CATATATTAT CTTTTTTTTT   540
   TTTTGAGACA AGTCTCGCTC TGTCGCCCAG GCTGGAGTGC AGTGGCATGA GGCTTGCTCA   600
   CTGCACCTCC GCCCCCGAGT TCAGCGACTC T                                  631
(2) INFORMATION FOR SEQ ID NO:16:
   (i) SEQUENCE CHARACTERISTICS:
      (A) LENGTH: 481 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:16:
   TGAGATCTAG ACCACATGGT CAAAGAGATA GAATGTGAGC AATAAATGAA CCTTAAATTT     60
   TTCAACAGCT ACTTTTTTTT TTTTTTTTTG AGACAGGGKC TTACTCTGTT GTCCCAGCTG    120
   GAGTACAGWG TGCGATCATG AGGCTTACTG TTGCTTGACT CCTAGGCTCA AGCGATCCTA    180
   TCACCTCAGT CTCCAAGTAG CTGGACTGTA AGTGCACACC ACCATATCCA GCTAAATTTT    240
   GTGTTTTCTG TAGAGACGGG GTTTCGCCAT GTTTCCCAGG CTGGTCTTGA ACTTTGGGCT    300
   TAACCCGTCT GCCCACCTAG GCATCCCAAA GTGCTAGGAT TACAGGTGTG AGTCATCATG    360
   CCTGGCCAGT ATTTTAGTTA GCTCTGTCTT TTCAAGTCAT ATACAAGTTC ATTTTCTTTT    420
   AAGTTTAGTT AACAACCTTA TATCATGTAT TCTTTTCTAG CATAAAGAAA GATTCGAGGC    480
   C                                                                    481
(2) INFORMATION FOR SEQ ID NO:17:
   (i) SEQUENCE CHARACTERISTICS:
      (A) LENGTH: 522 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:17:
   TGTGATCATA ACAGTAAGCC ATATGCATGT AAGTTCAGTT TTCATAGATC ATTGCTTATG     60
   TAGTTTAGGT TTTTGCTTAT GCAGCATCCA AAAACAATTA GGAAACTATT GCTTGTAATT    120
   CACCTGCCAT TACTTTTTAA ATGGCTCTTA AGGGCAGTTG TGAGATTATC TTTTCATGGC    180
   TATTTGCCTT TTGAGTATTC TTTCTACAAA AGGAAGTAAA TTAAATTGTT CTTTCTTTCT 240
   TTATAATTTA TAGATTTTGC ATGCTGAAAC TTCTCAACCA GAAGAAAGGG CCTTCACAGT 300
   GTCCTTTATG TAAGAATGAT ATAACCAAAA GGTATATAAT TTGGTAATGA TGCTAGGTTG 360
   GAAGCAACCA CAGTAGGAAA AAGTAGAAAT TATTTAATAA CATAGCGTTC CTATAAAACC 420
   ATTCATCAGA AAAATTTATA AAAGAGTTTT TAGCACACAG TAAATTATTT CCAAAGTTAT 480
   TTTCCTGAAA GTTTTATGGG CATCTGCCTT ATACAGGTAT TG 522
(2) INFORMATION FOR SEQ ID NO:18:
   (i) SEQUENCE CHARACTERISTICS:
      (A) LENGTH: 465 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:18:
   GGTAGGCTTA AATGAATGAC AAAAAGTTAC TAAATCACTG CCATCACACG GTTTATACAG 60
   ATGTCAATGA TGTATTGATT ATAGAGGTTT TCTACTGTTG CTGCATCTTA TTTTTATTTG 120
   TTTACATGTC TTTTCTTATT TTAGTGTCCT TAAAAGGTTG ATAATCACTT GCTGAGTGTG 180
   TTTCTCAAAC AATTTAATTT CAGGAGCCTA CAAGAAAGTA CGAGATTTAG TCAACTTGTT 240
   GAAGAGCTAT TGAAAATCAT TTGTGCTTTT CAGCTTGACA CAGGTTTGGA GTGTAAGTGT 300
   TGAATATCCC AAGAATGACA CTCAAGTGCT GTCCATGAAA ACTCAGGAAG TTTGCACAAT 360
   TACTTTCTAT GACGTGGTGA TAAGACCTTT TAGTCTAGGT TAATTTTAGT TCTGTATCTG 420
   TAATCTATTT TAAAAAATTA CTCCCACTGG TCTCACACCT TATTT 465
(2) INFORMATION FOR SEQ ID NO:19:
   (i) SEQUENCE CHARACTERISTICS:
      (A) LENGTH: 513 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:19:
   AAAAAATCAC AGGTAACCTT AATGCATTGT CTTAACACAA CAAAGAGCAT ACATAGGGTT    60
   TCTCTTGGTT TCTTTGATTA TAATTCATAC ATTTTTCTCT AACTGCAAAC ATAATGTTTT   120
   CCCTTGTATT TTACAGATGC AAACAGCTAT AATTTTGCAA AAAAGGAAAA TAACTCTCCT   180
   GAACATCTAA AAGATGAAGT TTCTATCATC CAAAGTATGG GCTACAGAAA CCGTGCCAAA   240
   AGACTTCTAC AGAGTGAACC CGAAAATCCT TCCTTGGTAA AACCATTTGT TTTCTTCTTC   300
   TTCTTCTTCT TCTTTTCTTT TTTTTTTCTT TTTTTTTTTG AGATGGAGTC TTGCTCTGTG   360
   GCCCAGGCTA GAAGCAGTCC TCCTGCCTTA GCCNCCTTAG TAGCTGGGAT TACAGGCACG   420
   CGCACCATGC CAGGCTAATT TTTGTATTTT TAGTAGAGAC GGGGTTTCAT CATGTTGGCC   480
   AGGCTGGTCT CGAACTCCTA ACCTCAGGTG ATC                                513
(2) INFORMATION FOR SEQ ID NO:20:
   (i) SEQUENCE CHARACTERISTICS:
      (A) LENGTH: 6769 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:20:
   ATGATGGAGA TCTTAAAAAG TAATCATTCT GGGGCTGGGC GTAGTAGCTT GCACCTGTAA 60
   TCCCAGCACT TCGGGAGGCT GAGGCAGGCA GATAATTTGA GGTCAGGAGT TTGAGACCAG 120
   CCTGGCCAAC ATGGTGAAAC CCATCTCTAC TAAAAATACA AAAATTAGCT GGGTGTGGTG 180
   GCACGTACCT GTAATCCCAG CTACTCGGGA GGCGGAGGCA CAAGAATTGC TTGAACCTAG 240
   GACGCGGAGG TTGCAGCGAG CCAAGATCGC GCCACTGCAC TCCAGCCTGG GCCGTAGAGT 300
   GAGACTCTGT CTCAAAAAAG AAAAAAAAGT AATTGTTCTA GCTGGGCGCA GTGGCTCTTG 360
   CCTGTAATCC CAGCACTTTG GGAGGCCAAG GCGGGTGGAT CTCGAGTCCT AGAGTTCAAG 420
   ACCAGCCTAG GCAATGTGGT GAAACCCCAT CGCTACAAAA AATACAAAAA TTAGCCAGGC 480
   ATGGTGGCGT GCGCATGTAG TCCCAGCTCC TTGGGAGGCT GAGGTGGGAG GATCACTTGA 540
   ACCCAGGAGA CAGAGGTTGC AGTGAACCGA GATCACGCCA CCACGCTCCA GCCTGGGCAA 600
   CAGAACAAGA CTCTGTCTAA AAAAATACAA ATAAAATAAA AGTAGTTCTC ACAGTACCAG 660
   CATTCATTTT TCAAAAGATA TAGAGCTAAA AAGGAAGGAA AAAAAAAGTA ATGTTGGGCT 720
   TTTAAATACT CGTTCCTATA CTAAATGTTC TTAGGAGTGC TGGGGTTTTA TTGTCATCAT 780
   TTATCCTTTT TAAAAATGTT ATTGGCCAGG CACGGTGGCT CATGGCTGTA ATCCCAGCAC 840
   TTTGGGAGGC CGAGGCAGGC AGATCACCTG AGGTCAGGAG TGTGAGACCA GCCTGGCCAA 900
   CATGGCGAAA CCTGTCTCTA CTAAAAATAC AAAAATTAAC TAGGCGTGGT GGTGTACGCC 960
   TGTAGTCCCA GCTACTCGGG AGGCTGAGGC AGGAGAATCA ACTGAACCAG GGAGGTGGAG 1020
   GTTGCAGTGT GCCGAGATCA CGCCACTGCA CTCTAGCCTG GCAACAGAGC AAGATTCTGT 1080
   CTCAAAAAAA AAAAACATAT ATACACATAT ATCCCAAAGT GCTGGGATTA CATATATATA 1140
   TATATATATA TATTATATAT ATATATATAT ATATATGTGA TATATATGTG ATATATATAT 1200
   AACATATATA TATGTAATAT ATATGTGATA TATATATAAT ATATATATGT AATATATATG 1260
   TGATATATAT ATATACACAC ACACACACAT ATATATGTAT GTGTGTGTAC ACACACACAC 1320
   ACAAATTAGC CAGGCATAGT TGCACACGCT TGGTAGACCC AGCTACTCAG GAGGCTGAGG 1380
   GAGGAGAATC TCTTGAACTT AGGAGGCGGA GGTTGCAGTG AGCTGAGATT GCGCCACTGC 1440
   ACTCCAGCCT GGGTGACAGA GCAGGACTCT GTACACCCCC CAAAACAAAA AAAAAAGTTA 1500
   TCAGATGTGA TTGGAATGTA TATCAAGTAT CAGCTTCAAA ATATGCTATA TTAATACTTC 1560
   AAAAATTACA CAAATAATAC ATAATCAGGT TTGAAAAATT TAAGACAACM SAARAAAAAA 1620
   WYCMAATCAC AMATATCCCA CACATTTTAT TATTMCTMCT MCWATTATTT TGWAGAGMCT 1680
   GGGTCTCACY CYKTTGCTWA TGCTGGTCTT TGAACYCCYK GCCYCAARCA RTCCTSCTCC 1740
   ABCCTCCCAA RGTGCTGGGG ATWATAGGCA TGARCTAACC GCACCCAGCC CCAGACATTT 1800
   TAGTGTGTAA ATTCCTGGGC ATTTTTTCAA GGCATCATAC ATGTTAGCTG ACTGATGATG 1860
   GTCAATTTAT TTTGTCCATG GTGTCAAGTT TCTCTTCAGG AGGAAAAGCA CAGAACTGGC 1920
   CAACAATTGC TTGACTGTTC TTTACCATAC TGTTTAGCAG GAAACCAGTC TCAGTGTCCA 1980
   ACTCTCTAAC CTTGGAACTG TGAGAACTCT GAGGACAAAG CAGCGGATAC AACCTCAAAA 2040
   GACGTCTGTC TACATTGAAT TGGGTAAGGG TCTCAGGTTT TTTAAGTATT TAATAATAAT 2100
   TGCTGGATTC CTTATCTTAT AGTTTTGCCA AAAATCTTGG TCATAATTTG TATTTGTGGT 2160
   AGGCAGCTTT GGGAAGTGAA TTTTATGAGC CCTATGGTGA GTTATAAAAA ATGTAAAAGA 2220
   CGCAGTTCCC ACCTTGAAGA ATCTTACTTT AAAAAGGGAG CAAAAGAGGC CAGGCATGGT 2280
   GGCTCACACC TGTAATCCCA GCACTTTGGG AGGCCAAAGT GGGTGGATCA CCTGAGGTCG 2340
   GGAGTTCGAG ACCAGCCTAG CCAACATGGA GAAACTCTGT CTGTACCAAA AAATAAAAAA 2400
   TTAGCCAGGT GTGGTGGCAC ATAACTGTAA TCCCAGCTAC TCGGGAGGCT GAGGCAGGAG 2460
   AATCACTTGA ACCCGGGAGG TGGAGGTTGC GGTGAACCGA GATCGCACCA TTGCACTCCA 2520
   GCCTGGGCAA AAATAGCGAA ACTCCATCTA AAAAAAAAAA AGAGAGCAAA AGAAAGAMTM 2580
   TCTGGTTTTA AMTMTGTGTA AATATGTTTT TGGAAAGATG GAGAGTAGCA ATAAGAAAAA 2640
   ACATGATGGA TTGCTACAGT ATTTAGTTCC AAGATAAATT GTACTAGATG AGGAAGCCTT 2700
   TTAAGAAGAG CTGAATTGCC AGGCGCAGTG GCTCACGCCT GTAATCCCAG CACTTTGGGA 2760
   GGCCGAGGTG GGCGGATCAC CTGAGGTCGG GAGTTCAAGA CCAGCCTGAC CAACATGGAG 2820
   AAACCCCATC TCTACTAAAA AAAAAAAAAA AAAAATTAGC CGGGGTGGTG GCTTATGCCT 2880
   GTAATCCCAG CTACTCAGGA GGCTGAGGCA GGAGAATCGC TTGAACCCAG GAAGCAGAGG 2940
   TTGCAGTGAG CCAAGATCGC ACCATTGCAC TCCAGCCTAG GCAACAAGAG TGAAACTCCA 3000
   TCTCAAAAAA AAAAAAAAAG AGCTGAATCT TGGCTGGGCA GGATGGCTCG TGCCTGTAAT 3060
   CCTAACGCTT TGGAAGACCG AGGCAGAAGG ATTGGTTGAG TCCACGAGTT TAAGACCAGC 3120
   CTGGCCAACA TAGGGGAACC CTGTCTCTAT TTTTAAAATA ATAATACATT TTTGGCCGGT 3180
   GCGGTGGCTC ATGCCTGTAA TCCCAATACT TTGGGAGGCT GAGGCAGGTA GATCACCTGA 3240
   GGTCAGAGTT CGAGACCAGC CTGGATAACC TGGTGAAACC CCTCTTTACT AAAAATACAA 3300
   AAAAAAAAAA AAATTAGCTG GGTGTGGTAG CACATGCTTG TAATCCCAGC TACTTGGGAG 3360
   GCTGAGGCAG GAGAATCGCT TGAACCAGGG AGGCGGAGGT TACAATGAGC CAACACTACA 3420
   CCACTGCACT CCAGCCTGGG CAATAGAGTG AGACTGCATC TCAAAAAAAT AATAATTTTT 3480
   AAAAATAATA AATTTTTTTA AGCTTATAAA AAGAAAAGTT GAGGCCAGCA TAGTAGCTCA 3540
   CATCTGTAAT CTCAGCAGTG GCAGAGGATT GCTTGAAGCC AGGAGTTTGA GACCAGCCTG 3600
   GGCAACATAG CAAGACCTCA TCTCTACAAA AAAATTTCTT TTTTAAATTA GCTGGGTGTG 3660
   GTGGTGTGCA TCTGTAGTCC CAGCTACTCA GGAGGCAGAG GTGAGTGGAT ACATTGAACC 3720
   CAGGAGTTTG AGGCTGTAGT GAGCTATGAT CATGCCACTG CACTCCAACC TGGGTGACAG 3780
   AGCAAGACCT CCAAAAAAAA AAAAAAAAGA GCTGCTGAGC TCAGAATTCA AACTGGGCTC 3840
   TCAAATTGGA TTTTCTTTTA GAATATATTT ATAATTAAAA AGGATAGCCA TCTTTTGAGC 3900
   TCCCAGGCAC CACCATCTAT TTATCATAAC ACTTACTGTT TTCCCCCCTT ATGATCATAA 3960
   ATTCCTAGAC AACAGGCATT GTAAAAATAG TTATAGTAGT TGATATTTAG GAGCACTTAA 4020
   CTATATTCCA GGCACTATTG TGCTTTTCTT GTATAACTCA TTAGATGCTT GTCAGACCTC 4080
   TGAGATTGTT CCTATTATAC TTATTTTACA GATGAGAAAA TTAAGGCACA GAGAAGTTAT 4140
   GAAATTTTTC CAAGGTATTA AACCTAGTAA GTGGCTGAGC CATGATTCAA ACCTAGGAAG 4200
   TTAGATGTCA GAGCCTGTGC TTTTTTTTTG TTTTTGTTTT TGTTTTCAGT AGAAACGGGG 4260
   GTCTCACTTT GTTGGCCAGG CTGGTCTTGA ACTCCTAACC TCAAATAATC CACCCATCTC 4320
   GGCCTCCTCA AGTGCTGGGA TTACAGGTGA GAGCCACTGT GCCTGGCGAA GCCCATGCCT 4380
   TTAACCACTT CTCTGTATTA CATACTAGCT TAACTAGCAT TGTACCTGCC ACAGTAGATG 4440
   CTCAGTAAAT ATTTCTAGTT GAATATCTGT TTTTCAACAA GTACATTTTT TTAACCCTTT 4500
   TAATTAAGAA AACTTTTATT GATTTATTTT TTGGGGGGAA ATTTTTTAGG ATCTGATTCT 4560
   TCTGAAGATA CCGTTAATAA GGCAACTTAT TGCAGGTGAG TCAAAGAGAA CCTTTGTCTA 4620
   TGAAGCTGGT ATTTTCCTAT TTAGTTAATA TTAAGGATTG ATGTTTCTCT CTTTTTAAAA 4680
   ATATTTTAAC TTTTATTTTA GGTTCAGGGA TGTATGTGCA GTTTGTTATA TAGGTAAACA 4740
   CACGACTTGG GATTTGGTGT ATAGATTTTT TTCATCATCC GGGTACTAAG CATACCCCAC 4800
   AGTTTTTTGT TTGCTTTCTT TCTGAATTTC TCCCTCTTCC CACCTTCCTC CCTCAAGTAG 4860
   GCTGGTGTTT CTCCAGACTA GAATCATGGT ATTGGAAGAA ACCTTAGAGA TCATCTAGTT 4920
   TAGTTCTCTC ATTTTATAGT GGAGGAAATA CCCTTTTTGT TTGTTGGATT TAGTTATTAG 4980
   CACTGTCCAA AGGAATTTAG GATAACAGTA GAACTCTGCA CATGCTTGCT TCTAGCAGAT 5040
   TGTTCTCTAA GTTCCTCATA TACAGTAATA TTGACACAGC AGTAATTGTG ACTGATGAAA 5100
   ATGTTCAAGG ACTTCATTTT CAACTCTTTC TTTCCTCTGT TCCTTATTTC CACATATCTC 5160
   TCAAGCTTTG TCTGTATGTT ATATAATAAA CTACAAGCAA CCCCAACTAT GTTACCTACC 5220
   TTCCTTAGGA ATTATTGCTT GACCCAGGTT TTTTTTTTTT TTTTTTTGGA GACGGGGTCT 5280
   TGCCCTGTTG CCAGGATGGA GTGTAGTGGC GCCATCTCGG CTCACTGCAA TCTCCAACTC 5340
   CCTGGTTCAA GCGATTCTCC TGTCTCAATC TCACGAGTAG CTGGGACTAC AGGTATACAC 5400
   CACCACGCCC GGTTAATTGA CCATTCCATT TCTTTCTTTC TCTCTTTTTT TTTTTTTTTT 5460
   TTGAGACAGA GTCTTGCTCT GTTGCCCAGG CTGGAGTACA GAGGTGTGAT CTCACCTCTC 5520
   CGCAACGTCT GCCTCCCAGG TTGAAGCCAT ACTCCTGCCT CAGCCTCTCT AGTAGCTGGG 5580
   ACTACAGGCG CGCGCCACCA CACCCGGCTA ATTTTTGTAT TTTTAGTAGA GATGGGGTTT 5640
   CACCATGTTG GCCAGGCTGG TCTTGAACTC ATGACCTCAA GTGGTCCACC CGCCTCAGCC 5700
   TCCCAAAGTG CTGGAATTAC AGGCTTGAGC CACCGTGCCC AGCAACCATT TCATTTCAAC 5760
   TAGAAGTTTC TAAAGGAGAG AGCAGCTTTC ACTAACTAAA TAAGATTGGT CAGCTTTCTG 5820
   TAATCGAAAG AGCTAAAATG TTTGATCTTG GTCATTTGAC AGTTCTGCAT ACATGTAACT 5880
   AGTGTTTCTT ATTAGGACTC TGTCTTTTCC CTATAGTGTG GGAGATCAAG AATTGTTACA 5940
   AATCACCCCT CAAGGAACCA GGGATGAAAT CAGTTTGGAT TCTGCAAAAA AGGGTAATGG 6000
   CAAAGTTTGC CAACTTAACA GGCACTGAAA AGAGAGTGGG TAGATACAGT ACTGTAATTA 6060
   GATTATTCTG AAGACCATTT GGGACCTTTA CAACCCACAA AATCTCTTGG CAGAGTTAGA 6120
   GTATCATTCT CTGTCAAATG TCGTGGTATG GTCTGATAGA TTTAAATGGT ACTAGACTAA 6180
   TGTACCTATA ATAAGACCTT CTTGTAACTG ATTGTTGCCC TTTCGCTTTT TTTTTTGTTT 6240
   GTTTGTTTGT TTTTTTTTGA GATGGGGTCT CACTCTGTTG CCCAGGCTGG AGTGCAGTGA 6300
   TGCAATCTTG GCTCACTGCA ACCTCCACCT CCAAAGGCTC AAGCTATCCT CCCACTTCAG 6360
   CCTCCTGAGT AGCTGGGACT ACAGGCGCAT GCCACCACAC CCGGTTAATT TTTTGTGGTT 6420
   TTATAGAGAT GGGGTTTCAC CATGTTACCG AGGCTGGTCT CAAACTCCTG GACTCAAGCA 6480
   GTCTGCCCAC TTCAGCCTCC CAAAGTGCTG CAGTTACAGG CTTGAGCCAC TGTGCCTGGC 6540
   CTGCCCTTTA CTTTTAATTG GTGTATTTGT GTTTCATCTT TTACCTACTG GTTTTTAAAT 6600
   ATAGGGAGTG GTAAGTCTGT AGATAGAACA GAGTATTAAG TAGACTTAAT GGCCAGTAAT 6660
   CTTTAGAGTA CATCAGAACC AGTTTTCTGA TGGCCAATCT GCTTTTAATT CACTCTTAGA 6720
   CGTTAGAGAA ATAGGTGTGG TTTCTGCATA GGGAAAATTC TGAAATTAA 6769
(2) INFORMATION FOR SEQ ID NO:21:
   (i) SEQUENCE CHARACTERISTICS:
      (A) LENGTH: 4249 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:21:
   GATCCTAAGT GGAAATAATC TAGGTAAATA GGAATTAAAT GAAAGAGTAT GAGCTACATC    60
   TTCAGTATAC TTGGTAGTTT ATGAGGTTAG TTTCTCTAAT ATAGCCAGTT GGTTGATTTC   120
   CACCTCCAAG GTGTATGAAG TATGTATTTT TTTAATGACA ATTCAGTTTT TGAGTACCTT   180
   GTTATTTTTG TATATTTTCA GCTGCTTGTG AATTTTCTGA GACGGATGTA ACAAATACTG   240
   AACATCATCA ACCCAGTAAT AATGATTTGA ACACCACTGA GAAGCGTGCA GCTGAGAGGC   300
   ATCCAGAAAA GTATCAGGGT AGTTCTGTTT CAAACTTGCA TGTGGAGCCA TGTGGCACAA   360
   ATACTCATGC CAGCTCATTA CAGCATGAGA ACAGCAGTTT ATTACTCACT AAAGACAGAA   420
   TGAATGTAGA AAAGGCTGAA TTCTGTAATA AAAGCAAACA GCCTGGCTTA GCAAGGAGCC   480
   AACATAACAG ATGGGCTGGA AGTAAGGAAA CATGTAATGA TAGGCGGACT CCCAGCACAG   540
   AAAAAAAGGT AGATCTGAAT GCTGATCCCC TGTGTGAGAG AAAAGAATGG AATAAGCAGA   600
   AACTGCCATG CTCAGAGAAT CCTAGAGATA CTGAAGATGT TCCTTGGATA ACACTAAATA   660
   GCAGCATTCA GAAAGTTAAT GAGTGGTTTT CCAGAAGTGA TGAACTGTTA GGTTCTGATG   720
   ACTCACATGA TGGGGAGTCT GAATCAAATG CCAAAGTAGC TGATGTATTG GACGTTCTAA   780
   ATGAGGTAGA TGAATATTCT GGTTCTTCAG AGAAAATAGA CTTACTGGCC AGTGATCCTC   840
   ATGAGGCTTT AATATGTAAA AGTGAAAGAG TTCACTCCAA ATCAGTAGAG AGTAATATTG   900
   AAGGCCAAAT ATTTGGGAAA ACCTATCGGA AGAAGGCAAG CCTCCCCAAC TTAAGCCATG   960
   TAACTGAAAA TCTAATTATA GGAGCATTTG TTACTGAGCC ACAGATAATA CAAGAGCGTC  1020
   CCCTCACAAA TAAATTAAAG CGTAAAAGGA GACCTACATC AGGCCTTCAT CCTGAGGATT  1080
   TTATCAAGAA AGCAGATTTG GCAGTTCAAA AGACTCCTGA AATGATAAAT CAGGGAACTA 1140
   ACCAAACGGA GCAGAATGGT CAAGTGATGA ATATTACTAA TAGTGGTCAT GAGAATAAAA 1200
   CAAAAGGTGA TTCTATTCAG AATGAGAAAA ATCCTAACCC AATAGAATCA CTCGAAAAAG 1260
   AATCTGCTTT CAAAACGAAA GCTGAACCTA TAAGCAGCAG TATAAGCAAT ATGGAACTCG 1320
   AATTAAATAT CCACAATTCA AAAGCACCTA AAAAGAATAG GCTGAGGAGG AAGTCTTCTA 1380
   CCAGGCATAT TCATGCGCTT GAACTAGTAG TCAGTAGAAA TCTAAGCCCA CCTAATTGTA 1440
   CTGAATTGCA AATTGATAGT TGTTCTAGCA GTGAAGAGAT AAAGAAAAAA AAGTACAACC 1500
   AAATGCCAGT CAGGCACAGC AGAAACCTAC AACTCATGGA AGGTAAAGAA CCTGCAACTG 1560
   GAGCCAAGAA GAGTAACAAG CCAAATGAAC AGACAAGTAA AAGACATGAC AGCGATACTT 1620
   TCCCAGAGCT GAAGTTAACA AATGCACCTG GTTCTTTTAC TAAGTGTTCA AATACCAGTG 1680
   AACTTAAAGA ATTTGTCAAT CCTAGCCTTC CAAGAGAAGA AAAAGAAGAG AACTAGAAAC 1740
   AGTTAAAGTG TCTAATAATG CTGAAGACCC CAAAGATCTC ATGTTAAGTG GAGAAAGGGT 1800
   TTTGCAAACT GAAAGATCTG TAGAGAGTAG CAGTATTTCA TTGGTACCTG GTACTGATTA 1860
   TGGCACTCAG GAAAGTATCT CGTTACTGGA AGTTAGCACT CTAGGGAAGG CAAAAACAGA 1920
   ACCAAATAAA TGTGTGAGTC AGTGTGCAGC ATTTGAAAAC CCCAAGGGAC TAATTCATGG 1980
   TTGTTCCAAA GATAATAGAA ATGACACAGA AGGCTTTAAG TATCCATTGG GACATGAAGT 2040
   TAACCACAGT CGGGAAACAA GCATAGAAAT GGAAGAAAGT GAACTTGATG CTCAGTATTT 2100
   GCAGAATACA TTCAAGGTTT CAAAGCGCCA GTCATTTGCT CCGTTTTCAA ATCCAGGAAA 2160
   TGCAGAAGAG GAATGTGCAA CATTCTCTGC CCACTCTGGG TCCTTAAAGA AACAAAGTCC 2220
   AAAAGTCACT TTTGAATGTG AACAAAAGGA AGAAAATCAA GGAAAGAATG AGTCTAATAT 2280
   CAAGCCTGTA CAGACAGTTA ATATCACTGC AGGCTTTCCT GTGGTTGGTC AGAAAGATAA 2340
   GCCAGTTGAT AATGCCAAAT GTAGTATCAA AGGAGGCTCT AGGTTTTGTC TATCATCTCA 2400
   GTTCAGAGGC AACGAAACTG GACTCATTAC TCCAAATAAA CATGGACTTT TACAAAACCC 2460
   ATATCGTATA CCACCACTTT TTCCCATCAA GTCATTTGTT AAAACTAAAT GTAAGAAAAA 2520
   TCTGCTAGAG GAAAACTTTG AGGAACATTC AATGTCACCT GAAAGAGAAA TGGGAAATGA 2580
   GAACATTCCA AGTACAGTGA GCACAATTAG CCGTAATAAC ATTAGAGAAA ATGTTTTTAA 2640
   AGAAGCCAGC TCAAGCAATA TTAATGAAGT AGGTTCCAGT ACTAATGAAG TGGGCTCCAG 2700
   TATTAATGAA ATAGGTTCCA GTGATGAAAA CATTCAAGCA GAACTAGGTA GAAACAGAGG 2760
   GCCAAAATTG AATGCTATGC TTAGATTAGG GGTTTTGCAA CCTGAGGTCT ATAAACAAAG 2820
   TCTTCCTGGA AGTAATTGTA AGCATCCTGA AATAAAAAAG CAAGAATATG AAGAAGTAGT 2880
   TCAGACTGTT AATACAGATT TCTCTCCATA TCTGATTTCA GATAACTTAG AACAGCCTAT 2940
   GGGAAGTAGT CATGCATCTC AGGTTTGTTC TGAGACACCT GATGACCTGT TAGATGATGG 3000
   TGAAATAAAG GAAGATACTA GTTTTGCTGA AAATGACATT AAGGAAAGTT CTGCTGTTTT 3060
   TAGCAAAAGC GTCCAGAAAG GAGAGCTTAG CAGGAGTCCT AGCCCTTTCA CCCATACACA 3120
   TTTGGCTCAG GGTTACCGAA GAGGGGCCAA GAAATTAGAG TCCTCAGAAG AGAACTTATC 3180
   TAGTGAGGAT GAAGAGCTTC CCTGCTTCCA ACACTTGTTA TTTGGTAAAG TAAACAATAT 3240
   ACCTTCTCAG TCTACTAGGC ATAGCACCGT TGCTACCGAG TGTCTGTCTA AGAACACAGA 3300
   GGAGAATTTA TTATCATTGA AGAATAGCTT AAATGACTGC AGTAACCAGG TAATATTGGC 3360
   AAAGGCATCT CAGGAACATC ACCTTAGTGA GGAAACAAAA TGTTCTGCTA GCTTGTTTTC 3420
   TTCACAGTGC AGTGAATTGG AAGACTTGAC TGCAAATACA AACACCCAGG ATCCTTTCTT 3480
   GATTGGTTCT TCCAAACAAA TGAGGCATCA GTCTGAAAGC CAGGGAGTTG GTCTGAGTGA 3540
   CAAGGAATTG GTTTCAGATG ATGAAGAAAG AGGAACGGGC TTGGAAGAAA ATAATCAAGA 3600
   AGAGCAAAGC ATGGATTCAA ACTTAGGTAT TGGAACCAGG TTTTTGTGTT TGCCCCAGTC 3660
   TATTTATAGA AGTGAGCTAA ATGTTTATGC TTTTGGGGAG CACATTTTAC AAATTTCCAA 3720
   GTATAGTTAA AGGAACTGCT TCTTAAACTT GAAACATGTT CCTCCTAAGG TGCTTTTCAT 3780
   AGAAAAAAGT CCTTCACACA GCTAGGACGT CATCTTTGAC TGAATGAGCT TTAACATCCT 3840
   AATTACTGGT GGACTTACTT CTGGTTTCAT TTTATAAAGC AAATCCCGGT GTCCCAAAGC 3900
   AAGGAATTTA ATCATTTTGT GTGACATGAA AGTAAATCCA GTCCTGCCAA TGAGAAGAAA 3960
   AAGACACAGC AAGTTGCAGC GTTTATAGTC TGCTTTTACA TCTGAACCTC TGTTTTTGTT 4020
   ATTTAAGGTG AAGCAGCATC TGGGTGTGAG AGTGAAACAA GCGTCTCTGA AGACTGCTCA 4080
   GGGCTATCCT CTCAGAGTGA CATTTTAACC ACTCAGGTAA AAAGCGTGTG TGTGTGTGCA 4140
   CATGCGTGTG TGTGGTGTCC TTTGCATTCA GTAGTATGTA TCCCACATTC TTAGGTTTGC 4200
   TGACATCATC TCTTTGAATT AATGGCACAA TTGTTTGTGG TTCATTGTC 4249
(2) INFORMATION FOR SEQ ID NO:22:
   (i) SEQUENCE CHARACTERISTICS:
      (A) LENGTH: 710 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:22:
   NGNGAATGTA ATCCTAATAT TTCNCNCCNA CTTAAAAGAA TACCACTCCA ANGGCATCNC    60
   AATACATCAA TCAATTGGGG AATTGGGATT TTCCCTCNCT AACATCANTG GAATAATTTC   120
   ATGGCATTAA TTGCATGAAT GTGGTTAGAT TAAAAGGTGT TCATGCTAGA ACTTGTAGTT   180
   CCATACTAGG TGATTTCAAT TCCTGTGCTA AAATTAATTT GTATGATATA TTNTCATTTA   240
   ATGGAAAGCT TCTCAAAGTA TTTCATTTTC TTGGTACCAT TTATCGTTTT TGAAGCAGAG   300
   GGATACCATG CAACATAACC TGATAAAGCT CCAGCAGGAA ATGGCTGAAC TAGAAGCTGT   360
   GTTAGAACAG CATGGGAGCC AGCCTTCTAA CAGCTACCCT TCCATCATAA GTGACTCTTC   420
   TGCCCTTGAG GACCTGCGAA ATCCAGAACA AAGCACATCA GAAAAAGGTG TGTATTGTTG   480
   GCCAAACACT GATATCTTAA GCAAAATTCT TTCCTTCCCC TTTATCTCCT TCTGAAGAGT   540
   AAGGACCTAG CTCCAACATT TTATGATCCT TGCTCAGCAC ATGGGTAATT ATGGAGCCTT   600
   GGTTCTTGTC CCTGCTCACA ACTAATATAC CAGTCAGAGG GACCCAAGGC AGTCATTCAT   660
   GTTGTCATCT GAGATACCTA CAACAAGTAG ATGCTATGGG GAGCCCATGG              710
(2) INFORMATION FOR SEQ ID NO:23:
   (i) SEQUENCE CHARACTERISTICS:
      (A) LENGTH: 473 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:23:
   CCATTGGTGC TAGCATCTGT CTGTTGCATT GCTTGTGTTT ATAAAATTCT GCCTGATATA     60
   CTTGTTAAAA ACCAATTTGT GTATCATAGA TTGATGCTTT TGAAAAAAAT CAGTATTCTA    120
   ACCTGAATTA TCACTATCAG AACAAAGCAG TAAAGTAGAT TTGTTTTCTC ATTCCATTTA    180
   AAGCAGTATT AACTTCACAG AAAAGTAGTG AATACCCTAT AAGCCAGAAT CCAGAAGGCC    240
   TTTCTGCTGA CAAGTTTGAG GTGTCTGCAG ATAGTTCTAC CAGTAAAAAT AAAGAACCAG    300
   GAGTGGAAAG GTAAGAAACA TCAATGTAAA GATGCTGTGG TATCTGACAT CTTTATTTAT    360
   ATTGAACTCT GATTGTTAAT TTTTTTCACC ATACTTTCTC CAGTTTTTTT GCATACAGGC    420
   ATTTATACAC TTTTATTGCT CTAGGATACT TCTTTTGTTT AATCCTATAT AGG           473
(2) INFORMATION FOR SEQ ID NO:24:
   (i) SEQUENCE CHARACTERISTICS:
      (A) LENGTH: 421 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:24:
   GGATAAGNTC AAGAGATATT TTGATAGGTG ATGCAGTGAT NAATTGNGAA AATTTNCTGC     60
   CTGCTTTTAA TCTTCCCCCG TTCTTTCTTC CTNCCTCCCT CCCTTCCTNC CTCCCGTCCT    120
   TNCCTTTCCT TTCCCTCCCT TCCNCCTTCT TTCCNTCTNT CTTTCCTTTC TTTCCTGTCT    180
   ACCTTTCTTT CCTTCCTCCC TTCCTTTTCT TTTCTTTCTT TCCTTTCCTT TTCTTTCCTT    240
   TCTTTCCTTT CCTTTCTTTC TTGACAGAGT CTTGCTCTGT CACTCAGGCT GGAGTGCAGT    300
   GGCGTGATCT CGNCTCACTG CAACCTCTGT CTCCCAGGTT CAAGCAATTT TCCTGCCTCA    360
   GCCTCCCGAG TAGCTGAGAT TACAGGCGCC AGCCACCACA CCCAGCTACT GACCTGCTTT    420
   T                                                                    421
(2) INFORMATION FOR SEQ ID NO:25:
   (i) SEQUENCE CHARACTERISTICS:
      (A) LENGTH: 997 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:25:
   AAACAGCTGG GAGATATGGT GCCTCAGACC AACCCCATGT TATATGTCAA CCCTGACATA     60
   TTGGCAGGCA ACATGAATCC AGACTTCTAG GCTGTCATGC GGGCTCTTTT TTGCCAGTCA    120
   TTTCTGATCT CTCTGACATG AGCTGTTTCA TTTATGCTTT GGCTGCCCAG CAAGTATGAT    180
   TTGTCCTTTC ACAATTGGTG GCGATGGTTT TCTCCTTCCA TTTATCTTTC TAGGTCATCC    240
   CCTTCTAAAT GCCCATCATT AGATGATAGG TGGTACATGC ACAGTTGCTC TGGGAGTCTT    300
   CAGAATAGAA ACTACCCATC TCAAGAGGAG CTCATTAAGG TTGTTGATGT GGAGGAGCAA    360
   CAGCTGGAAG AGTCTGGGCC ACACGATTTG ACGGAAACAT CTTACTTGCC AAGGCAAGAT    420
   CTAGGTAATA TTTCATCTGC TGTATTGGAA CAAACACTYT GATTTTACTC TGAATCCTAC    480
   ATAAAGATAT TCTGGTTAAC CAACTTTTAG ATGTACTAGT CTATCATGGA CACTTTTGTT    540
   ATACTTAATT AAGCCCACTT TAGAAAAATA GCTCAAGTGT TAATCAAGGT TTACTTGAAA    600
   ATTATTGAAA CTGTTAATCC ATCTATATTT TAATTAATGG TTTAACTAAT GATTTTGAGG    660
   ATGWGGGAGT CKTGGTGTAC TCTAMATGTA TTATTTCAGG CCAGGCATAG TGGCTCACGC    720
   CTGGTAATCC CAGTAYYCMR GAGCCCGAGG CAGGTGGAGC CAGCTGAGGT CAGGAGTTCA    780
   AGACCTGTCT TGGCCAACAT GGGNGAAACC CTGTCTTCTT CTTAAAAAAN ACAAAAAAAA    840
   TTAACTGGGT TGTGCTTAGG TGNATGCCCC GNATCCTAGT TNTTCTTGNG GGTTGAGGGA    900
   GGAGATCACN TTGGACCCCG GAGGGGNGGG TGGGGGNGAG CAGGNCAAAA CACNGACCCA    960
   GCTGGGGTGG AAGGGAAGCC CACTCNAAAA AANNTTN                             997
(2) INFORMATION FOR SEQ ID NO:26:
   (i) SEQUENCE CHARACTERISTICS:
      (A) LENGTH: 639 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:26:
   TTTTTAGGAA ACAAGCTACT TTGGATTTCC ACCAACACCT GTATTCATGT ACCCATTTTT    60
   CTCTTAACCT AACTTTATTG GTCTTTTTAA TTCTTAACAG AGACCAGAAC TTTGTAATTC   120
   AACATTCATC GTTGTGTAAA TTAAACTTCT CCCATTCCTT TCAGAGGGAA CCCCTTACCT   180
   GGAATCTGGA ATCAGCCTCT TCTCTGATGA CCCTGAATCT GATCCTTCTG AAGACAGAGC   240
   CCCAGAGTCA GCTCGTGTTG GCAACATACC ATCTTCAACC TCTGCATTGA AAGTTCCCCA   300
   ATTGAAAGTT GCAGAATCTG CCCAGAGTCC AGCTGCTGCT CATACTACTG ATACTGCTGG   360
   GTATAATGCA ATGGAAGAAA GTGTGAGCAG GGAGAAGCCA GAATTGACAG CTTCAACAGA   420
   AAGGGTCAAC AAAAGAATGT CCATGGTGGT GTCTGGCCTG ACCCCAGAAG AATTTGTGAG   480
   TGTATCCATA TGTATCTCCC TAATGACTAA GACTTAACAA CATTCTGGAA AGAGTTTTAT   540
   GTAGGTATTG TCAATTAATA ACCTAGAGGA AGAAATCTAG AAAACAATCA CAGTTCTGTG   600
   TAATTTAATT TCGATTACTA ATTTCTGAAA ATTTAGAAY                           639
(2) INFORMATION FOR SEQ ID NO:27:
   (i) SEQUENCE CHARACTERISTICS:
      (A) LENGTH: 922 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:27:
   NCCCNNCCCC CNAATCTGAA ATGGGGGTAA CCCCCCCCCA ACCGANACNT GGGTNGCNTA    60
   GAGANTTTAA TGGCCCNTTC TGAGGNACAN AAGCTTAAGC CAGGNGACGT GGANCNATGN   120
   GTTGTTTNTT GTTTGGTTAC CTCCAGCCTG GGTGACAGAG CAAGACTCTG TCTAAAAAAA   180
   AAAAAAAAAA AAATCGACTT TAAATAGTTC CAGGACACGT GTAGAACGTG CAGGATTGCT   240
   ACGTAGGTAA ACATATGCCA TGGTGGGATA ACTAGTATTC TGAGCTGTGT GCTAGAGGTA   300
   ACTCATGATA ATGGAATATT TGATTTAATT TCAGATGCTC GTGTACAAGT TTGCCAGAAA   360
   ACACCACATC ACTTTAACTA ATCTAATTAC TGAAGAGACT ACTCATGTTG TTATGAAAAC   420
   AGGTATACCA AGAACCTTTA CAGAATACCT TGCATCTGCT GCATAAAACC ACATGAGGCG   480
   AGGCACGGTG GCGCATGCCT GTAATCGCAG CACTTTGGGA GGCCGAGGCG GGCAGATCAC   540
   GAGATTAGGA GATCGAGACC ATCCTGGCCA GCATGGTGAA ACCCCGTCTC TACTANNAAA   600
   TGGNAAAATT ANCTGGGTGT GGTCGCGTGC NCCTGTAGTC CCAGCTACTC GTGAGGCTGA   660
   GGCAGGAGAA TCACTTGAAC CGGGGAAATG GAGGTTTCAG TGAGCAGAGA TCATNCCCCT   720
   NCATTCCAGC CTGGCGACAG AGCAAGGCTC CGTCNCCNAA AAAATAAAAA AAAACGTGAA   780
   CAAATAAGAA TATTTGTTGA GCATAGCATG GATGATAGTC TTCTAATAGT CAATCAATTA   840
   CTTTATGAAA GACAAATAAT AGTTTTGCTG CTTCCTTACC TCCTTTTGTT TTGGGTTAAG   900
   ATTTGGAGTG TGGGCCAGGC AC                                            922
(2) INFORMATION FOR SEQ ID NO:28:
   (i) SEQUENCE CHARACTERISTICS:
      (A) LENGTH: 867 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:28:
   GATCTATAGC TAGCCTTGGC GTCTAGAAGA TGGGTGTTGA GAAGAGGGAG TGGAAAGATA      60
   TTTCCTCTGG TCTTAACTTC ATATCAGCCT CCCCTAGACT TCCAAATATC CATACCTGCT     120
   GGTTATAATT AGTGGTGTTT TCAGCCTCTG ATTCTGTCAC CAGGGGTTTT AGAATCATAA     180
   ATCCAGATTG ATCTTGGGAG TGTAAAAAAC TGAGGCTCTT TAGCTTCTTA GGACAGCACT     240
   TCCTGATTTT GTTTTCAACT TCTAATCCTT TGAGTGTTTT TCATTCTGCA GATGCTGAGT     300
   TTGTGTGTGA ACGGACACTG AAATATTTTC TAGGAATTGC GGGAGGAAAA TGGGTAGTTA     360
   GCTATTTCTG TAAGTATAAT ACTATTTCTC CCCTCCTCCC TTTAACACCT CAGAATTGCA     420
   TTTTTACACC TAACATTTAA CACCTAAGGT TTTTGCTGAT GCTGAGTCTG AGTTACCAAA     480
   AGGTCTTTAA ATTGTAATAC TAAACTACTT TTATCTTTAA TATCACTTTG TTCAAGATAA     540
   GCTGGTGATG CTGGGAAAAT GGGTCTCTTT TATAACTAAT AGGACCTAAT CTGCTCCTAG     600
   CAATGTTAGC ATATGAGCTA GGGATTTATT TAATAGTCGG CAGGAATCCA TGTGCARCAG     660
   NCAAACTTAT AATGTTTAAA TTAAACATCA ACTCTGTCTC CAGAAGGAAA CTGCTGCTAC     720
   AAGCCTTATT AAAGGGCTGT GGCTTTAGAG GGAAGGACCT CTCCTCTGTC ATTCTTCCTG     780
   TGCTCTTTTG TGAATCGCTG ACCTCTCTAT CTCCGTGAAA AGAGCACGTT CTTCTGCTGT     840
   ATGTAACCTG TCTTTTCTAT GATCTCT                                          867
(2) INFORMATION FOR SEQ ID NO:29:
   (i) SEQUENCE CHARACTERISTICS:
      (A) LENGTH: 561 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:29:
   NAAAAACGGG GNNGGGANTG GGCCTTAAAN CCAAAGGGCN AACTCCCCAA CCATTNAAAA 60
   ANTGACNGGG GATTATTAAA ANCGGCGGGA AACATTTCAC NGCCCAACTA ATATTGTTAA 120
   ATTAAAACCA CCACCNCTGC NCCAAGGAGG GAAACTGCTG CTACAAGCCT TATTAAAGGG 180
   CTGTGGCTTT AGAGGGAAGG ACCTCTCCTC TGTCATTCTT CCTGTGCTCT TTTGTGAATC 240
   GCTGACCTCT CTATGTCCGT GAAAAGAGCA CGTTCTTCGT CTGTATGTAA CCTGTCTTTT 300
   CTATGATCTC TTTAGGGGTG ACCCAGTCTA TTAAAGAAAG AAAAATGCTG AATGAGGTAA 360
   GTACTTGATG TTACAAACTA ACCAGAGATA TTCATTCAGT CATATAGTTA AAAATGTATT 420
   TGCTTCCTTC CATCAATGCA CCACTTTCCT TAACAATGCA CAAATTTTCC ATGATAATGA 480
   GGATCATCAA GAATTATGCA GGCCTGCACT GTGGCTCATA CCTATAATCC CAGCGCTTTG 540
   GGAGGCTGAG GCGCTTGGAT C 561
(2) INFORMATION FOR SEQ ID NO:30:
   (i) SEQUENCE CHARACTERISTICS:
      (A) LENGTH: 567 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:30:
   AATTTTTTGT ATTTTTAGTA GAGATGAGGT TCACCATGTT GGTCTAGATC TGGTGTCGAA 60
   CGTCCTGACC TCAAGTGATC TGCCAGCCTC AGTCTCCCAA AGTGCTAGGA TTACAGGGGT 120
   GAGCCACTGC GCCTGGCCTG AATGCCTAAA ATATGACGTG TCTGCTCCAC TTCCATTGAA 180
   GGAAGCTTCT CTTTCTCTTA TCCTGATGGG TTGTGTTTGG TTTCTTTCAG CATGATTTTG 240
   AAGTCAGAGG AGATGTGGTC AATGGAAGAA ACCACCAAGG TCCAAAGCGA GCAAGAGAAT 300
   CCCAGGACAG AAAGGTAAAG CTCCCTCCCT CAAGTTGACA AAAATCTCAC CCCACCACTC 360
   TGTATTCCAC TCCCCTTTGC AGAGATGGGC CGCTTCATTT TGTAAGACTT ATTACATACA 420
   TACACAGTGC TAGATACTTT CACACAGGTT CTTTTTTCAC TCTTCCATCC CAACCACATA 480
   AATAAGTATT GTCTCTACTT TATGAATGAT AAAACTAAGA GATTTAGAGA GGCTGTGTAA 540
   TTTGGATTCC CGTCTCGGGT TCAGATC 567
(2) INFORMATION FOR SEQ ID NO:31:
   (i) SEQUENCE CHARACTERISTICS:
      (A) LENGTH: 633 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:31:
   TTGGCCTGAT TGGTGACAAA AGTGAGATGC TCAGTCCTTG AATGACAAAG AATGCCTGTA 60
   GAGTTGCAGG TCCAACTACA TATGCACTTC AAGAAGATCT TCTGAAATCT AGTAGTGTTC 120
   TGGACATTGG ACTGCTTGTC CCTGGGAAGT AGCAGCAGAA ATGATCGGTG GTGAACAGAA 180
   GAAAAAGAAA AGCTCTTCCT TTTTGAAAGT CTGTTTTTTG AATAAAAGCC AATATTCTTT 240
   TATAACTAGA TTTTCCTTCT CTCCATTCCC CTGTCCCTCT CTCTTCCTCT CTTCTTCCAG 300
   ATCTTCAGGG GGCTAGAAAT CTGTTGCTAT GGGCCCTTCA CCAACATGCC CACAGGTAAG 360
   AGCCTGGGAG AACCCCAGAG TTCCAGCACC AGCCTTTGTC TTACATAGTG GAGTATTATA 420
   AGCAAGGTCC CACGATGGGG GTTCCTCAGA TTGCTGAAAT GTTCTAGAGG CTATTCTATT 480
   TCTCTACCAC TCTCCAAACA AAACAGCACC TAAATGTTAT CCTATGGCAA AAAAAAACTA 540
   TACCTTGTCC CCCTTCTCAA GAGCATGAAG GTGGTTAATA GTTAGGATTC AGTATGTTAT 600
   GTGTTCAGAT GGCGTTGAGC TGCTGTTAGT GCC 633
(2) INFORMATION FOR SEQ ID NO:32:
   (i) SEQUENCE CHARACTERISTICS:
      (A) LENGTH: 470 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:32:
   TTTGAGAGAC TATCAAACCT TATACCAAGT GGCCTTATGG AGACTGATAA CCAGAGTACA     60
   TGGCATATCA GTGGCAAATT GACTTAAAAT CCATACCCCT ACTATTTTAA GACCATTGTC    120
   CTTTGGAGCA GAGAGACAGA CTCTCCCATT GAGAGGTCTT GCTATAAGCC TTCATCCGGA    180
   GAGTGTAGGG TAGAGGGCCT GGGTTAAGTA TGCAGATTAC TGCAGTGATT TTACATGTAA    240
   ATGTCCATTT TAGATCAACT GGAATGGATG GTACAGCTGT GTGGTGCTTC TGTGGTGAAG    300
   GAGCTTTCAT CATTCACCCT TGGCACAGTA AGTATTGGGT GCCCTGTCAG TGTGGGAGGA    360
   CACAATATTC TCTCCTGTGA GCAAGACTGG CACCTGTCAG TCCCTATGGA TGCCCCTACT    420
   GTAGCCTCAG AAGTCTTCTC TGCCCACATA CCTGTGCCAA AAGACTCCAT               470
(2) INFORMATION FOR SEQ ID NO:33:
   (i) SEQUENCE CHARACTERISTICS:
      (A) LENGTH: 517 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:33:
   GGTGGTACGT GTCTGTAGTT CCAGCTACTT GGGAGGCTGA GATGGAAGGA TTGCTTGAGC 60
   CCAGGAGGCA GAGGTGGNAN NTTACGCTGA GATCACACCA CTGCACTCCA GCCTGGGTGA 120
   CAGAGCAAGA CCCTGTCTCA AAAACAAACA AAAAAAATGA TGAAGTGACA GTTCCAGTAG 180
   TCCTACTTTG ACACTTTGAA TGCTCTTTCC TTCCTGGGGA TCCAGGGTGT CCACCCAATT 240
   GTGGTTGTGC AGCCAGATGC CTGGACAGAG GACAATGGCT TCCATGGTAA GGTGCCTCGC 300
   ATGTACCTGT GCTATTAGTG GGGTCCTTGT GCATGGGTTT GGTTTATCAC TCATTACCTG 360
   GTGCTTGAGT AGCACAGTTC TTGGCACATT TTTAAATATT TGTTGAATGA ATGGCTAAAA 420
   TGTCTTTTTG ATGTTTTTAT TGTTATTTGT TTTATATTGT AAAAGTAATA CATGAACTGT 480
   TTCCATGGGG TGGGAGTAAG ATATGAATGT TCATCAC 517
(2) INFORMATION FOR SEQ ID NO:34:
   (i) SEQUENCE CHARACTERISTICS:
      (A) LENGTH: 434 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:34:
   CAGTAATCCT NAGAACTCAT ACGACCGGGC CCCTGGAGTC GNTGNTTNGA GCCTAGTCCN    60
   GGAGAATGAA TTGACACTAA TCTCTGCTTG TGTTCTCTGT CTCCAGCAAT TGGGCAGATG   120
   TGTGAGGCAC CTGTGGTGAC CCGAGAGTGG GTGTTGGACA GTGTAGCACT CTACCAGTGC   180
   CAGGAGCTGG ACACCTACCT GATACCCCAG ATCCCCCACA GCCACTACTG ACTGCAGCCA   240
   GCCACAGGTA CAGAGCCACA GGACCCCAAG AATGAGCTTA CAAAGTGGCC TTTCCAGGCC   300
   CTGGGAGCTC CTCTCACTCT TCAGTCCTTC TACTGTCCTG GCTACTAAAT ATTTTATGTA   360
   CATCAGCCTG AAAAGGACTT CTGGCTATGC AAGGGTCCCT TAAAGATTTT CTGCTTGAAG   420
   TCTCCCTTGG AAAT                                                     434
(2) INFORMATION FOR SEQ ID NO:35:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:35:
      GATAAATTAA AACTGCGACT GCGCGGCGTG 30
(2) INFORMATION FOR SEQ ID NO:36:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:36:
      GTAGTAGAGT CCCGGGAAAG GGACAGGGGG 30
(2) INFORMATION FOR SEQ ID NO:37:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:37:
      ATATATATAT GTTTTTCTAA TGTGTTAAAG 30
(2) INFORMATION FOR SEQ ID NO:38:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:38:
      GTAAGTCAGC ACAAGAGTGT ATTAATTTGG 30
(2) INFORMATION FOR SEQ ID NO:39:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:39:
      TTTCTTTTTC TCCCCCCCCT ACCCTGCTAG 30
(2) INFORMATION FOR SEQ ID NO:40:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:40:
      GTAAGTTTGA ATGTGTTATG TGGCTCCATT 30
(2) INFORMATION FOR SEQ ID NO:41:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:41:
      AGCTACTTTT TTTTTTTTTT TTTGAGACAG 30
(2) INFORMATION FOR SEQ ID NO:42:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:42:
      GTAAGTGCAC ACCACCATAT CCAGCTAAAT 30
(2) INFORMATION FOR SEQ ID NO:43:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:43:
      AATTGTTCTT TCTTTCTTTA TAATTTATAG 30
(2) INFORMATION FOR SEQ ID NO:44:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:44:
      GTATATAATT TGGTAATGAT GCTAGGTTGG 30
(2) INFORMATION FOR SEQ ID NO:45:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:45:
      GAGTGTGTTT CTCAAACAAT TTAATTTCAG 30
(2) INFORMATION FOR SEQ ID NO:46:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:46:
      GTAAGTGTTG AATATCCCAA GAATGACACT 30
(2) INFORMATION FOR SEQ ID NO:47:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:47:
      AAACATAATG TTTTCCCTTG TATTTTACAG 30
(2) INFORMATION FOR SEQ ID NO:48:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:48:
      GTAAAACCAT TTGTTTTCTT CTTCTTCTTC 30
(2) INFORMATION FOR SEQ ID NO:49:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:49:
      TGCTTGACTG TTCTTTACCA TACTGTTTAG 30
(2) INFORMATION FOR SEQ ID NO:50:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:50:
      GTAAGGGTCT CAGGTTTTTT AAGTATTTAA 30
(2) INFORMATION FOR SEQ ID NO:51:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:51:
      TGATTTATTT TTTGGGGGGA AATTTTTTAG 30
(2) INFORMATION FOR SEQ ID NO:52:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:52:
      GTGAGTCAAA GAGAACCTTT GTCTATGAAG 30
(2) INFORMATION FOR SEQ ID NO:53:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:53:
      TCTTATTAGG ACTCTGTCTT TTCCCTATAG 30
(2) INFORMATION FOR SEQ ID NO:54:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:54:
      GTAATGGCAA AGTTTGCCAA CTTAACAGGC 30
(2) INFORMATION FOR SEQ ID NO:55:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:55:
      GAGTACCTTG TTATTTTTGT ATATTTTCAG 30
(2) INFORMATION FOR SEQ ID NO:56:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:56:
      GTATTGGAAC CAGGTTTTTG TGTTTGCCCC 30
(2) INFORMATION FOR SEQ ID NO:57:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:57:
      ACATCTGAAC CTCTGTTTTT GTTATTTAAG 30
(2) INFORMATION FOR SEQ ID NO:58:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:58:
      AGGTAAAAAG CGTGTGTGTG TGTGCACATG 30
(2) INFORMATION FOR SEQ ID NO:59:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:59:
      CATTTTCTTG GTACCATTTA TCGTTTTTGA 30
(2) INFORMATION FOR SEQ ID NO:60:
   (i) SEQUENCE CHARACTERISTICS :
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:60:
      GTGTGTATTG TTGGCCAAAC ACTGATATCT 30
(2) INFORMATION FOR SEQ ID NO:61:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:61:
      AGTAGATTTG TTTTCTCATT CCATTTAAAG 30
(2) INFORMATION FOR SEQ ID NO:62:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:62:
      GTAAGAAACA TCAATGTAAA GATGCTGTGG 30
(2) INFORMATION FOR SEQ ID NO:63:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:63:
      ATGGTTTTCT CCTTCCATTT ATCTTTCTAG 30
(2) INFORMATION FOR SEQ ID NO:64:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:64:
      GTAATATTTC ATCTGCTGTA TTGGAACAAA 30
(2) INFORMATION FOR SEQ ID NO:65:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:65:
      TGTAAATTAA ACTTCTCCCA TTCCTTTCAG 30
(2) INFORMATION FOR SEQ ID NO:66:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:66:
      GTGAGTGTAT CCATATGTAT CTCCCTAATG 30
(2) INFORMATION FOR SEQ ID NO:67:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:67:
      ATGATAATGG AATATTTGAT TTAATTTCAG 30
(2) INFORMATION FOR SEQ ID NO:68:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:68:
      GTATACCAAG AACCTTTACA GAATACCTTG 30
(2) INFORMATION FOR SEQ ID NO:69:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:69:
      CTAATCCTTT GAGTGTTTTT CATTCTGCAG 30
(2) INFORMATION FOR SEQ ID NO:70:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:70:
      GTAAGTATAA TACTATTTCT CCCCTCCTCC 30
(2) INFORMATION FOR SEQ ID NO:71:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:71:
      TGTAACCTGT CTTTTCTATG ATCTCTTTAG 30
(2) INFORMATION FOR SEQ ID NO:72:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:72:
      GTAAGTACTT GATGTTACAA ACTAACCAGA 30
(2) INFORMATION FOR SEQ ID NO:73:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:73:
      TCCTGATGGG TTGTGTTTGG TTTCTTTCAG 30
(2) INFORMATION FOR SEQ ID NO:74:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:74:
      GTAAAGCTCC CTCCCTCAAG TTGACAAAAA 30
(2) INFORMATION FOR SEQ ID NO:75:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:75:
      CTGTCCCTCT CTCTTCCTCT CTTCTTCCAG 30
(2) INFORMATION FOR SEQ ID NO:76:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:76:
      GTAAGAGCCT GGGAGAACCC CAGAGTTCCA 30
(2) INFORMATION FOR SEQ ID NO:77:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:77:
      AGTGATTTTA CATGTAAATG TCCATTTTAG 30
(2) INFORMATION FOR SEQ ID NO:78:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:78:
      AGTGATTTTA CATGTAAATG TCCATTTTAG 30
(2) INFORMATION FOR SEQ ID NO:79:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:79:
      TTGAATGCTC TTTCCTTCCT GGGGATCCAG 30
(2) INFORMATION FOR SEQ ID NO:80:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:80:
      GTAAGGTGCC TCGCATGTAC CTGTGCTATT 30
(2) INFORMATION FOR SEQ ID NO:81:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Homo sapiens
   (xi) SEQUENCE DESCRIPTION: SEQ ID NO:81:
      CTAATCTCTG CTTGTGTTCT CTGTCTCCAG 30
(2) INFORMATION FOR SEQ ID NO:82:
   (i) SEQUENCE CHARACTERISTICS:
      (A) LENGTH: 42 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (vi) ORIGINAL SOURCE:
      (A) ORGANISM: Homo sapiens
   (xi) SEQUENCE DESCRIPTION: SEQ ID NO:82:
   Cys Pro Ile Cys Leu Glu Leu Ile Lys Glu Pro Val Ser Thr Lys Cys
   1               5                   10                  15
   Asp His Ile Phe Cys Lys Phe Cys Met Leu Lys Leu Leu Asn Gln Lys
               20                  25                  30
   Lys Gly Pro Ser Gln Cys Pro Leu Cys Lys
           35                  40
(2) INFORMATION FOR SEQ ID NO:83:
   (i) SEQUENCE CHARACTERISTICS:
      (A) LENGTH: 45 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (xi) SEQUENCE DESCRIPTION: SEQ ID NO:83:
   Cys Pro Ile Cys Leu Glu Leu Leu Lys Glu Pro Val Ser Ala Asp Cys
   1               5                   10                  15
   Asn His Ser Phe Cys Arg Ala Cys Ile Thr Leu Asn Tyr Glu Ser Asn
               20                  25                  30
   Arg Asn Thr Asp Gly Lys Gly Asn Cys Pro Val Cys Arg
           35                  40                  45
(2) INFORMATION FOR SEQ ID NO:84:
   (i) SEQUENCE CHARACTERISTICS:
      (A) LENGTH: 41 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (xi) SEQUENCE DESCRIPTION: SEQ ID NO:84:
   Cys Pro Ile Cys Leu Asp Met Leu Lys Asn Thr Met Thr Thr Lys Glu
   1               5                   10                  15
   Cys Leu His Arg Phe Cys Ser Asp Cys Ile Val Thr Ala Leu Arg Ser
               20                  25                  30
   Gly Asn Lys Glu Cys Pro Thr Cys Arg
           35                  40
(2) INFORMATION FOR SEQ ID NO:85:
   (i) SEQUENCE CHARACTERISTICS:
      (A) LENGTH: 42 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (xi) SEQUENCE DESCRIPTION: SEQ ID NO:85:
   Cys Pro Val Cys Leu Gln Tyr Phe Ala Glu Pro Met Met Leu Asp Cys
   1               5                   10                  15
   Gly His Asn Ile Cys Cys Ala Cys Leu Ala Arg Cys Trp Gly Thr Ala
               20                  25                  30
   Cys Thr Asn Val Ser Cys Pro Gln Cys Arg
           35                  40